# EUROPEAN PATENT SPECIFICATION

(11) **EP 2 809 326 B1**
(45) Date of publication and mention of the grant of the patent: **25.07.2018**
(21) Application number: 13704317.0
(22) Date of filing: 30.01.2013
(51) Int. Cl.: A61K 31/715, A61P 7/04

(54) **NON-ANTICOAGULANT SULFATED OR SULFONATED POLYSACCHARIDES**
NICHT-ANTIKOAGULIERENDE SULFATIERTE ODER SULFONIERTE POLYSACCHARIDE
POLYSACCHARIDES SULFATÉS OU SULFONÉS NON ANTICOAGULANTS

(30) Priority: 30.01.2012 US 201261592549 P
(43) Date of publication of application: 10.12.2014
(73) Proprietor: Baxalta GmbH, 6300 Zug (CH); Baxalta Incorporated, Bannockburn, IL 60015 (US)
(72) Inventor: DOCKAL, Michael, 1150 Vienna (AT); SCHEIFLINGER, Fritz, 1090 Vienna (AT); KNAPPE, Sabine, 1060 Vienna (AT); TILL, Susanne, 1050 Vienna (AT); HAI, Ton, Round Lake, IL 60073 (US); SANDERS, Paul, Greendale, WI 53129 (US); DANDE, Prasad, Lake Villa, IL 60046 (US); JIANG, Cong, Gurnee, IL 60031 (US)
(74) Representative: Hoffmann Eitle
(86) International application number: PCT/US2013/023892
(87) International publication number: WO 2013/116366

(56) References cited:
- WO-A1-2008/134600
- WO-A1-2009/087581
- US-A- 4 713 373
- US-A1- 2007 244 166

## Description

### CROSS-REFERENCES TO RELATED APPLICATIONS

The present application claims the benefit of U.S. Provisional Application No. 61/592,549, filed January 30, 2012.

### BACKGROUND OF THE INVENTION

Normal blood coagulation is a complex physiological and biochemical process involving activation of a coagulation factor cascade leading to fibrin formation and platelet aggregation along with local vasoconstriction (reviewed by Davie, et al., Biochemistry, 30:10363, 1991). The clotting cascade is composed of an "extrinsic" pathway thought to be the primary means of normal coagulation initiation and an "intrinsic" pathway contributing to an expanded coagulation response. The normal response to a bleeding insult involves activation of the extrinsic pathway. Activation of the extrinsic pathway initiates when blood comes in contact with tissue factor (TF), a cofactor for Factor VII that becomes exposed or expressed on tissues following insult. TF forms a complex with FVII that facilitates the production of FVIIa. FVIIa then associates with TF to convert FX to the serine protease FXa, which is a critical component of the prothrombinase complex. The conversion of prothrombin to thrombin by the FXa/FVa/calcium/phospholipid complex stimulates the formation of fibrin and activation of platelets, all of which is essential to normal blood clotting. Normal hemostasis is further enhanced by intrinsic pathway Factors IXa and VIIIa, which also convert FX to FXa.

Blood clotting is inadequate in bleeding disorders, which may be caused by congenital coagulation disorders, acquired coagulation disorders, or hemorrhagic conditions induced by trauma. Bleeding is one of the most serious and significant manifestations of disease, and may occur from a local site or be generalized. Localized bleeding may be associated with lesions and may be further complicated by a defective haemostatic mechanism. Congenital or acquired deficiencies of any of the coagulation factors may be associated with a hemorrhagic tendency. Congenital coagulation disorders include hemophilia, a recessive X-linked disorder involving a deficiency of coagulation Factor VIII (hemophilia A) or Factor IX (hemophilia B) and von Willebrand disease, a rare bleeding disorder involving a severe deficiency of von Willebrand Factor. Acquired coagulation disorders may arise in individuals without a previous history of bleeding as a result of a disease process. For example, acquired coagulation disorders may be caused by inhibitors or autoimmunity against blood coagulation factors, such as Factor VIII, von Willebrand Factor, Factors IX, V, XI, XII and XIII; or by hemostatic disorders such as caused by liver disease, which may be associated with decreased synthesis of coagulation factors. Coagulation factor deficiencies are typically treated by factor replacement which is expensive, inconvenient (intravenous), and not always effective.

The treatment of blood clotting disorders including hemophilia (hem), severe von Willebrand (svWD) disease, and severe Factor VII deficiency are typically treated with coagulation factors such as Factor VIII (used to treat hem and svWD). The downside associated with treatments centered on administering coagulation factors include their high cost, the necessity of intravenous administration of these proteins, and the generation of antibodies which neutralize the effects of the coagulation factors. Up to approximately 20% of patients receiving chronic factor replacement therapy may generate neutralizing antibodies to replacement factors.

Thus, there remains a need for new therapeutic approaches for treating bleeding disorders. A single pharmaceutical agent that is safe, convenient and effective in a broad range of bleeding disorders would favorably impact clinical practice.

### SUMMARY OF THE INVENTION

The present invention provides compositions for treating bleeding disorders using non-anticoagulant sulfated or sulfonated polysaccharides (NASPs) as procoagulants. NASPs can be administered as single agents, or in combination with one another, or with other hemostatic agents. In particular, the use of NASPs in treatment of bleeding disorders, including congenital coagulation disorders, acquired coagulation disorders, and trauma induced hemorrhagic conditions is provided.

The present invention provides numerous advantages. For example, polysaccharides as base molecules for sulfation or sulfonation are structurally well-defined, many are of low molecular weight and are commercially available. Furthermore, chemical sulfation or sulfonation of polysaccharides allows adjustment of sulfation or sulfonation degree and sulfation or sulfonation pattern, which allows for the characterizion of the structure activity relationship of the sulfated or sulfonated polysaccharides. In an exemplary embodiment, the invention provides an oral dosage form incorporating one or more NASP of the invention, which improves patient care through increased ease of administration and patient compliance.

In one embodiment, the invention provides a sulfated or sulfonated polysaccharide with the ability to enhance coagulation of mammalian blood in vivo and/or in vitro. In various embodiments, the sulfated or sulfonated polysaccharide has procoagulant activity. In various aspects the procoagulant activity of the sulfated or sulfonated polysaccharide is of sufficient magnitude that it is measurable using a standard assay, *e.g.*, the Thrombin Generation Assay (TGA).

Exemplary sulfated or sulfonated polysaccharides of the invention are characterized by providing a subject administered one of these polysaccharides a therapeutically relevant procoagulant effect. Exemplary sulfated or sulfonated polysaccharides of the invention also exert an anticoagulant effect upon administration to a subject; in various embodiments, the polysaccharides of the invention do not induce a degree of anticoagulant effect sufficient to entirely offset the procoagulant effect of the polysaccharide.

In various embodiments, the invention provides a sulfated or sulfonated polysaccharide in which the base polysaccharide is selected from cellotriose, cellotetraose, cellopentaose, maltotriose, maltotetraose, maltopentaose, xylohexaose, raffinose, melezitose, stachyose, α-cyclodextrin, β-cyclodextrin and 6-carboxyiocdextrin, and icodextrin. In various embodiments the NASP of the invention decreases blood clotting time when tested in the TFPI- dilute prothrombin time (TFPI-dPT) assay.

In an exemplary embodiment, the sulfated or sulfonated polysaccharide is of use in a method for treating a subject in need of enhanced blood coagulation comprising administering a therapeutically effective amount of a composition comprising a non-anticoagulant, sulfated or sulfonated polysaccharide to the subject.

In various aspects, the specification provides a method for treating a subject in need of enhanced blood coagulation. The method includes administering a therapeutically effective amount of a composition comprising a non-anticoagulant sulfated or sulfonated polysaccharide (NASP) of the invention to the subject.

In certain instances, the specification provides a method for treating a subject having a bleeding disorder comprising administering a therapeutically effective amount of a composition comprising a NASP of the specification to the subject.

In certain embodiments, a NASP of the invention is administered to a subject to treat a bleeding disorder selected from the group consisting of hemophilia A, hemophilia B, von Willebrand disease, idiopathic thrombocytopenia, a deficiency of one or more coagulation factors (*e.g*., Factor XI, Factor XII, prekallikrein, and high molecular weight kininogen (HMWK)), a deficiency of one or more factors associated with clinically significant bleeding (*e.g*., Factor V, Factor VII, Factor VIII, Factor IX, Factor X, Factor XIII, Factor II (hypoprothrombinemia), and von Willebrand Factor), a vitamin K deficiency, a disorder of fibrinogen (*e.g*., afibrinogenemia, hypofibrinogenemia, and dysfibrinogenemia), an alpha2-antiplasmin deficiency, and excessive bleeding such as caused by liver disease, renal disease, thrombocytopenia, platelet dysfunction, hematomas, internal hemorrhage, hemarthroses, surgery, trauma, hypothermia, menstruation, and pregnancy.

In certain embodiments, a NASP is administered to a subject to treat a congenital coagulation disorder or an acquired coagulation disorder caused by a blood factor deficiency. The blood factor deficiency may be caused by deficiencies of one or more factors (*e.g*., Factor V, Factor VII, Factor VIII, Factor IX, Factor XI, Factor XII, Factor XIII, and von Willebrand Factor).

In exemplary embodiments, the NASP of the invention can be coadministered with one or more different NASPs and/or in combination with one or more other therapeutic agents. In certain embodiments, a subject having a bleeding disorder is administered a therapeutically effective amount of a composition comprising a NASP of the invention in combination with another therapeutic agent. For example, the subject may be administered a therapeutically effective amount of a composition comprising a NASP of the invention and one or more factors. Exemplary factors of use in this embodiment include, without limitation, Factor XI, Factor XII, prekallikrein, HMWK, Factor V, Factor VII, Factor VIII, Factor IX, Factor X, Factor XIII, Factor II, Factor VIIa, and von Willebrand Factor. Treatment may further comprise administering a procoagulant such as thrombin; an activator of the intrinsic coagulation pathway, including Factor Xa, Factor IXa, Factor XIa, Factor XIIa, and VIIIa, prekallikrein, and HMWK; or an activator of the extrinsic coagulation pathway, including tissue factor, Factor VIIa, Factor Va, and Factor Xa. Therapeutic agents used to treat a subject having a bleeding disorder can be administered in the same or different compositions and concurrently, before, or after administration of a NASP of the invention.

In various aspects, the invention provides a method for reversing the effects of an anticoagulant in a subject, the method comprising administering a therapeutically effective amount of a composition comprising a non-anticoagulant sulfated or sulfonated polysaccharide (NASP) of the invention to the subject. In certain embodiments, the subject may have been treated with an anticoagulant including, but not limited to, heparin, a coumarin derivative, such as warfarin or dicumarol, tissue factor pathway inhibitor (TFPI), antithrombin III, lupus anticoagulant, nematode anticoagulant peptide (NAPc2), active-site blocked Factor VIIa (Factor VIIai), Factor IXa inhibitors, Factor Xa inhibitors, including fondaparinux, idraparinux, DX-9065a, and razaxaban (DPC906), inhibitors of Factors Va and VIIIa, including activated protein C (APC) and soluble thrombomodulin, thrombin inhibitors, including hirudin, bivalirudin, argatroban, and ximelagatran. In certain embodiments, the anticoagulant in the subject may be an antibody that binds a coagulation factor, including but not limited to, an antibody that binds to Factor V, Factor VII, Factor VIII, Factor IX, Factor X, Factor XIII, Factor II, Factor XI, Factor XII, von Willebrand Factor, prekallikrein, or high-molecular weight kininogen (HMWK).

In certain embodiments, a NASP of the invention can be coadministered with one or more different NASPs and/or in combination with one or more other therapeutic agents for reversing the effects of an anticoagulant in a subject. For example, the subject may be administered a therapeutically effective amount of a composition comprising a NASP of the invention and one or more factors selected from the group consisting of Factor XI, Factor XII, prekallikrein, HMWK, Factor V, Factor VII, FactorVIII, Factor IX, Factor X, Factor XIII, Factor II, Factor VIIa, and von Willebrand Factor. Treatment may further comprise administering a procoagulant, such as an activator of the intrinsic coagulation pathway, including Factor Xa, Factor IXa, Factor XIa, Factor XIIa, and VIIIa, prekallikreinprekallikrein, and HMWK; or an activator of the extrinsic coagulation pathway, including tissue factor, Factor VIIa, Factor Va, and Factor Xa. Therapeutic agents used in combination with a NASP of the invention to reverse the effects of an anticoagulant in a subject can be administered in the same or different compositions and concurrently, before, or after administration of the NASP of the invention.

In another aspect, the invention provides a method for treating a subject undergoing a surgical or invasive procedure in which improved blood clotting is desirable. The method includes administering a therapeutically effective amount of a composition comprising a non-anticoagulant sulfated or sulfonated polysaccharide (NASP) of the invention to the subject. In certain embodiments, the NASP of the invention can be coadministered with one or more different NASPs and/or in combination with one or more other therapeutic agents, such as those factors, and/or procoagulant agents discussed herein. Therapeutic agents used to treat a subject undergoing a surgical or invasive procedure can be administered in the same or different compositions and concurrently, before, or after administration of the NASP of the invention.

In another embodiment, the invention provides a method of inhibiting TFPI activity in a subject, the method comprising administering a therapeutically effective amount of a composition comprising a NASP of the invention to the subject.

In an exemplary embodiment, the invention provides a method of inhibiting TFPI activity in a biological sample. The method includes combining the biological sample (e.g., blood or plasma) with a sufficient amount of a non-anticoagulant sulfated or sulfonated polysaccharide (NASP) of the invention to inhibit TFPI activity.

In another embodiment, the invention provides a composition comprising a NASP of the invention. In certain embodiments, the NASP is a sulfated or sulfonated polysaccharide in which the base polysaccharide is selected from cellotriose, cellotetraose, cellopentaose, maltotriose, maltotetraose, maltopentaose, xylohexaose, raffinose, melezitose, stachyose, α-cyclodextrin, β-cyclodextrin, 6-carboxyicodextrin, and in certain embodiments, the composition further comprises a pharmaceutically acceptable excipient. In certain embodiments, the composition further comprises one or more different NASPs, and/or one or more therapeutic agents, and/or reagents. For example, the composition may further comprise one or more factors selected from the group consisting of Factor XI, Factor XII, prekallikrein, HMWK, Factor V, Factor VII, Factor VIII, Factor IX, Factor X, Factor XIII, Factor II, and von Willebrand Factor, tissue factor, Factor VIIa, Factor Va, and Factor Xa, Factor IXa, Factor XIa, Factor XIIa, and VIIIa; and/or one or more composition selected from the group consisting of APTT reagent, thromboplastin, fibrin, TFPI, Russell's viper venom, micronized silica particles, ellagic acid, sulfatides, and kaolin.

These and other embodiments of the subject invention will readily occur to those of skill in the art in view of the disclosure herein.

### BRIEF DESCRIPTION OF THE DRAWINGS

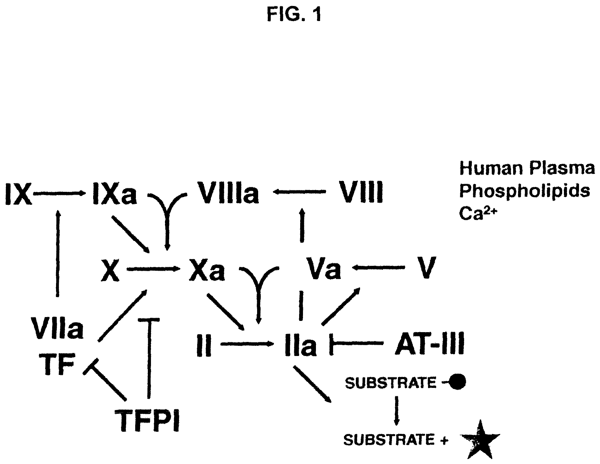

**FIG. 1** is a flowchart showing the generation of thrombin

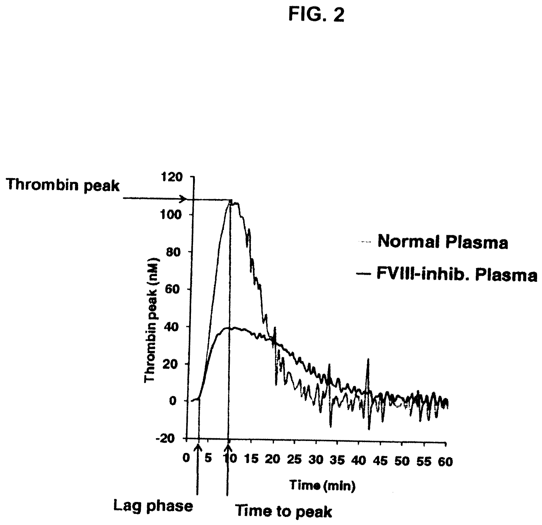

**FIG. 2** is an exemplary calibrated automatic thrombogram (CAT).
**FIG. 3A-B** shows CATs of FVIII-inhibited plasma including cellotriose. The NASP in **FIG. 3A** was 70% unsulfated; 30% monosulfated; <1% S. The measurements were taken at 37°C with 1 pM hTF and 4 µM PL. The oligosaccharide was procoagulant at >300 µg/mL. The NASP in **FIG. 3B** was 30% unsulfated; 70% monosulfated; <2% S. The measurements were taken at 37° C with 1 pM hTF and 4µ M PL. The polysaccharide was procoagulant at >300 µg/mL.
**FIG. 4A-B** shows CATs of FVIII-inhibited plasma including cellotetrose. The NASP in **FIG. 4A** was 80% unsulfated; 20% monosulfated; <0.5% S. The measurements were taken at 37° C with 1 pM hTF and 4 µM PL. The polysaccharide was procoagulant at >300 µg/mL. The NASP in **FIG. 4B** was 40% unsulfated; 30% monosulfated; 30% degradation product; <1% S. The measurements were taken at 37° C with 1 pM hTF and 4 µM PL. The polysaccharide was procoagulant at >300 µg/mL.
**FIG. 5A-B** shows CATs of FVIII-inhibited plasma including cellopentose. The NASP in **FIG. 5A** was 90% unsulfated; 10% monosulfated; <0.5% S. The measurements were taken at 37° C with 1 pM hTF and 4 µM PL. The polysaccharide was procoagulant at >300 µg/mL. The NASP in **FIG. 5B** was 80% unsulfated; 10% monosulfated; 10% degradation product; <0.5% S. The measurements were taken at 37° C with 1 pM hTF and 4 µM PL. The polysaccharide was procoagulant at >300 µg/mL.
**FIG. 6A-B** shows CATs of FVIII-inhibited plasma including maltotriose. The NASP in **FIG. 6A** was 50% monosulfated; 25% disulfated; 25% trisulfated; ∼4% S. The measurements were taken at 37° C with 1 pM hTF and 4 µM PL. The polysaccharide was procoagulant at >300 µg/mL. The NASP in **FIG. 6B** was 60% monosulfated; 40% degradation product; <2% S. The measurements were taken at 37° C with 1 pM hTF and 4 µM PL. The polysaccharide was procoagulant at >300 µg/mL.
**FIG. 7A-B** shows CATs of FVIII-inhibited plasma including maltotetrose. The NASP in **FIG. 7A** was 70% unsulfated; 30% monosulfated; <1% S. The measurements were taken at 37° C with 1 pM hTF and 4 µM PL. The polysaccharide was procoagulant at >300 µg/mL. The NASP in **FIG. 7B** was 50% unsulfated; 50% monosulfated; <1% S. The measurements were taken at 37° C with 1 pM hTF and 4µM PL. The polysaccharide was procoagulant at >300 µg/mL.
**FIG. 8A-B** shows CATs of FVIII-inhibited plasma including maltopentaose. The NASP in **FIG. 8A** was 60% unsulfated; 40% monosulfated; <1% S. The measurements were taken at 37° C with 1 pM hTF and 4 µM PL. The polysaccharide was procoagulant at >300 µg/mL. The NASP in **FIG. 8B** was 50% unsulfated; 30% monosulfated; 20% degradation product; <0.5% S. The measurements were taken at 37° C with 1 pM hTF and 4 µM PL. The polysaccharide was procoagulant at >300 µg/mL.
**FIG. 9A-B** shows CATs of FVIII-inhibited plasma including raffinose. The NASP in **FIG. 9A** was 70% unsulfated; 30% monosulfated; <1% S. The measurements were taken at 37° C with 1 pM hTF and 4 µM PL. The polysaccharide was procoagulant at >100 µg/mL. The NASP in **FIG. 9B** was 20% unsulfated; 30% monosulfated; 50% degradation product; <1% S. The measurements were taken at 37° C with 1 pM hTF and 4 µM PL. The polysaccharide was procoagulant at >100 µg/mL.
**FIG. 10A-B** shows CATs of FVIII-inhibited plasma including melezitose. The NASP in **FIG. 10A** was 40% unsulfated; 50% monosulfated; 10% disulfated; <2% S. The measurements were taken at 37° C with 1 pM hTF and 4 µM PL. The polysaccharide was procoagulant at >100 µg/mL. The NASP in **FIG. 10B** was 25% unsulfated; 50% monosulfated; 25% monosulfated; <3% S. The measurements were taken at 37° C with 1 pM hTF and 4 µM PL. The polysaccharide was procoagulant at >30 µg/mL.
**FIG. 11A-B** shows CATs of FVIII-inhibited plasma including α-cyclodextrin. The NASP in **FIG. 11A** was 45% unsulfated; 50% monosulfated; 5% disulfated; <1% S. The measurements were taken at 37° C with 1 pM hTF and 4 µM PL. The polysaccharide was procoagulant at >300 µg/mL. The NASP in **FIG. 11B** was 50% unsulfated; 50% monosulfated; <1% S. The measurements were taken at 37° C with 1 pM hTF and 4 µM PL. The polysaccharide was procoagulant at >300 µg/mL.
**FIG. 12A-B** shows CATs of FVIII-inhibited plasma including β-cyclodextrin. The NASP in **FIG. 12A** was 70% unsulfated; 30% monosulfated; <0.5% S. The measurements were taken at 37° C with 1 pM hTF and 4 µM PL. The polysaccharide was procoagulant at >300 µg/mL. The NASP in **FIG. 12B** was 60% unsulfated; 40% monosulfated; <0.5% S. The measurements were taken at 37° C with 1 pM hTF and 4 µM PL. The polysaccharide was procoagulant at >300 µg/mL.
**FIG. 13** shows CATs of FVIII-inhibited plasma including α-cyclodextrin. The NASP in **FIG. 13** had 15.3% S; ∼64% sulfation; ∼11 sulfates. The measurements were taken at 37° C with 1 pM hTF and 4 µM PL. The polysaccharide was procoagulant at >0.5 µg/mL.
**FIG. 14A-B** shows a CAT of FVIII-inhibited plasma including β-cyclodextrin. **FIG. 14A** shows a NASP with 13.5% S; ∼56% sulfation; ∼12 sulfates. The measurements were taken at 37° C with 1 pM hTF and 4µM PL. The polysaccharide was procoagulant with an EC50 of 2.1 µg/mL. **FIG. 14B** shows a NASP with 18.9% S; ∼2.9 kDa. The measurements were taken at 37° C with 1 pM hTF and 4 µM PL. The polysaccharide was procoagulant with an EC50 of 0.7 µg/mL.
**FIG. 15** shows a CAT of FVIII-inhibited plasma including melezitose. The NASP contained 18.7% S; ∼73% sulfation; ∼8 sulfates. The measurements were taken at 37° C with 1 pM hTF and 4 µM PL. The polysaccharide was procoagulant with an EC50 of 13.5 µg/mL.
**FIG. 16** shows a CAT of FVIII-inhibited plasma including stachyose. The NASP contained 18.4% S; ∼73% sulfation; ∼10 sulfates. The measurements were taken at 37° C with 1 pM hTF and 4 µM PL. The polysaccharide was procoagulant with an EC50 of 2.3 µg/mL.
**FIG. 17** is a CAT of FVIII-inhibited plasma including raffinose. The NASP contained 14.9% S; ∼58% sulfation; ∼6 sulfates. The measurements were taken at 37° C with 1 pM hTF and 4 µM PL. The polysaccharide was procoagulant with an EC50 of 7.4 µg/mL.
**FIG. 18** is a CAT of FVIII-inhibited plasma including maltotriose. The NASP contained 15.7% S; ∼61% sulfation; ∼7 sulfates. The measurements were taken at 37° C with 1 pM hTF and 4 µM PL. The polysaccharide was procoagulant with an EC50 of 20.6 µg/mL.
**FIG. 19** shows a CAT of FVIII-inhibited plasma including maltotetraose. The NASP contained 13.8% S; ∼55% sulfation; ∼8 sulfates. The measurements were taken at 37° C with 1 pM hTF and 4 µM PL. The polysaccharide was procoagulant with an EC50 of 5.0 µg/mL.
**FIG. 20** shows a CAT of FVIII-inhibited plasma including maltopentose. The NASP contained 13.9% S; ∼56% sulfation; ∼9 sulfates. The measurements were taken at 37° C with 1 pM hTF and 4 µM PL. The polysaccharide was procoagulant with an EC50 of 2.1 µg/mL.
**FIG. 21** shows a CAT of FVIII-inhibited plasma including cellotriose. The NASP contained 12.8% S; ∼50% sulfation; ∼5 sulfates. The measurements were taken at 37° C with 1 pM hTF and 4 µM PL. The polysaccharide was procoagulant with an EC50 of 30.9 µg/mL.
**FIG. 22** shows a CAT of FVIII-inhibited plasma including cellotetraose. The NASP contained 13% S; ∼51% sulfation; ∼7 sulfates. The measurements were taken at 37° C with 1 pM hTF and 4 µM PL. The polysaccharide was procoagulant with an EC50 of 4.8 µg/mL.
**FIG. 23** shows a CAT of FVIII-inhibited plasma including cellopentaose. The NASP contained 18% S; ∼72% sulfation; ∼12 sulfates. The measurements were taken at 37° C with 1 pM hTF and 4 µM PL. The polysaccharide was procoagulant with an EC50 of 1.9 µg/mL.
**FIG. 24** shows a CAT of FVIII-inhibited plasma including xylohexaose. The NASP contained 13.9% S; ∼59% sulfation; ∼8 sulfates. The measurements were taken at 37° C with 1 pM hTF and 4 µM PL. The polysaccharide was procoagulant with an EC50 of 4.8 µg/mL.
**FIG. 25** shows a CAT of FVIII-inhibited plasma including maltopentose. Sulfated maltopentaose of molecular weight 1.4 kD and 15% S was analyzed. The measurements were taken at 37° C with 1 pM hTF and 4 µM PL. The polysaccharide was procoagulant with an EC50 of 2.4 µg/mL.
**FIG. 26** is a comparison of the CATs in FVIII-inhibited plasma containing maltopentose or β-cyclodextrin. Maltopentaose contains 13.9% S; β-cyclodextrin has 18.9% S and a molecular weight of 2.9 kD. The measurements were taken at 37° C with 1 pM hTF and 4 µM PL. The polysaccharides were procoagulant with an EC50 of about 2 µg/mL.
**FIG. 27** is a comparison of the CATs of FVIII-inhibited plasma containing maltopentose (13.9% S) and maltopentose (<1% S). This comparison demonstrates the relationship of sulfation to procoagulant activity. The measurements were taken at 37° C with 1 pM hTF and 4 µM PL.
**FIG. 28** is a comparison of the CATs of FVIII-inhibited plasma containing α-cyclodextrin (18.1%S), β-cyclodextrin (18.9% S) and γ-cyclodextrin (20.0% S). The measurements were taken at 37° C with 1 pM hTF and 4 µM PL. This comparison demonstrates the relationship of molecular weight to procoagulant activity.
**FIG. 29** is a comparison of aPTT assays with β-cyclodextrin, α-cyclodextrin, and meletzitose. This comparison demonstrates the anticoagulant activity of the compounds. The concentration where the clotting time is 50% increased over a normal plasma control was determined. The oligosaccharides become anticoagulant at their optimal procoagulant concentration.
**FIG. 30** is a rotational thromboelastogram (ROTEM) of sulfated maltopentose (15% S) in FVIII-inhibited human whole blood (0.044 pM TF), showing that sulfated maltopentose restores coagulation in FVIII-inhibited blood.
**FIG. 31** is a ROTEM of sulfated β-cyclodextrin (18.9% S) in FVIII-inhibited human whole blood (0.044 pM TF), showing that sulfated β-cyclodextrin restores coagulation in FVIII-inhibited blood.
**FIG. 32** is a CAT of sulfated maltopentaose (15% S) in normal plasma, showing that sulfated maltopentaose does not activate the contact pathway in the absence of CTI up to 33 µg/mL. The measurements were taken at 37° C with 1 pM hTF, 4µM PL and ± 41 µg/mL CTI.
**FIG. 33** is a CAT of sulfated β-cyclodextrin (2.9 kDa,18.9% S) in normal plasma, showing that sulfated β-cyclodextrin does not activate the contact pathway up to 33 µg/mL. The measurements were taken at 37° C with 1 pM hTF, 4µM PL and ± 41 µg/mL CTI.
**FIG. 34** is a plot showing TFPI-dPT vs. log concentration of sulfated maltopentaose (15% S) in normal human plasma, showing that sulfated maltopentaose reverses the effect of recombinant Full Length-Tissue Factor Pathway Inhibitor (rec. FL-TFPI) in plasma. The EC50 of this compound is 0.15 µg/mL.
**FIG. 35** is a plot showing TFPI -dPT vs. log concentration of sulfated β-cyclodextrin (2.9 kDa, 18.9% S) in normal human plasma, showing that sulfated β-cyclodextrin reverses the effect of rec. FL-TFPI in plasma. The EC50 of this compound is 0.08 µg/mL.

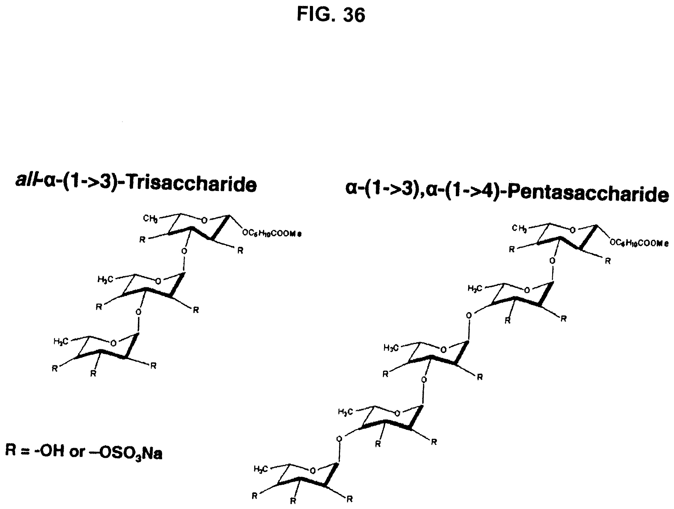

**FIG. 36** is a synthetic scheme showing a route to the de novo synthesis of sulfated fucose oligosaccharides.

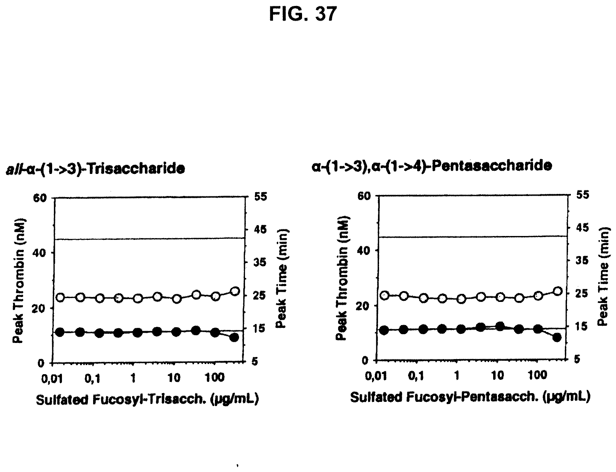

**FIG. 37** is a CAT in FVIII-inhibited plasma showing that fucosyl polysaccharides do not have procoagulant activity up to 300 µg/mL. The measurements were taken at 37° C with 1 pM hTF and 4 µM PL.

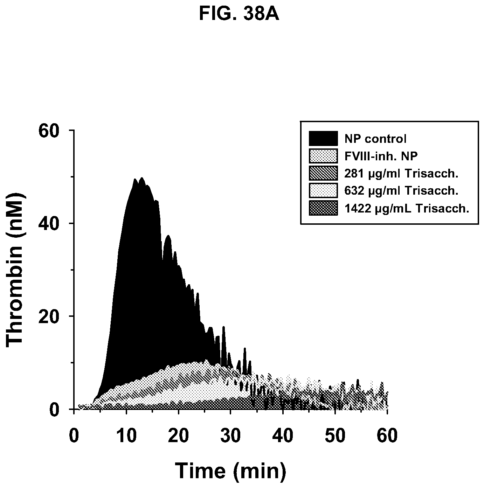

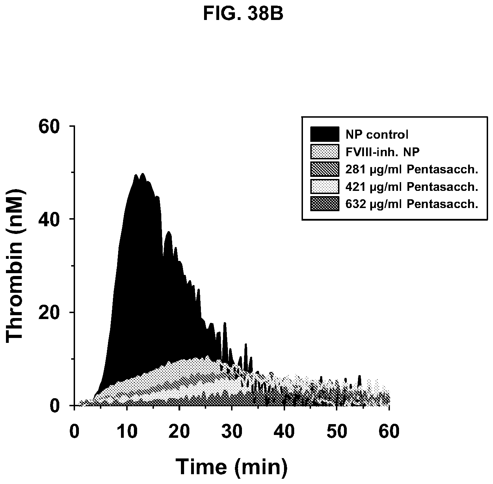

**FIG. 38A-B** is a CAT in FVIII-inhibited plasma showing that fucosyl polysaccharides become anticoagulant at >200 µg/mL. (A) trifucosyl saccharide; (B) pentafucosyl saccharide. The measurements were taken at 37° C with 1 pM hTF and 4 µM PL.
**FIG. 39** shows the structures of icodextrin/6-carboxy-icodextrin and xylan.
**FIG. 40** is a CAT in FVIII-inhibited plasma showing that sulfated xylan (14.7% S, 22 kD) is procoagulant at low concentrations. The measurements were taken at 37° C with 1 pM hTF and 4 µM PL.
**FIG. 41** is a CAT in FVIII-inhibited plasma showing a comparison of two depolymerized (6.5 kDa; 13% S and 2.8 kDa; 15.5% S) sulfated xylans. Depolymerization of xylan reduces procoagulant activity. The measurements were taken at 37 °C with 1 pM hTF and 4 µM PL.
**FIG. 42** is a CAT in FVIII-inhibited plasma showing a comparison of unsulfated icodextrin and 6-carboxyicodextrin. Unsulfated icodextrins are not procoagulant. The measurements were taken at 37° C with 1 pM hTF and 4 µM PL.
**FIG. 43** is a CAT in FVIII-inhibited plasma showing the procoagulant activity of sulfated 6-carboxyicodextrin (21.6 kDa, 10.6% S), which is procoagulant at very low concentrations. The measurements were taken at 37° C with 1 pM hTF and 4µM PL. The EC50 of this compound is 0.04 µg/mL.
**FIG. 44** is a CAT in FVIII-inhibited plasma showing the procoagulant activity of sulfated 6-carboxyicodextrin (35 kDa, 10.1% S), which is procoagulant at very low concentrations. The measurements were taken at 37° C with 1 pM hTF and 4 µM PL. The EC50 of this compound is 0.07 µg/mL.
**FIG. 45** is a ROTEM of sulfated 6-carboxy-icodextrin (35 kDa; 10.1% S) in FVIII- inhibited whole blood, showing that sulfated 6-carboxyicodextrin restores coagulation in human FVIII-inhibited blood.
**FIG. 46** is a CAT of sulfated 6-carboxy-icodextrin (35 kDa;10.1% S) in normal plasma, showing that sulfated 6-carboxy-icodextrin activates the contact pathway at 33 µg/mL. The measurements were taken at 37°C with 1 pM hTF, 4 µM PL and ± 41 µg/mL CTI.
**FIG. 47** is a plot showing TFPI-dPTvs. log concentration of sulfated 6-carboxyicodextrin (21.6 kDa, 10.6% S) in normal human plasma, showing that sulfated 6-carboxyicodextrin reverses the effect of rec. FL-TFPI in plasma. The EC50 of this compound is 0.26 µg/mL.

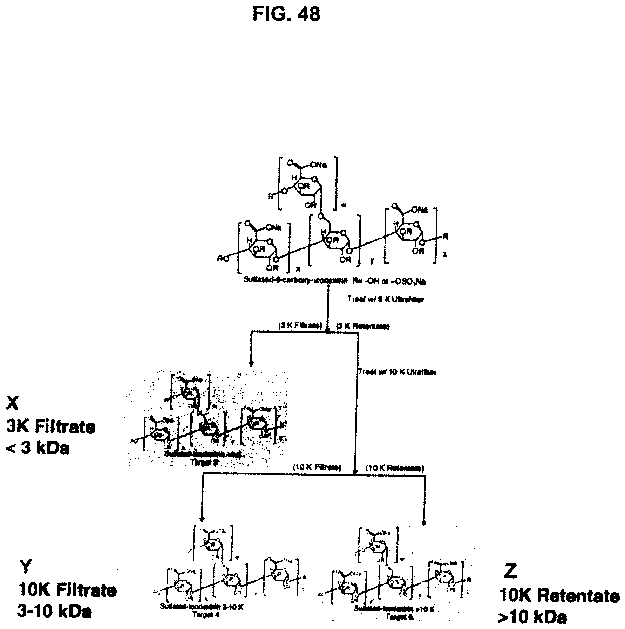

**FIG. 48** shows an exemplary process chart for the fractionation of 6-carboxyicodextrin.

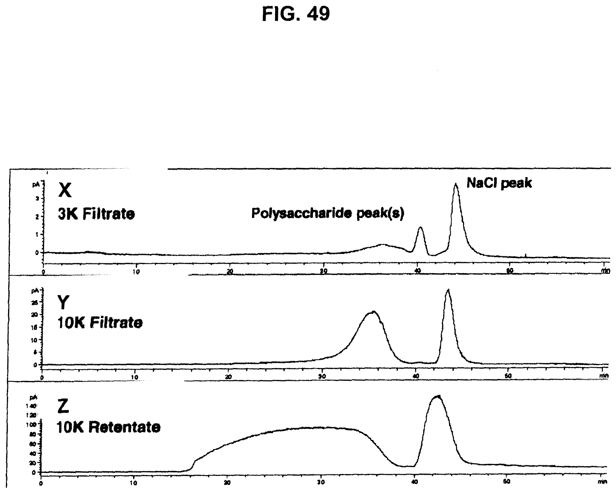

**FIG. 49** shows size exclusion chromatograms of fractionated sulfated 6-carboxyicodextrin.
**FIG. 50** is a CAT showing the procoagulant activity of sulfated 6-carboxyicodextrin fractions (>10kDa, 3-10 kDa, <3 kDa) in FVIII-inhibited plasma. Even low molecular weight sulfated 6-carboxy-icodextrin is procoagulant. The measurements were taken at 37 °C temperature with 1 pM hTF and 4 µM PL.

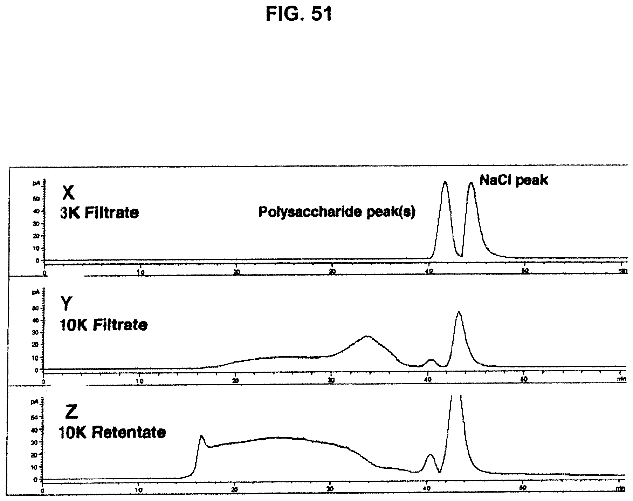

**FIG. 51** shows size exclusion chromatograms of fractionated sulfated icodextrin. Fractionation leads to sample with different molecular weight distributions.

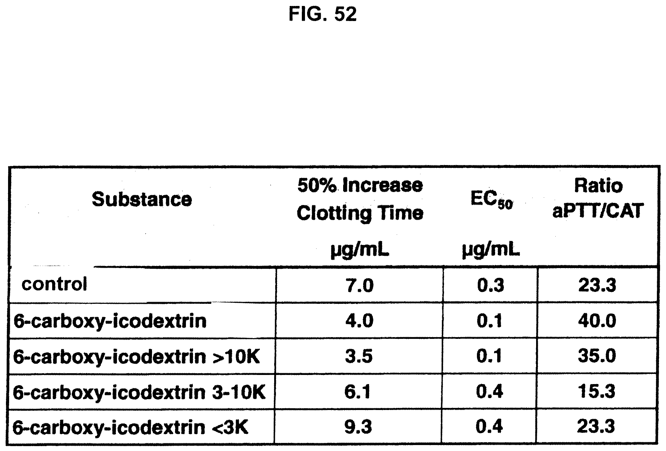

**FIG. 52** is a table showing the EC50s and ratios of aPTT/CAT for fractions of sulfated 6-carboxy-icodextrin.

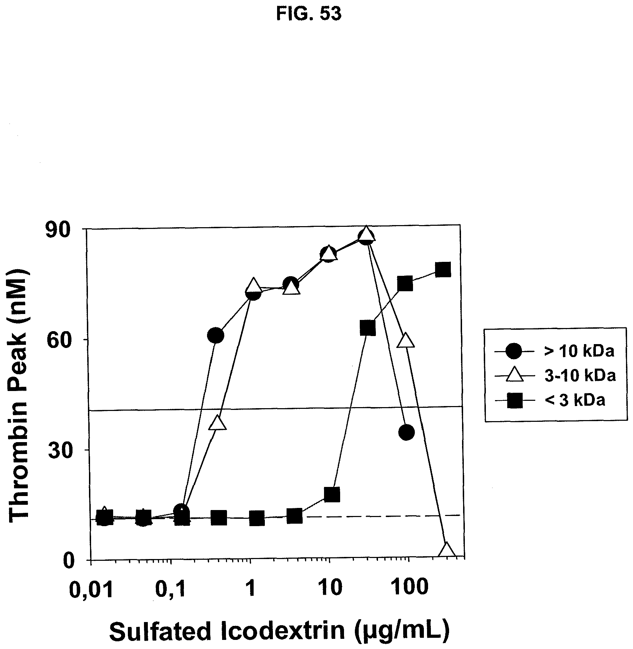

**FIG. 53** is a CAT showing the procoagulant activity of sulfated icodextrin fractions (>10kDa, 3-10 kDa, <3 kDa) in FVIII-inhibited plasma. Even low molecular weight sulfated icodextrin is procoagulant. The measurements were taken at 37° C with 1 pM hTF and 4 µM PL.

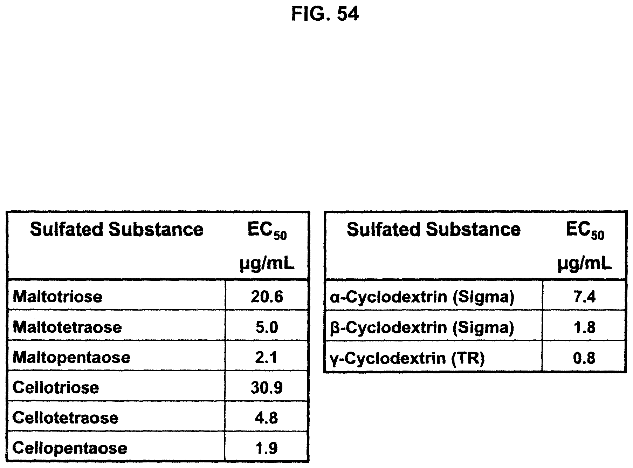

**FIG. 54** is a tabulation of representative NASPs of the invention and their EC50 values derived from CAT assays.
**FIG. 55** is an exemplary synthetic route for the sulfation of 6-carboxy-icodextrin.
**FIG. 56** is a tabulation of therapeutic windows and optimal concentrations for sulfated xylan of the invention.
**FIG. 57** shows CATs comparing the effect on procoagulant activity of sulfating the xylan under different conditions. The measurements were taken at 37° C with 1 pM hTF and 4 µM PL.
**FIG. 58** shows CATs comparing the effect on procoagulant activity of sulfating the xylan under different conditions. The measurements were taken at 37° C with 1 pM hTF and 4 µM PL.
**FIG. 59** is a CAT in FVIII-inhibited plasma showing that a longer sulfation reaction time does not alter the procoagulant properties of the NASP. The measurements were taken at 37° C with 1 pM hTF and 4 µM PL
**FIG. 60** is a CAT in FVIII-inhibited plasma showing that sulfation of 6-carboxyicodextrin confers procoagulant activity at very low concentrations of the compound. The measurements were taken at 37° C with 1 pM hTF and 4 µM PL.
**FIG. 61** is a plot of clotting time determined by aPTT assays versus NASP concentration showing that the sulfated NASPs of the invention are more anticoagulant than a control (fucoidan).
**FIG. 62** compares CAT and aPTT data of sulfated xylans showing that sulfated xylans are not anticoagulant at their optimal procoagulant concentration.
**FIG. 63** shows CAT and aPTT results of sulfated 6-carboxy-icodextrin (11%S) showing that this NASP is non-anticoagulant at its optimal procoagulant concentration. The measurements were taken at 37° C.
**FIG. 64** is a tabulation of the concentration at which anticoagulant activity begins and maximum clotting time for selected NASPs of the invention.
**FIG. 65** is an exemplary synthetic route for the synthesis of sulfated xylan.
**FIG. 66A-B** is a matrix table showing exemplary combinations of certain types of NASPs (based on their base polysaccharide) with additional agents.
**FIG. 67A-G** is a matrix table showing exemplary dosages of the respective NASP in each of the combinations identified in **FIG. 66A-B.**
**FIG. 68** is a table showing exemplary dosages of additional agents in the combination therapeutics of the invention.
**FIG. 69** is resorption of sulfated maltopenatose in the Caco-2 cell model with or without permeation enhancers: The amount of NASP transported onto the basolateral side of the cells was determined by an activity-based thrombin generation assay in FVIII-inhibited human plasma.
**FIG. 70** is resorption of sulfated β-cyclodextrin in the Caco-2 cell model with or without permeation enhancers: The amount of NASP transported onto the basolateral side of the cells was determined by an activity-based thrombin generation assay in FVIII-inhibited human plasma.
**FIG. 71** is a resorption of fractionated sulfated 6-carboxy-icodextrin (Lot 137) in the Caco-2 cell model with or without permeation enhancers: The amount of NASP transported onto the basolateral side of the cells was determined by an activity-based thrombin generation assay in FVIII-inhibited human plasma.
**FIG. 72** is resorption of unfractionated sulfated 6-carboxy-icodextrin (Lot 171A) in the Caco-2 cell model with or without permeation enhancers: The amount of NASP transported onto the basolateral side of the cells was determined by an activity-based thrombin generation assay in FVIII-inhibited human plasma.

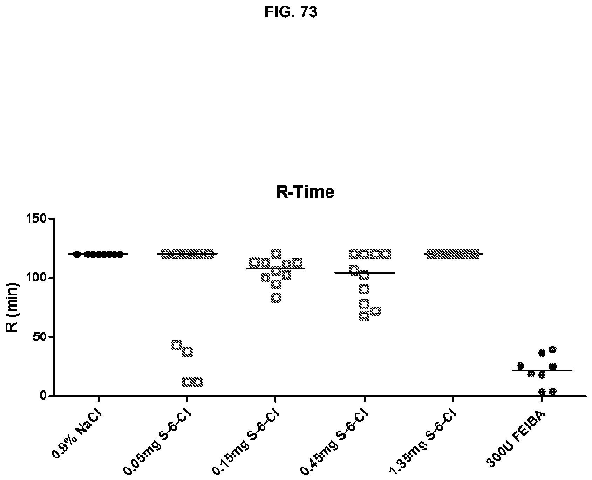

**FIG. 73** is a TEG assay showing that clotting time (R-time) is decreased after intravenous administration sulfated 6-carboxy-icodextrin to FVIII-inhibited guinea pigs. Guinea pigs treated with anti-FVIII inhibitor plasma were injected with four doses of sulfated 6-carboxy-icodextrin, saline or FEIBA (N=5 in duplicate each). Five minutes after administration, TEG measurements were performed with citrated whole blood and the R-time was recorded. A procoagulant effect superior to vehicle control as reflected by a reduction in R-time, was observed for NASP at 0.15 and 0.45 mg/kg and the positive control FEIBA. The graph shows medians and individual results.

### DETAILED DESCRIPTION OF THE INVENTION

### Introduction

Blood clotting disorders including hemophilia (Hem) A and Hem B, severe von Willebrand disease (svWD), and severe Factor VII (FVII) deficiency have typically been treated by factor replacement, *e.g*., Factor VIII for Hem A and svWD, Factor IX for Hem B, and Factor VII(a) for FVII-deficiency and others (reviewed in Bishop, et al. (2004) Nat. Rev. Drug Discov., 3:684-694; Carcao, et al. (2004) Blood Rev., 18:101-113; Roberts, et al. (2004) Anesthesiology 100:722-730; and Lee (2004) Int. Anesthesiol. Clin., 42:59-76). While such therapies are often effective, characteristics limiting utility include high cost, inconvenience *(i.e.,* intravenous administration), and neutralizing antibody generation (Bishop, *et al*., *supra;* Carcao, *et al*., *supra;* Roberts, *et al*., *supra;* Lee, *supra;* and Bohn, et al. (2004) Haemophilia 10 Suppl., 1:2-8). While FVIIa is increasingly utilized in various bleeding disorders (Roberts, *et al*., *supra*), alternative single compound procoagulant therapies devoid of the aforementioned constraints and with broad application are of interest.

One general approach to improving hemostasis in individuals with bleeding disorders is to improve the initiation of clotting by upregulating the extrinsic pathway of blood coagulation. While the intrinsic and extrinsic pathways of coagulation contribute to thrombin generation and fibrin clot formation (Davie, et al. (1991) Biochemistry, 30:10363-10370), the extrinsic--or tissue factor (TF) mediated--path is critical for initiation, and contributes to propagation of coagulation in vivo (Mann (2003) Chest, 124(3 Suppl): 1S-3S; Rapaport, et al. (1995) Thromb. Haemost., 74:7-17). One potential mechanism for upregulating extrinsic pathway activity is the attenuation of Tissue Factor Pathway Inhibitor (TFPI). TFPI is a Kunitz-type proteinase inhibitor of FVIIa/TF that provides tonic downregulation of extrinsic pathway activation (see Broze (1992) Semin. Hematol., 29:159-169; Broze (2003) J. Thromb. Haemost., 1:1671-1675; and Johnson, et al. (1998) Coron. Artery Dis., 9(2-3):83-87 for review). Indeed, heterozygous TFPI deficiency in mice can result in exacerbation of thrombus formation (Westrick, et al. (2001) Circulation, 103:3044-3046), and TFPI gene mutation is a risk factor for thrombosis in humans (Kleesiek, et al. (1999) Thromb. Haemost., 82:1-5). Regulating clotting in hemophilia via the targeting of TFPI was described by Nordfang, *et al.* and Wun, *et al*., who showed that anti-TFPI antibodies could shorten the coagulation time of hemophilic plasma (Nordfang, et al. (1991) Thromb. Haemost., 66:464-467; Welsch, et al. (1991) Thromb. Res., 64:213-222) and that anti-TFPI IgG improved the bleeding time of rabbits that were Factor VIII-deficient (Erhardtsen, et al. (1995) Blood Coagul. Fibrinolysis, 6:388-394).

As a class, sulfated polysaccharides are characterized by a plethora of biological activities with often favorable tolerability profiles in animals and humans. These polyanionic molecules are often derived from plant and animal tissues and encompass a broad range of subclasses including heparins, glycosaminoglycans, fucoidans, carrageenans, pentosan polysulfates, and dermatan or dextran sulfates (Toida, et al. (2003) Trends in Glycoscience and Glycotechnology, 15:29-46). Lower molecular weight, less heterogeneous, and chemically synthesized sulfated polysaccharides have been reported and have reached various stages of drug development (Sinay (1999) Nature, 398:377-378; Bates, et al. (1998) Coron. Artery Dis., 9:65-74; Orgueira, et al. (2003) Chemistry, 9:140-169; McAuliffe (1997) Chemical Industry Magazine, 3:170-174; Williams, et al. (1998) Gen. Pharmacol., 30:337-341). Heparin-like sulfated polysaccharides exhibit differential anticoagulant activity mediated through antithrombin III and/or heparin cofactor II interactions (Toida, *et al*., *supra*). Notably, certain compounds, of natural origin or chemically modified, exhibit other biological activities at concentrations (or doses) at which anticoagulant activity is not substantial (Williams, et al. 1998) Gen. Pharmacol., 30:337-341; Wan, et al. (2002) Inflamm. Res., 51:435-443; Bourin, et al. (1993) Biochem. J., 289 (Pt 2):313-330; McCaffrey, et al. (1992) Biochem. Biophys. Res. Commun., 184:773-781; Luyt, et al. (2003) J. Pharmacol. Exp. Ther., 305:24-30). In addition, heparin sulfate has been shown to exhibit strong interactions with TFPI (Broze (1992) Semin. Hematol., 29:159-169; Broze (2003) J. Thromb. Haemost., 1:1671-1675; Johnson, et al. (1998) Coron. Artery Dis., 9:83-87; Novotny, et al. (1991) Blood, 78(2):394-400).

As described herein, certain sulfated or sulfonated polysaccharides interact with TFPI and inhibit its activity at lower concentrations than those associated with anticoagulation. Such molecules may be of use in settings where clot formation is compromised.

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein.

### Definitions

In describing the present invention, the following terms will be employed, and are intended to be defined as indicated below.

As used in this specification and the appended claims, the singular forms "a", "an" and "the" include plural referents unless the content clearly dictates otherwise. Thus, for example, reference to "a NASP" includes a mixture of two or more such agents, and the like.

A "NASP" as used herein refers to a sulfated or sulfonated polysaccharide that exhibits anticoagulant activity in a dilute prothrombin time (dPT) or activated partial thromboplastin time (aPTT) clotting assay that is no more than one-third, and preferably less than one-tenth, the anticoagulant (increase in clotting time) activity of unfractionated heparin *(e.g.,* as measured by increase per µg/mL). NASPs of the invention may be purified and/or modified from natural sources (*e.g*., brown algae, tree bark, animal tissue) or may be synthesized de novo and may range in molecular weight from 100 Daltons to 1,000,000 Daltons. NASPs of the invention may be used in the methods of the invention for improving hemostasis in treating bleeding disorders, particularly those associated with deficiencies of coagulation factors or for reversing the effects of anticoagulants. The ability of NASPs of the invention to promote clotting and reduce bleeding is readily determined using various in vitro global hemostatic and clotting assays (*e.g*., TFPI-dPT, aPTT, CAT and ROTEM assays) and in vivo bleeding models (*e.g*., tail transection, transverse cut, whole blood clotting time, or cuticle bleeding time determination in hemophilic mice or dogs). See, *e.g.,* PDR Staff. Physicians' Desk Reference. 2004, Anderson, et al. (1976) Thromb. Res., 9:575-580; Nordfang, et al. (1991) Thromb Haemost., 66:464-467; Welsch, et al. (1991) Thrombosis Research, 64:213-222; Broze, et al. (2001) Thromb Haemost, 85:747-748; Scallan, et al. (2003) Blood, 102:2031-2037; Pijnappels, et al. (1986) Thromb. Haemost., 55:70-73; and Giles, et al. (1982) Blood, 60:727-730.

A "procoagulant" is used herein in its conventional sense to refer to any factor or reagent capable of initiating or accelerating clot formation. Exemplary procoagulants include a NASP, any activator of the intrinsic or extrinsic coagulation pathways, such as a coagulation factor selected from the group consisting of Factor Xa, Factor IXa, Factor XIa, Factor XIIa, and VIIIa, prekallikrein, HMWK, tissue factor, Factor VIIa, and Factor Va. Other reagents that promote clotting include kallikrein, APTT initiator *(i.e.,* a reagent containing a phospholipid and a contact activator), Russel's viper venom (RVV), and thromboplastin (for dPT). Contact activators that can be used in the methods of the invention as procoagulant reagents include micronized silica particles, ellagic acid, sulfatides, kaolin or the like known to those of skill in the art. Procoagulants may be from a crude natural extract, a blood or plasma sample, isolated and substantially purified, synthetic, or recombinant. Procoagulants may include naturally occurring coagulation factors or fragments, variants or covalently modified derivatives thereof that retain biological activity *(i.e.,* promote clotting). Optimal concentrations and dosages of the procoagulant to treat a selected disease can be determined by those of skill in the art.

The term "polysaccharide," as used herein, refers to a polymer comprising a plurality *(i.e.,* two or more) of covalently linked saccharide residues. Linkages may be natural or unnatural. Natural linkages include, for example, glycosidic bonds, while unnatural linkages may include, for example, ester, amide, or oxime linking moieties. Polysaccharides have any of a wide range of average molecular weight (MW) values, but generally are of at least about 100 Daltons. For example, the polysaccharides can have molecular weights of at least about 500, 1000, 2000, 4000, 6000, 8000, 10,000, 20,000, 30,000, 50,000, 100,000, 500,000 Daltons or higher. Polysaccharides may have a linear chain or branched structures. Polysaccharides may include fragments of polysaccharides generated by degradation (*e.g*., hydrolysis) of larger polysaccharides. Degradation can be achieved by any of a variety of means known to those skilled in the art including treatment of polysaccharides with acid, base, heat, or enzymes to yield degraded polysaccharides. Polysaccharides may be chemically altered and may have modifications, including but not limited to, sulfation, polysulfation, sulfonation, polysulfonation, esterification, and alkylation, *e.g*., methylation.

The term "derived from" is used herein to identify the original source of a molecule but is not meant to limit the method by which the molecule is made which can be, for example, by chemical synthesis or recombinant means.

Molecular weight", as discussed herein, can be expressed as either a number average molecular weight or a weight average molecular weight. Unless otherwise indicated, all references to molecular weight herein refer to the weight average molecular weight. Both molecular weight determinations, number average and weight average, can be measured using for example, gel permeation chromatography or other liquid chromatography techniques.

"Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.

The phrase "therapeutically effective amount" as used herein means that amount of a compound, material, or composition comprising a NASP of the present invention which is effective for producing a desired therapeutic effect, at a reasonable benefit/risk ratio, such as those generally applicable to the treatment of the bleeding disorder using art-standard pharmaceuticals. "Therapeutically effective dose or amount" of a NASP, blood factor, or other therapeutic agent refers to an amount of this substance that, when administered as described herein, brings about a positive therapeutic response, such as reduced bleeding or shorter clotting times.

The term "pharmaceutically acceptable salts" includes salts of the active compounds which are prepared with relatively non-toxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively non-toxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al., Journal of Pharmaceutical Science, 66: 1-19 (1977)). Certain specific compounds of the present invention contain both basic and acidic functionalities that allow the compounds to be converted into either base or acid addition salts.

When a residue is defined as "SO₃⁻", then the formula is meant to optionally include an organic or inorganic cationic counterion. Preferably, the resulting salt form of the compound is pharmaceutically acceptable. This structure also encompasses the protonated species, "SO₃H".

The neutral forms of the compounds are preferably regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

The term "bleeding disorder" as used herein refers to any disorder associated with excessive bleeding, such as a congenital coagulation disorder, an acquired coagulation disorder, or a trauma induced hemorrhagic condition. Such bleeding disorders include, but are not limited to, hemophilia (Hem) A, Hem B, von Willebrand disease, idiopathic thrombocytopenia, a deficiency of one or more coagulation factors, such as Factor XI, Factor XII, prekallikrein, and HMWK, a deficiency of one or more factors associated with clinically significant bleeding, such as Factor V, Factor VII, Factor VIII, Factor IX, Factor X, Factor XIII, Factor II (hypoprothrombinemia), and von Willebrand Factor, a vitamin K deficiency, a disorder of fibrinogen, including afibrinogenemia, hypofibrinogenemia, and dysfibrinogenemia, an alpha2-antiplasmin deficiency, and excessive bleeding such as caused by liver disease, renal disease, thrombocytopenia, platelet dysfunction, hematomas, internal hemorrhage, hemarthroses, surgery, trauma, hypothermia, menstruation, and pregnancy.

"Icodex", as used herein refers to sulfated 6-carboxy-icodextrin.

By "subject" is meant any member of the subphylum chordata, including, without limitation, humans and other primates, including non human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term does not denote a particular age. Thus, both adult and newborn individuals are of interest.

The term "patient," is used in its conventional sense to refer to a living organism suffering from or prone to a condition that can be prevented or treated by administration of a NASP of the invention, and includes both humans and non-human species.

"TFPI" and "flTFPI", as used herein, refer to tissue factor pathway inhibitor and full length tissue factor pathway inhibitor, respectively.

"TFPI160" refers to a polypeptide including amino acid 1-160, including KD1 and KD2 domains, of TFPI. The KD3 and C-terminus of full length TFPI are absent.

### THE EMBODIMENTS

Aspects of the invention include compositions, formulations containing these compositions and methods for enhancing blood coagulation in a subject. In practicing methods according to certain embodiments, an amount of a non-anticoagulant sulfated or sulfonated polysaccharide (NASP) is administered to a subject in a manner sufficient to enhance blood coagulation in the subject. Kits for practicing methods of the invention are also provided.

Before the invention is described in greater detail, it is to be understood that the invention is not limited to particular embodiments described herein as such embodiments may vary. It is also to be understood that the terminology used herein is for the purpose of describing particular embodiments only, and the terminology is not intended to be limiting. The scope of the invention will be limited only by the appended claims. Unless defined otherwise, all technical and scientific terms used herein have the same meaning as commonly understood by one of ordinary skill in the art to which this invention belongs. Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention. Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number, which, in the context in which it is presented, provides the substantial equivalent of the specifically recited number. The citation of any publication is for its disclosure prior to the filing date and should not be construed as an admission that the invention described herein is not entitled to antedate such publication by virtue of prior invention. Further, the dates of publication provided might be different from the actual publication dates, which may need to be independently confirmed.

It is noted that the claims may be drafted to exclude any optional element. As such, this statement is intended to serve as antecedent basis for use of such exclusive terminology as "solely," "only," and the like in connection with the recitation of claim elements, or use of a "negative" limitation. As will be apparent to those of skill in the art upon reading this disclosure, each of the individual embodiments described and illustrated herein has discrete components and features which may be readily separated from or combined with the features of any of the other several embodiments without departing from the scope of the invention. Any recited method may be carried out in the order of events recited or in any other order that is logically possible. Although any methods and materials similar or equivalent to those described herein may also be used in the practice or testing of the invention, representative illustrative methods and materials are now described.

### A. NASPs

In one aspect, the invention provides a sulfated or sulfonated polysaccharide with the ability to enhance coagulation of mammalian blood in vivo or in vitro. In various embodiments, the sulfated or sulfonated polysaccharide has procoagulant activity. In an exemplary embodiment, the procoagulant activity of the sulfated or sulfonated polysaccharide is of sufficient magnitude that is measurable using a standard global hemostatic assay, *e.g*., the Thrombin Generation Assay (TGA).

Exemplary NASPs of the invention are characterized by providing a subject administered one of these polysaccharides a therapeutically effective procoagulant effect. Exemplary NASPs of the invention also exert an anticoagulant effect upon administration to a subject; in various embodiments, the NASPs of the invention do not induce a degree of anticoagulant effect sufficient to entirely offset the therapeutically effective procoagulant effect of the NASP. Exemplary NASPs of the invention are procoagulant at a concentration of from about 0.01 µg/mL to about 700 µg/mL of plasma (*e.g.,* human plasma), *e.g.,* from about 10 µg/mL to about 600 µg/mL plasma, e.g, from about 20 µg/mL to about 500 µg/mL plasma, *e.g.,* from about 30 µg/mL to about 400 µg/mL, *e.g.,* from about 40 µg/mL to about 300 µg/mL plasma, *e.g.,* from about 50 µg/mL to about 200 µg/mL plasma, *e.g.,* from about 60 µg/mL to about 100 µg/mL plasma. In various embodiment, the NASPs of the invention have a procoagulant effect at concentrations of from about 1 µg/mL to about 300 µg/mL, *e.g.,* from about 5 µg/mL to about 250 µg/mL, *e.g.,* from about 10 µg/mL to about 200 µg/mL, *e.g.,* from about 15 µg/mL to about 150 µg/mL, *e.g.,* from about 20 µg/mL to about 100 µg/mL, *e.g.,* from about 25 µg/mL to about 50 µg/mL. In various embodiments, the compounds of the invention have a procoagulant effect at a concentration of not more than about 400 µg/mL, *e.g.,* not more than about 350 µg/mL, *e.g.,* not more than about 300 µg/mL, *e.g.,* not more than about 250 µg/mL, *e.g.,* not more than about 200 µg/mL, *e.g.,* not more than about 150 µg/mL, *e.g.,* not more than about 100 µg/mL, *e.g.,* not more than about 50 µg/mL.

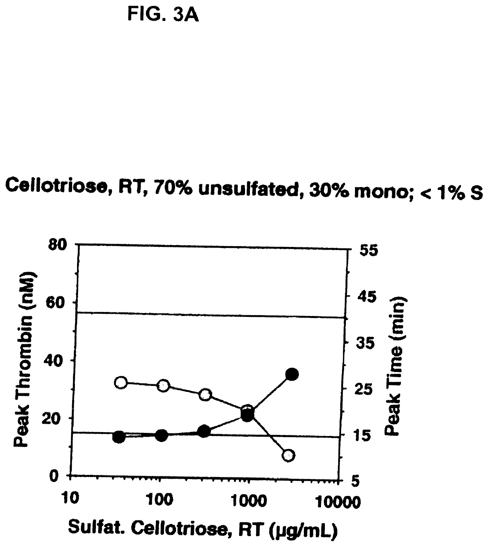

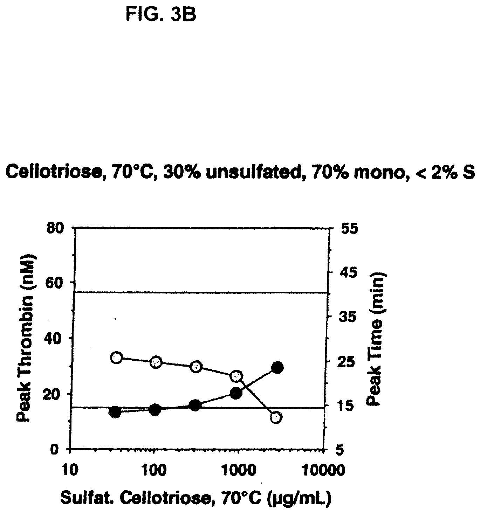

Exemplary NASPs of the invention are substantially non-anticoagulant at the aforementioned concentrations as thrombin generation is above the hemophilia plasma level as measured in a standard asssay such as calibrated automatic thrombography (CAT), examples of which are set forth herein. See, *e.g*., Example 2 and **FIG. 3****.** Both the procoagulant effect and peak thrombin can be measured by CAT.

Exemplary NASPs of the invention are sulfated or sulfonated polysaccharides with procoagulant activity and anticoagulant activity. The anticoagulant properties of potential NASPs are determined using the activated partial thromboplastin time (aPTT) clotting assays Non-anticoagulant sulfated or sulfonated polysaccharides exhibit no more than one-third, and preferably less than one-tenth, the anticoagulant activity (measured by statistically significant increase in clotting time) of unfractionated heparin.

The ability of NASPs to promote clotting and reduce bleeding is readily determined using various in vitro clotting and global hemostatic assays (*e.g*., dPT, aPTT, CAT and ROTEM assays) and in vivo bleeding models (*e.g*., tail snip and/or transection or cuticle bleeding time determination in hemophilic mice or dogs). See, *e.g.,* PDR Staff. Physicians' Desk Reference, 2004, Nordfang, et al. (1991) Thromb Haemost., 66:464-467; Anderson, et al. (1976) Thromb. Res., 9:575-580; Welsch, et al. (1991) Thrombosis Research, 64:213-222; Broze, et al. (2001) Thromb Haemost, 85:747-748; Scallan, et al. (2003) Blood, 102:2031-2037; Pijnappels, et al. (1986) Thromb. Haemost., 55:70-73; and Giles, et al. (1 982) Blood, 60:727-730. Clotting assays may be performed in the presence of one or more NASP and one or more blood factors, procoagulants, or other therapeutic agent. For example, one or more factors can be administered in conjunction with one or more NASP, including but not limited to, Factor XI, Factor XII, prekallikrein, HMWK, Factor V, Factor VII, Factor VIII, Factor IX, Factor X, Factor XIII, Factor II, and von Willebrand Factor, tissue factor, Factor VIIa, Factor Va, and Factor Xa, Factor IXa, Factor XIa, Factor XIIa, and VIIIa; and/or one or more therapeutic agent, including but not limited to, APTT reagent, thromboplastin, fibrin, TFPI, Russell's viper venom, micronized silica particles, ellagic acid, sulfatides, and kaolin.

The Examples and the Figures appended hereto confirm that the agents referred to herein as NASPs are truly "non-anticoagulant," *i.e.,* they do not significantly increase clotting times within a selected concentration range. Such compounds can be used in the methods and compositions of the present invention provided that any anticoagulant activity that they may exhibit only appears at concentrations significantly above the concentration at which they exhibit procoagulant activity. The ratio of the concentration at which undesired anticoagulant properties occur to the concentration at which desired procoagulant activities occur is referred to as the procoagulant index (*e.g*., therapeutic index) for the NASP in question. An exemplary therapeutic index for NASPs of the present invention is about 3, 5, 10, 30, 100, 300, 1000 or more. In an exemplary embodiment, the aPTT:CAT ratio isdetermined using standard methods , wherein "aPTT" is activated partial thromboplastin time and "CAT" is calibrated automatic thrombography. For example, in case of the CAT assay, the EC50 is derived from the thrombin generation (CAT) curve. In case of the aPTT assay, the concentration at which clotting time is 50% increased over a normal plasma control is determined. From those two values the aPTT/CAT ratio can be calculated.

In various embodiments, the invention provides NASPs that include at least about 5%, at least about 10%, at least about 15%, or at least about 20% sulfur. In an exemplary embodiment, this amount of sulfur is determined by elemental analysis of the NASP.

In exemplary embodiments, the NASPs of the invention have an EC50, as measured in a CAT assay of from about 0.001 µg/mL to about 30 µg/mL of plasma, *e.g*., from about 0.01 µg/mL to about 10 µg/mL, *e.g.,* from about 0.05 µg/mL to about 5 µg/mL. In various embodiments, the NASP of the invention is not substantially anticoagulant at its EC50. An exemplary NASP of the invention is not substantially anticoagulant at a concentration of up to about 1.1x, 1.3x. 1.6x. 1.9x, 2.5x, 3x, 3.5x, 4.0x, 5x, 10x, 20x, 30x, 40x or 50x its EC50.

In various embodiments, the invention provides a NASP, which is a member selected from cellotriose, cellotetraose, cellopentaose, maltotriose, maltotetraose, maltopentaose, xylohexaose, raffinose, melezitose, stachyose, α-cyclodextrin, β-cyclodextrin, γ-cyclodextrin, icodextrin, xylan and 6-carboxyicodextrin, having an amount of sulfur as set forth above. Also provided are NASPs, which are oxidized saccharides (such as oxidized analogs of those saccharides recited above), like 6-carboxy-icodextrin.

### B. Pharmaceutical Formulations

In various embodiments, the NASP of the invention is incorporated into a pharmaceutical formulation. In various embodiments, depending on the desired effects and potency of the NASPs, one or more NASPs may be formulated together. For example, two or more NASPs may be formulated together, such as three or more NASPs and including four or more NASPs. Where more than one NASP is employed, the mass percentage of each NASP in the composition may vary, ranging from 1% or more of the total mass of the composition, such as about 2% or more, such as about 5% or more, such as about 10% or more, such as about 25% or more and including as much as about 50% or more of the total mass of the composition.

In various embodiments, the pharmaceutical formulations of the invention optionally contain one or more pharmaceutically acceptable excipient. Exemplary excipients include, without limitation, carbohydrates, inorganic salts, antimicrobial agents, antioxidants, surfactants, buffers, acids, bases, and combinations thereof. Liquid excipients include water, alcohols, polyols, glycerine, vegetable oils, phospholipids, and surfactants. A carbohydrate such as a sugar, a derivatized sugar such as an alditol, aldonic acid, an esterified sugar, and/or a sugar polymer may be present as an excipient. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol, sorbitol (glucitol), pyranosyl sorbitol, myoinositol, and the like. The excipient can also include an inorganic salt or buffer such as citric acid, sodium chloride, potassium chloride, sodium sulfate, potassium nitrate, sodium phosphate monobasic, sodium phosphate dibasic, and combinations thereof.

A pharmaceutical formulation of the invention can also include an antimicrobial agent for preventing or deterring microbial growth. Non-limiting examples of antimicrobial agents suitable for the present invention include benzalkonium chloride, benzethonium chloride, benzyl alcohol, cetylpyridinium chloride, chlorobutanol, phenol, phenylethyl alcohol, phenylmercuric nitrate, thimersol, and combinations thereof.

An antioxidant can be present in the formulation as well. Antioxidants are used to prevent oxidation, thereby preventing the deterioration of the NASP or other components of the formulation. Suitable antioxidants for use in the formulations of the present invention include, for example, ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

A surfactant can be present as an excipient. Exemplary surfactants include: polysorbates, such as "Tween 20" and "Tween 80", and pluronics such as F68 and F88 (BASF, Mount Olive, N.J.); sorbitan esters; lipids, such as phospholipids such as lecithin and other phosphatidylcholines, phosphatidylethanolamines (although preferably not in liposomal form), fatty acids and fatty esters; steroids, such as cholesterol; chelating agents, such as EDTA; and zinc and other such suitable cations.

Acids or bases can be present as an excipient in the formulation. Non-limiting examples of acids that can be used include those acids selected from the group consisting of hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof. Examples of suitable bases include, without limitation, bases selected from the group consisting of sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumnerate, and combinations thereof.

The amount of the NASP in the formulation will vary depending on a number of factors, but will optimally be a therapeutically effective dose when the formulation is in a unit dosage form (*e.g.,* a pill, or capsule) or container (*e.g.,* a vial or bag). A therapeutically effective dose can be determined experimentally by repeated administration of increasing amounts of the formulation in order to determine which amount produces a clinically desired endpoint.

The amount of any individual excipient in the formulation will vary depending on the nature and function of the excipient and particular needs of the composition. Typically, the optimal amount of any individual excipient is determined through routine experimentation, *i.e.,* by preparing formulations containing varying amounts of the excipient (ranging from low to high), examining the stability and other parameters, and then determining the range at which optimal performance is attained with no significant adverse effects. Generally, however, the excipient(s) is present in the composition in an amount of about 1% to about 99% by weight, preferably from about 5% to about 98% by weight, more preferably from about 15 to about 95% by weight of the excipient, with concentrations less than 30% by weight most preferred. These foregoing pharmaceutical excipients along with other excipients are described in "Remington: The Science & Practice of Pharmacy", 19th ed., Williams & Williams, (1995), the "Physician's Desk Reference", 52nd ed., Medical Economics, Montvale, N.J. (1998), and Kibbe, A. H., Handbook of Pharmaceutical Excipients, 3rd Edition, American Pharmaceutical Association, Washington, D.C., 2000.

In other embodiments the NASP is selected from the group consisting of low molecular weight fragments of the previously listed compounds. In certain embodiments, the formulation may further comprise a pharmaceutically acceptable excipient. In certain embodiments, the formulations may further comprise one or more different NASPs, and/or one or more therapeutic agents, and/or reagents.

The formulations encompass all types of formulations and in particular those that are suited for oral administration or injection. Additional preferred compositions include those for oral, ocular, or localized delivery.

The pharmaceutical formulations herein can also be housed in an infusion bag, a syringe, an implantation device, or the like, depending upon the intended mode of delivery and use. Preferably, the NASP compositions described herein are in unit dosage form, meaning an amount of composition of the invention appropriate for a single dose, in a premeasured or pre-packaged form.

The formulations can conveniently be presented in unit dosage form and can be prepared by any of the methods well known in the art of pharmacy. Exemplary methods include the step of bringing into association a compound or a pharmaceutically acceptable salt or solvate thereof ("active ingredient") with the carrier which constitutes one or more accessory ingredients. In general, the formulations are prepared by uniformly and intimately bringing into association the compound of the invention with liquid carriers or finely divided solid carriers or both and then, if necessary, shaping the product into the desired formulation. Oral formulations are well known to those skilled in the art, and general methods for preparing them are found in any standard pharmacy school textbook, for example, Remington: The Science and Practice of Pharmacy, A.R. Gennaro, ed. (1995), the entire disclosure of which is incorporated herein by reference.

In various embodiments, the invention provides a unit dosage formulation including one or more NASP of the invention. In an exemplary embodiment, the unit dosage formulation includes a therapeutically effective dosage of a NASP, preferably sufficient to induce a clinically detectable and, preferably, a clinically meaningful alteration in the clotting status of the subject to whom the single dosage formulation is administered. In exemplary embodiments, the amount of a NASP of the invention in the formulation ranges from an amount sufficient to provide a dosage of about 0.01 mg/kg to about 200 mg/kg of a NASP. In various embodiments, the amount of a NASP of the invention is sufficient to provide a dosage of from about 0.01 mg/kg to about 20 mg/kg, *e.g*., from about 0.02 mg/kg to about 2 mg/kg. The amount of compound in the unit dosage formulation will depend on the potency of the specific NASP and the magnitude or procoagulant effect desired and the route of administration.

Exemplary unit dosage formulations are those containing an effective dose, or an appropriate fraction thereof, of the active ingredient, or a pharmaceutically acceptable salt thereof. A prophylactic or therapeutic dose typically varies with the nature and severity of the condition to be treated and the route of administration. The dosage, and perhaps the dosing frequency, will also vary according to the age, body weight and response of the individual patient. In general, for the compounds of the invention, the total dose in a unit dosage form of the invention ranges from about 1 mg to about 7000 mg, *e.g.,* from about 1 mg to about 500 mg, *e.g.,* from about 10 mg to about 200 mg, *e.g.,* from about 20 mg to about 100 mg, *e.g.,* from about 20 mg to about 80 mg, *e.g.,* from about 20 mg to about 60 mg. In some embodiments, the amount of a NASP of the invention in a unit dosage form ranges from about 50 mg to about 500 mg, *e.g.,* from about 100 mg to about 500 mg.

The NASP compositions herein may optionally be in combination with one or more additional agents, such as hemostatic agents, blood factors, or other medications used to treat a subject for a condition or disease. In various embodiments, the invention provides combination preparations including one or more blood factors such as Factor XI, Factor XII, prekallikrein, HMWK, Factor V, Factor VII, Factor VIII, Factor IX, Factor X, Factor XIII, Factor II, Factor VIIa, and von Willebrand Factor. NASP compositions may also include other procoagulants, such as an activator of the intrinsic coagulation pathway, including but not limited to, Factor Xa, Factor IXa, Factor XIa, Factor XIIa, and VIIIa, prekallikrein, and HMWK; or and activator of the extrinsic coagulation pathway, including but not limited to, tissue factor, Factor VIIa, Factor Va, and Factor Xa. NASP compositions may include naturally occurring, synthetic, or recombinant coagulation factors or fragments, variants or covalently modified derivatives thereof that retain biological activity (*i.e.,* promote clotting). Alternatively, such agents can be contained in a separate composition from the NASP and coadministered concurrently, before, or after the NASP composition of the invention.

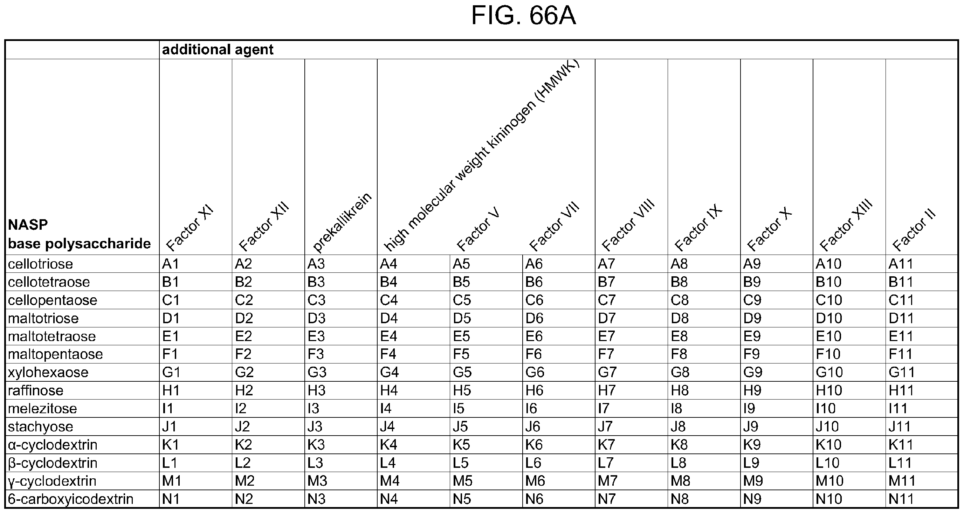

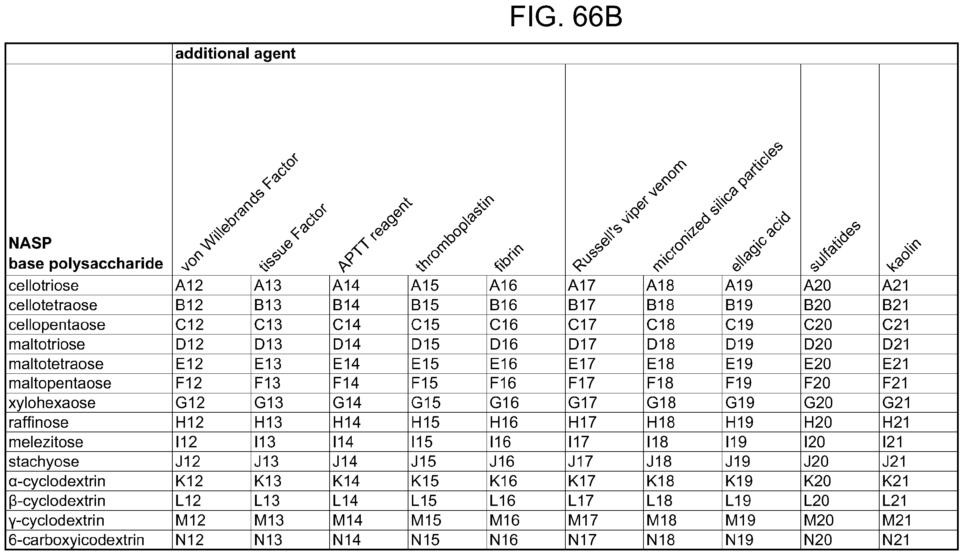

Exemplary combinations of certain NASPs (based on their base polysaccharide) with additional agents are shown in **FIG. 66A-B.** Each combination therein is identified by a capital letter (referring to a type of NASP) followed by a number (referring to the additional agent). For example, "C5" refers to the combination of a NASP having a cellopentaose base polysaccharide with Factor V.

The individual components of such combinations may be administered either simultaneously or sequentially in a unit dosage form. The unit dosage form may be a single or multiple unit dosage form. In an exemplary embodiment, the invention provides a combination in a single unit dosage form. An example of a single unit dosage form is a capsule wherein both the compound of the invention and the additional therapeutic agent are contained within the same capsule. In an exemplary embodiment, the invention provides a combination in a two unit dosage form. An example of a two unit dosage form is a first capsule which contains the compound of the invention and a second capsule which contains the additional therapeutic agent. Thus the term 'single unit' or 'two unit' or 'multiple unit' refers to the object which the patient ingests, not to the interior components of the object. Appropriate doses of known therapeutic agents will be readily appreciated by those skilled in the art.

In various embodiments, the NASP compositions herein may, when intended for oral administration, optionally include one or more permeation enhancer. Appropriate permeation enhancers and their use with procoagulants, such as those provided by the present invention are disclosed in U.S. Provisional Patent Application No. 61/509,514, filed July 19, 2011, titled "Absorption Enhancers as Additives to Improve the Oral Formulation of Non-Anticoagulant Sulfated Polysaccharides". In some embodiments the permeation enhancer is a gastrointestinal epithelial barrier permeation enhancer. Depending on the physiology of the subject, the phrase "gastrointestinal epithelial" as used herein, refers to the epithelial tissue of the digestive tract, such as the stomach and intestinal tract (*e.g*., duodenum, jejunum, ileum), and may further include other structures which participate in the gastrointestinal functions of the body including the lower part of the esophagus, the rectum and the anus. In various embodiments, compositions of the invention include a procoagulant amount of a NASP in combination with a gastrointestinal epithelial barrier permeation enhancer. Amounts of permeation enhancer of use in this invention are generally identical to those set forth in above-referenced U.S. Provisional Patent Application No. 61/509,514. Similarly, in exemplary embodiments, appropriate amounts of an NASP are identical to those amounts set forth for NASPs in the above-referenced application. In various embodiments, compositions of the invention include a combination of a procoagulant amount of a NASP with a gastrointestinal epithelial barrier permeation enhancer and a blood coagulation factor. Exemplary amounts of NASP and a second agent, e.g, a blood coagulation factor, are set forth herein. Gastrointestinal epithelial barrier permeation enhancers include compounds that, when orally administered, increase the amount of NASP that is absorbed by the gastrointestinal system. Furthermore, gastrointestinal permeation enhancers may also accelerate the initiation (*i.e.,* reducing the amount time for absorption to begin) of NASP absorption through the gastrointestinal epithelium as well as accelerate the overall rate of transport of the NASP across the gastrointestinal epithelium of the subject (*i.e.,* reducing the amount of time for NASP absorption by the gastrointestinal system to be complete). In embodiments of the invention, gastrointestinal epithelial barrier permeation enhancers may vary, depending on the particular blood coagulation disorder, the physiology of the subject and the desired enhancement of absorption by the gastrointestinal system. In some embodiments, gastrointestinal epithelial barrier permeation enhancers are tight junction modulators. The term "tight junction" is employed in its conventional sense to refer to the closely associated cellular areas where membranes of adjacent cells are joined together. In embodiments of the invention, tight junction modulators may include, but are not limited to enzymes, bile acids, polysaccharides, fatty acids and salts thereof and any combination thereof.

In an exemplary embodiment of the invention the invention provides a pharmaceutical formulation comprising a) a compound of the invention; b) an additional therapeutic agent and c) a pharmaceutically acceptable excipient. In an exemplary embodiment, the pharmaceutical formulation is a unit dosage form. In an exemplary embodiment, the pharmaceutical formulation is a single unit dosage form. In an exemplary embodiment, the pharmaceutical formulation is a two unit dosage form. In an exemplary embodiment, the pharmaceutical formulation is a two unit dosage form comprising a first unit dosage form and a second unit dosage form, wherein the first unit dosage form includes a) a compound of the invention and b) a first pharmaceutically acceptable excipient; and the second unit dosage form includes c) an additional therapeutic agent and d) a second pharmaceutically acceptable excipient.

It should be understood that in addition to the ingredients particularly mentioned above, the formulations of this invention can include other agents conventional in the art having regard to the type of formulation in question, for example those suitable for oral administration can include flavoring agents.

Formulations of the present invention suitable for oral administration can be presented as discrete units such as capsules (*e.g*., soft-gel capsules), cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient can also be presented as a bolus, electuary or paste.

A tablet can be made by compression or molding, optionally using one or more accessory ingredients. Compressed tablets can be prepared by compressing in a suitable machine the active ingredient in a free-flowing form such as a powder or granules, optionally mixed with a binder, lubricant, inert diluent, lubricating, surface active or dispersing agent. Molded tablets can be made by molding in a suitable machine a mixture of the powdered compound moistened with an inert liquid diluent. The tablets can optionally be coated or scored and can be formulated so as to provide sustained, delayed or controlled release of the active ingredient therein. Oral and parenteral sustained release drug delivery systems are well known to those skilled in the art, and general methods of achieving sustained release of orally or parenterally administered drugs are found, for example, in Remington: The Science and Practice of Pharmacy, pages 1660-1675 (1995), the disclosure of which is incorporated herein by reference.

In an exemplary embodiment, the invention provides a unit dosage formulation of a NASP of the invention in a form appropriate for administration by injection (e.g., infusion). The unit dosage formulation can be diluted with an appropriate pharmaceutically acceptable diluent shortly prior to use, or it can be packaged as a diluted unit dosage for infusion. Suitable forms for dilution prior to injection include, *e.g*., powders or lyophilates that can be reconstituted with a solvent prior to use, as well as ready for injection solutions or suspensions, dry insoluble compositions for combination with a vehicle prior to use, and emulsions and liquid concentrates for dilution prior to administration. Examples of suitable diluents for reconstituting solid compositions prior to injection include bacteriostatic water for injection, dextrose 5% in water, phosphate buffered saline, Ringer's solution, saline, sterile water, deionized water, and combinations thereof. With respect to liquid pharmaceutical compositions, solutions and suspensions are envisioned. In general, the amounts discussed above as appropriate for oral unit dosage forms are applicable to the injectable unit dosage as well.

In a further exemplary embodiment, the invention provides the injectable unit dosage formulation and a device for administration of the unit dosage formulation by injection (*e.g*., infusion). In various embodiments, the device is an infusion bag. In an example of this embodiment, the invention provides an infusion bag, or similar device, into which the unit dosage formulation is pre-charged diluted or in a form appropriate for dilution.

Sulfated or sulfonated polysaccharides with potential NASP activity (*i.e.,* procoagulant activity) include, but are not limited to, sulfated or sulfonated polysaccharides in which the base polysaccharide is selected from cellotriose, cellotetraose, cellopentaose, maltotriose, maltotetraose, maltopentaose, xylohexaose, raffinose, melezitose, stachyose, α-cyclodextrin, β-cyclodextrin, γ-cyclodextrin, icodextrin, and 6-carboxyicodextrin.

### C. NASPs as Promoters of Clotting

In exemplary embodiments, the invention provides methods for regulating hemostasis that, paradoxically, utilize sulfated or sulfonated polysaccharides, such as heparin-like sulfated or sulfonated polysaccharides to promote clotting. Selected sulfated or sulfonated polysaccharides described herein are essentially devoid of anticoagulant activity, or exhibit clot-promoting activity at concentrations lower, preferably significantly lower, than the concentration at which they exhibit anticoagulant activity, and are hence denoted "non-anticoagulant sulfated or sulfonated polysaccharides".

NASPs for use in the methods of the invention are sulfated or sulfonated polysaccharides that have procoagulant activity. The properties of potential NASPs are determined using TFPI-dilute prothrombin time (dPT) or activated partial thromboplastin time (aPTT) clotting assays. Procoagulant activity of NASPs of the invention can be determined by global hemostatic assay like thrombin generation or thromboelostography at low TF. Non-anticoagulant sulfated or sulfonated polysaccharides exhibit no more than one-third, and preferably less than one-tenth, the anticoagulant activity (measured by increase in clotting time) of unfractionated heparin.

As shown in the Examples herein, NASPs of the invention promote clotting of plasma and whole blood. In the experiments disclosed herein, certain candidate NASPs are shown in clotting assays to demonstrate at least about a three-fold, at least about a five-fold or at least about a ten-fold lower anticoagulant activity as compared to heparin. These results indicate that systemic administration of select NASPs represents a unique approach for regulating hemostasis in bleeding disorders.

Thus, in exemplary embodiments, the invention relates to the use of NASPs to control hemostasis in subjects with bleeding disorders, including congenital coagulation disorders, acquired coagulation disorders, and trauma induced hemorrhagic conditions.

In one aspect, the invention provides a method for treating a subject in need of enhanced blood coagulation comprising administering a therapeutically effective amount of a composition comprising a non-anticoagulant sulfated or sulfonated polysaccharide (NASP) of the invention to the subject. In certain embodiments, the invention provides a method for treating a subject having a bleeding disorder comprising administering a therapeutically effective amount of a composition comprising a NASP of the invention to the subject.

In an exemplary embodiment, the composition is of use in a method for treating a subject in need of enhanced blood coagulation. The method comprises administering a therapeutically effective amount of a composition comprising a non-anticoagulant sulfated or sulfonated polysaccharide to the subject.

In an exemplary embodiment, the invention provides a method in which a NASP is coadministered with one or more different NASPs and/or in combination with one or more other therapeutic agent(s).

In certain embodiments, a NASP of the invention is administered to a subject to treat a bleeding disorder selected from the group consisting of hemophilia A, hemophilia B, von Willebrand disease, idiopathic thrombocytopenia, a deficiency of one or more coagulation factors (*e.g*., Factor XI, Factor XII, prekallikrein, and HMWK), a deficiency of one or more factors associated with clinically significant bleeding (*e.g*., Factor V, Factor VII, Factor VIII, Factor IX, Factor X, Factor XIII, Factor II (hypoprothrombinemia), and von Willebrand Factor), a vitamin K deficiency, a disorder of fibrinogen (*e.g*., afibrinogenemia, hypofibrinogenemia, and dysfibrinogenemia), an alpha2-antiplasmin deficiency, and excessive bleeding such as caused by liver disease, renal disease, thrombocytopenia, platelet dysfunction, hematomas, internal hemorrhage, hemarthroses, surgery, trauma, hypothermia, menstruation, and pregnancy.

In certain embodiments, a NASP is administered to a subject to treat a congenital coagulation disorder or an acquired coagulation disorder caused by a blood factor deficiency. The blood factor deficiency may be caused by deficiencies of one or more factors (*e.g*., Factor V, Factor VII, Factor VIII, Factor IX, Factor XI, Factor XII, Factor XIII, and von Willebrand Factor).

In certain embodiments, the subject having a bleeding disorder is administered a therapeutically effective amount of a composition comprising a NASP in combination with another therapeutic agent as set forth above.

In another embodiment, the invention provides a method for reversing the effects of an anticoagulant in a subject. The method comprises administering a therapeutically effective amount of a composition comprising a non-anticoagulant sulfated or sulfonated polysaccharide (NASP) of the invention to the subject. In certain embodiments, the subject may have been treated with an anticoagulant including, but not limited to, heparin, a coumarin derivative, such as warfarin or dicumarol, tissue TFPI, antithrombin III, lupus anticoagulant, nematode anticoagulant peptide (NAPc2), active-site blocked Factor VIIa (Factor VIIai), Factor IXa inhibitors, Factor Xa inhibitors, including fondaparinux, idraparinux, DX-9065a, and razaxaban (DPC906), inhibitors of Factors Va and VIIIa, including activated protein C (APC) and soluble thrombomodulin, thrombin inhibitors, including hirudin, bivalirudin, argatroban, and ximelagatran. In certain embodiments, the anticoagulant in the subject may be an antibody that binds a coagulation factor, including but not limited to, an antibody that binds to Factor V, Factor VII, Factor VIII, Factor IX, Factor X, Factor XIII, Factor II, Factor XI, Factor XII, von Willebrand Factor, prekallikrein, or HMWK.

In certain embodiments, a NASP is coadministered with one or more different NASP(s) and/or in combination with one or more other therapeutic agents for reversing the effects of an anticoagulant in a subject. For example, the subject may be administered a therapeutically effective amount of a composition comprising a NASP of the invention and one or more therapeutic agents such as those set forth above. Therapeutic agents used in combination with a NASP to reverse the effects of an anticoagulant in a subject can be administered in the same or different compositions and concurrently, before, or after administration of the NASP.

In another aspect, the invention provides a method for treating a subject undergoing a surgical or invasive procedure in which improved blood clotting is desirable. The method includes administering a therapeutically effective amount of a composition comprising a non-anticoagulant sulfated or sulfonated polysaccharide (NASP) of the invention to the subject. In certain embodiments, the NASP can be coadministered with one or more different NASPs and/or in combination with one or more other therapeutic agents such as those set forth above. Therapeutic agents used to treat a subject undergoing a surgical or invasive procedure can be administered in the same or different compositions and concurrently, before, or after administration of the NASP.

In another aspect, the invention provides a method of inhibiting TFPI activity in a subject, the method comprising administering a therapeutically effective amount of a composition comprising a NASP of the invention to the subject.

In another aspect, the invention provides a method of inhibiting TFPI activity in a biological sample, the method comprising combining the biological sample (*e.g*., blood or plasma) with a sufficient amount of a non-anticoagulant sulfated or sulfonated polysaccharide (NASP) of the invention to inhibit TFPI activity.

In another aspect, the invention provides a method of measuring acceleration of clotting by a NASP of the invention in a biological sample, the method comprising:
a) combining the biological sample with a composition comprising the NASP,
b) measuring the clotting time of the biological sample,
c) comparing the clotting time of the biological sample to the clotting time of a corresponding biological sample not exposed to the NASP, wherein a decrease in the clotting time of the biological sample exposed to the NASP, if observed, is indicative of a NASP that accelerates clotting.

In certain embodiments, one or more different NASPs of the invention and/or therapeutic agents, and/or reagents is/are added to the biological sample for measurements of clotting time. For example, one or more factors can be added, including but not limited to, Factor XI, Factor XII, prekallikrein, HMWK, Factor V, Factor VII, Factor VIII, Factor IX, Factor X, Factor XIII, Factor II, and von Willebrand Factor, tissue factor, Factor VIIa, Factor Va, and Factor Xa, Factor IXa, Factor XIa, Factor XIIa, and VIIIa; and/or one or more reagents, including but not limited to, APTT reagent, tissue factor, thromboplastin, fibrin, TFPI, Russell's viper venom, micronized silica particles, ellagic acid, sulfatides, and kaolin.

### D. Administration

In an exemplary embodiment, at least one therapeutically effective cycle of treatment with a NASP of the invention is administered to a subject. "Therapeutically effective cycle of treatment" refers to a cycle of treatment that, when administered, brings about a positive therapeutic response in an individual for a bleeding disorder. Of particular interest is a cycle of treatment with a NASP that improves hemostasis. For example, one or more therapeutically effective cycles of treatment may increase clotting (*e.g.,* the rate of clotting) as determined by clotting and global hemostatic assays (*e.g*., CAT, aPTT, described in detail below) by about 1% or more, such as about 5% or more, such as about 10% or more, such as about 15% or more, such as about 20% or more, such as about 30% or more, such as about 40% or more, such as about 50% or more, such as about 75% or more, such as about 90% or more, such as about 95% or more, including increasing the rate of blood clot formation by about 99% or more. In other instances, one or more therapeutically effective cycles of treatment may increase the rate of blood clot formation by about 1.5-fold or more, such as about 2-fold or more, such as about 5-fold or more, such as about 10-fold or more, such as about 50-fold or more, including increasing the rate of blood clot formation by about 100-fold or more. In some embodiments, subjects treated by methods of the invention exhibit a positive therapeutic response. As used herein, "positive therapeutic response" means that the individual undergoing treatment according to the invention exhibits an improvement in one or more symptoms of a bleeding disorder, including such improvements as shortened blood clotting times and reduced bleeding and/or reduced need for factor replacement therapy.

In certain embodiments, multiple therapeutically effective doses of compositions comprising one or more NASPs and/or other therapeutic agents, such as hemostatic agents, blood factors, or other medications are administered. The compositions of the present invention are typically, although not necessarily, administered orally, via injection (subcutaneously, intravenously or intramuscularly), by infusion, or locally. The pharmaceutical preparation can be in the form of a liquid solution or suspension immediately prior to administration, but may also take another form such as a syrup, cream, ointment, tablet, capsule, powder, gel, matrix, suppository, or the like. Additional modes of administration are also contemplated, such as pulmonary, rectal, transdermal, transmucosal, intrathecal, pericardial, intraarterial, intracerebral, intraocular, intraperitoneal, and so forth. The pharmaceutical compositions comprising NASPs and other agents may be administered using the same or different routes of administration in accordance with any medically acceptable method known in the art.

In an exemplary embodiment, a composition of the invention is used for localized delivery of a NASP of the invention, for example, for the treatment of bleeding as a result of a lesion, injury, or surgery. The preparations according to the invention are also suitable for local treatment. For example, a NASP may be administered by injection at the site of bleeding or in the form of a solid, liquid, or ointment, preferably via an adhesive tape or a wound cover. Suppositories, capsules, in particular gastric-juice-resistant capsules, drops or sprays may also be used. The particular preparation and appropriate method of administration are chosen to target the site of bleeding.

In another embodiment, the pharmaceutical compositions comprising NASPs and/or other agents are administered prophylactically, *e*.*g.*, before a planned surgery. Though of general utility, such prophylactic uses are of particular value for subjects with known preexisting blood coagulation disorders.

In another embodiment of the invention, the pharmaceutical compositions comprising NASPs and/or other agents, are in a sustained-release formulation, or a formulation that is administered using a sustained-release device. Such devices are well known in the art, and include, for example, transdermal patches, and miniature implantable pumps that can provide for drug delivery over time in a continuous, steady-state fashion at a variety of doses to achieve a sustained-release effect with a non-sustained-release pharmaceutical composition.

A prophylactic or therapeutic dose typically varies with the nature and severity of the condition to be treated and the route of administration. The dosage, and perhaps the dosing frequency, will also vary according to the age, body weight and response of the individual patient. In general, the total daily dose (in single or divided doses) ranges from about 1 mg per day to about 7000 mg per day, *e.g*., about 1 mg per day to about 500 mg per day, *e.g.,* from about 10 mg per day to about 200 mg per day, *e.g.,* from about 20 mg to about 100 mg, *e.g.,* about 20 mg to about 80 mg, *e.g.,* about 20 mg to about 60 mg. In some embodiments, the total daily dose can range from about 50 mg to about 500 mg per day, *e.g.,* about 100 mg to about 500 mg per day. It is further recommended that children, patients over 65 years old, and those with impaired renal or hepatic function, initially receive low doses and that the dosage is titrated based on individual physiological responses and/or pharmacokinetics. It can be necessary to use dosages outside these ranges in some cases, as will be apparent to those in the art. Further, it is noted that the clinician or treating physician knows how and when to interrupt, adjust or terminate therapy in conjunction with an individual patient's response.

The invention also provides a method for administering a conjugate comprising a NASP of the invention as provided herein to a patient suffering from a condition that is responsive to treatment with a NASP contained in the conjugate or composition. The method comprises administering, via any of the herein described modes, a therapeutically effective amount of the conjugate or drug delivery system, preferably provided as part of a pharmaceutical composition. The method of administering may be used to treat any condition that is responsive to treatment with a NASP. More specifically, the compositions herein are effective in treating bleeding disorders, including Hem A, Hem B, von Willebrand disease, idiopathic thrombocytopenia, a deficiency of one or more coagulation factors, such as Factor XI, Factor XII, prekallikrein, and HMWK, a deficiency of one or more factors associated with clinically significant bleeding, such as Factor V, Factor VII, Factor VIII, Factor IX, Factor X, Factor XIII, Factor II (hypoprothrombinemia), and von Willebrand Factor, a vitamin K deficiency, a disorder of fibrinogen, including afibrinogenemia, hypofibrinogenemia, and dysfibrinogenemia, an alpha2-antiplasmin deficiency, and excessive bleeding such as caused by liver disease, renal disease, thrombocytopenia, platelet dysfunction, hematomas, internal hemorrhage, hemarthroses, surgery, trauma, hypothermia, menstruation, and pregnancy.

In exemplary embodiments, the NASP of the invention is administered orally, intraperitoneally (i.p.), intravenously (i.v.) or through a combination of the administration modes.

Those of ordinary skill in the art will appreciate which conditions a specific NASP can effectively treat. The actual dose to be administered will vary depending upon the age, weight, and general condition of the subject as well as the severity of the condition being treated, the judgment of the health care professional, and conjugate being administered. Therapeutically effective amounts can be determined by those skilled in the art, and are adjusted to the particular requirements of each particular case.

A prophylactic or therapeutic dose typically varies with the nature and severity of the condition to be treated and the route of administration. The dosage, and perhaps the dosing frequency, will also vary according to the age, body weight and response of the individual patient. In general, for the compounds of the invention, the total dose in a unit dosage form of the invention ranges from about 1 mg to about 7000 mg , *e.g*., about 1 mg to about 500 mg, *e.g.,* from about 10 mg to about 200 mg, *e.g.,* from about 20 mg to about 100 mg, *e.g.,* about 20 mg to about 80 mg, *e.g.,* about 20 mg to about 60 mg. In some embodiments, the amount of a NASP of the invention in a unit dosage form ranges from about 50 mg to about 500 mg, *e.g*., about 100 mg to about 500 mg.

Generally, a therapeutically effective amount will range from about 0.01 mg/kg to about 200 mg/kg of a NASP daily, more preferably from about 0.01 mg/kg to about 20 mg/kg daily, even more preferably from about 0.02 mg/kg to about 2 mg/kg daily. In an exemplary embodiment, such doses are in the range of from about 0.01 to about 50 mg/kg four times a day (QID), from about 0.01 to about 10 mg/kg QID, from about 0.01 to about 2 mg/kg QID, from about 0.01 to about 0.2 mg/kg QID, from about 0.01 to about 50 mg/kg three times a day (TID), from about 0.01 to about 10 mg/kg TID, from about 0.01 to about 2 mg/kg TID, from about 0.01 to about 0.2 mg/kg TID, from about 0.01 to about 200 mg/kg twice daily from about 0.01 to about 100 mg/kg twice daily (BID), from about 0.01 to about 10 mg/kg BID, from about 0.01 to about 2 mg/kg BID, or from about 0.01 to about 0.2 mg/kg BID. The amount of compound administered depends on the severity of the subject's condition, potency of the specific NASP and the magnitude or procoagulant effect desired and the route of administration.

A NASP (*e.g*., provided as part of a pharmaceutical preparation) of the invention can be administered alone or in combination with other NASPs or therapeutic agents, such as hemostatic agents, blood factors, or other medications used to treat a particular condition or disease according to a variety of dosing schedules depending on the judgment of the clinician, needs of the patient, and so forth. The specific dosing schedule is known by those of ordinary skill in the art or can be determined experimentally using routine methods. Exemplary dosing schedules include, without limitation, administration five times a day, four times a day, three times a day, twice daily, once daily, three times weekly, twice weekly, once weekly, twice monthly, once monthly, and any combination thereof. Preferred compositions are those requiring dosing no more than once a day.

A NASP of the invention can be administered prior to, concurrent with, or subsequent to other agents. If provided at the same time as other agents, the NASP can be provided in the same or in a different composition. Thus, NASPs and other agents can be presented to the individual by way of concurrent therapy. By "concurrent therapy" is intended administration to a subject such that the therapeutic effect of the combination of the substances is caused in the subject undergoing therapy. For example, concurrent therapy may be achieved by administering a dose of a pharmaceutical composition comprising a NASP and a dose of a pharmaceutical composition comprising at least one other agent, such as a hemostatic agent or coagulation factor, including, for example, one or more blood factors such as Factor XI, Factor XII, prekallikrein, HMWK, Factor V, Factor VII, Factor VIII, Factor IX, Factor X, Factor XIII, Factor II, Factor VIIa, and von Willebrand Factor. NASP compositions may also include other procoagulants, such as an activator of the intrinsic coagulation pathway, including but not limited to, Factor Xa, Factor IXa, Factor XIa, Factor XIIa, and VIIIa, prekallikrein, and HMWK; or and activator of the extrinsic coagulation pathway, including but not limited to, tissue factor, Factor VIIa, Factor Va, and Factor Xa, which in combination comprise a therapeutically effective dose, according to a particular dosing regimen. Similarly, one or more NASPs and therapeutic agents can be administered in at least one therapeutic dose. When the NASPs and other therapeutic agent(s) are administered as separate pharmaceutical compositions, administration of the separate pharmaceutical compositions can be performed simultaneously or at different times (*i.e.,* sequentially, in either order, on the same day, or on different days), so long as the therapeutic effect of the combination of these substances is caused in the subject undergoing therapy.

### F. Applications

In one embodiment, NASPs of the invention are used in the methods of the invention for improving hemostasis in treating bleeding disorders, particularly those associated with deficiencies of coagulation factors or for reversing the effects of anticoagulants in a subject. NASPs may be administered to a subject to treat bleeding disorders, including congenital coagulation disorders, acquired coagulation disorders, and hemorrhagic conditions induced by trauma. Examples of bleeding disorders that may be treated with NASPs include, but are not limited to, Hem A, Hem B, von Willebrand disease, idiopathic thrombocytopenia, a deficiency of one or more coagulation factors, such as Factor XI, Factor XII, prekallikrein, and HMWK, a deficiency of one or more factors associated with clinically significant bleeding, such as Factor V, Factor VII, Factor VIII, Factor IX, Factor X, Factor XIII, Factor II (hypoprothrombinemia), and von Willebrand Factor, a vitamin K deficiency, a disorder of fibrinogen, including afibrinogenemia, hypofibrinogenemia, and dysfibrinogenemia, an alpha2-antiplasmin deficiency, and excessive bleeding such as caused by liver disease, renal disease, thrombocytopenia, platelet dysfunction, hematomas, internal hemorrhage, hemarthroses, surgery, trauma, hypothermia, menstruation, and pregnancy. In certain embodiments, NASPs are used to treat congenital coagulation disorders including hemophilia A, hemophilia B, and von Willebrand disease. In other embodiments, NASPs are used to treat acquired coagulation disorders, including deficiencies of Factor VIII, von Willebrand Factor, Factor IX, Factor V, Factor XI, Factor XII and Factor XIII, particularly disorders caused by inhibitors or autoimmunity against blood coagulation factors, or haemostatic disorders caused by a disease or condition that results in reduced synthesis of coagulation factors.

The needs of the patient will depend on the particular bleeding disorder being treated. For example, a NASP of the invention may be administered to treat a chronic condition (*e.g*., a congenital or acquired coagulation factor deficiency) in multiple doses over an extended period. Alternatively, a NASP may be administered to treat an acute condition (*e.g*., bleeding caused by surgery or trauma, or factor inhibitor/autoimmune episodes in subjects receiving coagulation replacement therapy) in single or multiple doses for a relatively short period, for example one to two weeks. In addition, NASP therapy may be used in combination with other hemostatic agents, blood factors, and medications as set forth previously. In addition, transfusion of blood products may be necessary to replace blood loss in subjects experiencing excessive bleeding, and in cases of injury, surgical repair may be appropriate to stop bleeding.

The invention also provides a method for reversing the effects of an anticoagulant in a subject. The method includes administering a therapeutically effective amount of a composition comprising a NASP of the invention to the subject. In certain embodiments, the subject may have been treated with an anticoagulant including, but not limited to, heparin, a coumarin derivative, such as warfarin or dicumarol, TFPI, AT III, lupus anticoagulant, nematode anticoagulant peptide (NAPc2), active-site blocked Factor VIIa (Factor VIIai), Factor IXa inhibitors, Factor Xa inhibitors, including fondaparinux, idraparinux, DX-9065a, and razaxaban (DPC906), inhibitors of Factors Va and VIIIa, including activated protein C (APC) and soluble thrombomodulin, thrombin inhibitors, including hirudin, bivalirudin, argatroban, and ximelagatran. In certain embodiments, the anticoagulant in the subject may be an antibody that binds a coagulation factor, including but not limited to, an antibody that binds to Factor V, Factor VII, Factor VIII, Factor IX, Factor X, Factor XIII, Factor II, Factor XI, Factor XII, von Willebrand Factor, prekallikrein, or HMWK.

In another aspect, the invention provides a method for improving clotting in a subject undergoing a surgical or invasive procedure, the method comprising administering a therapeutically effective amount of a composition comprising a non-anticoagulant sulfated or sulfonated polysaccharide (NASP) of the invention to the subject. In certain embodiments, the NASP can be administered alone or coadministered with one or more different NASPs and/or in combination with one or more other therapeutic agents as set forth previously to the subject undergoing a surgical or invasive procedure. For example, the subject may be administered a therapeutically effective amount of one or more factors selected from the group consisting of Factor XI, Factor XII, prekallikrein, HMWK, Factor V, Factor VII, Factor VIII, Factor IX, Factor X, Factor XIII, Factor II, Factor VIIa, and von Willebrand Factor. Treatment may further comprise administering a procoagulant, such as an activator of the intrinsic coagulation pathway, including Factor Xa, Factor IXa, Factor XIa, Factor XIIa, and Factor VIIIa, prekallikrein, and HMWK; or an activator of the extrinsic coagulation pathway, including tissue factor, Factor VIIa, Factor Va, and Factor Xa.

In addition to the uses set forth above, the compounds of the invention find use in a variety of other treatment modalities. For example, in one embodiment, the compounds of the invention are of use to treat interstitial cystitis (see, *e.g.,* Urology, (2000, Dec.) 164(6):2119-2125; Urology, (1999, June) 53(6):1133-1139; International Congress Series, (2001, Dec.) 1223:227-237; Urology, (2008, Jan.) 179(1):177-185; European Urology Supplements, (2003, Sept.) 2(4):14-16; Urology, (2011, Sept.) 78(3):S210-S211; European Urology Supplements, (2011, Oct.) 10(6)451-459; Urology, (2011, April) 185(4):e384).

In various embodiments, the compounds of the invention also find use as antiinflammatory agents, and in the treatment and prevention of neurodegenerative disorders (see, *e.g.,* Food and Chemical Toxicology, (2011, Aug.) 49(8):1745-1752; Food and Chemical Toxicology, (2011, Sept.) 49(9):2090-2095; Biochimica et Biophysica Acta (BBA) - Proteins & Proteomics, (2003, Sept.) 1651(1-2)).

In an exemplary embodiment, compounds of the invention also find use for their anti-cancer activity (see, *e.g.,* Carbohydrate Polymers, (2012, Jan. 4) 87(1, 4):186-194; Carbohydrate Polymers, (2010, May 23) 81(1, 23):41-48; Carbohydrate Polymers, (2012, Jan. 4) 87(1, 4):186-194; International Journal of Biological Macromolecules, (2011, Oct. 1) 49(3, 1):331-336; Advances in Food and Nutrition Research, (2011) 64:391-402).

In various embodiments, the compounds of the invention also find use as agents for the prevention of adhesion formation (see, *e.g.,* Journal of Surgical Research, (2011, Dec.) 171(2):495-503; Fertility and Sterility, (2009, Sept.) 92(3):S58; Journal of Minimally Invasive Gynecology, (2009, Nov.-Dec.) 16(6):S120).

In an exemplary embodiment, compounds of the invention also have antiviral activity (see, *e.g.,* Phytomedicine, (1999, Nov.) 6(5):335-340; Antiviral Research, (1991, Feb.) 15(2):139-148; Phytochemistry, (2010, Feb.) 71(2-3):235-242; and Advances in Food and Nutrition Research, (2011) 64:391-402).

In various embodiments, compounds of the invention are also inhibitors of the complement system (see, *e.g.,* Comparative Biochemistry and Physiology Part C: Pharmacology, Toxicology and Endocrinology, (2000, July) 126(3):209-215).

In each of these different treatment modalities, treatment of a subject in need of such treatment if effected by administering to the subject a therapeutically effective amount of an agent of the invention. In various embodiments, the compound is administered to a subject to treat a condition and this subject is not otherwise in need of treatment with a compound of the invention for a different condition.

In practicing methods of the invention, protocols for enhancing blood coagulation in a subject may vary, such as for example by age, weight, severity of the blood clotting disorder, the general health of the subject, as well as the particular composition and concentration of the NASP of the invention being administered. In embodiments of the invention, the concentration of NASPs achieved in a subject following oral administration and absorption by the gastrointestinal system may vary, in some instances, ranging from about 0.01 nM to about 500 nM. Exemplary NASPs of interest are procoagulant at their optimal concentration. By "optimal concentration" is meant the concentration in which NASPs exhibit the highest amount of procoagulant activity. Since exemplary NASPs also demonstrated anticoagulant activity at much higher concentrations than the optimal concentration, preferred NASPs of the invention show non-anticoagulant behavior in the range of its optimal concentration. As such, depending on the potency of the NASP as well as the desired effect, the optimal concentration of an exemplary NASPs provided by methods of the invention may range, from 0.01 µg/kg to 500 µg/kg, such as 0.1 µg/kg to 250 µg/kg, such as 0.1 µg/kg to 100 µg/kg, such as 0.1 µg/kg to 75 µg/kg, such as 0.1 µg/kg to 50 µg/kg, such as 0.1 µg/kg to 25 µg/kg, such as 0.1 µg/kg to 10 µg/kg, and including 0.1 µg/kg to 1 µg/kg. Optimal concentrations and activity level as determined by calibrated automated thrombography (CAT) assay of NASPs of interest are described in greater detail in United States Patent Application Serial No. 11/140,504, filed on May 27, 2005, now United States Patent No. 7,767,654, and United States Patent Application Serial No. 13/006,396, filed on January 13, 2011, the disclosures of which is herein incorporated by reference in their entirety. Likewise, the present application discloses examples of CAT assays of use in determining optimal concentration of a NASP of the invention.

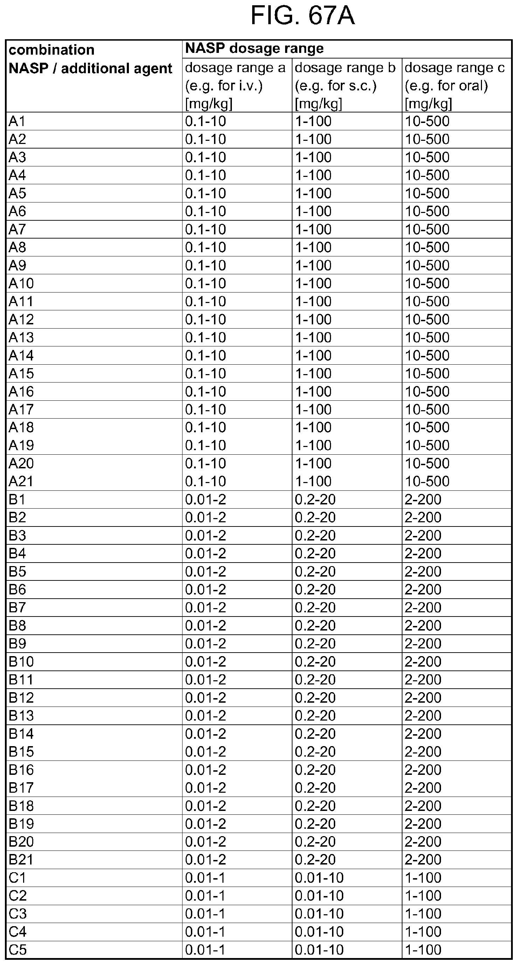

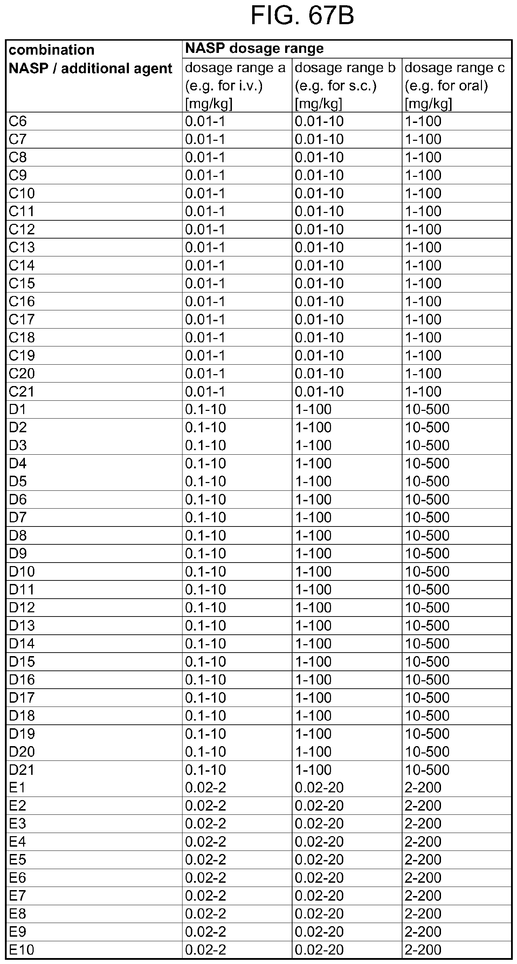

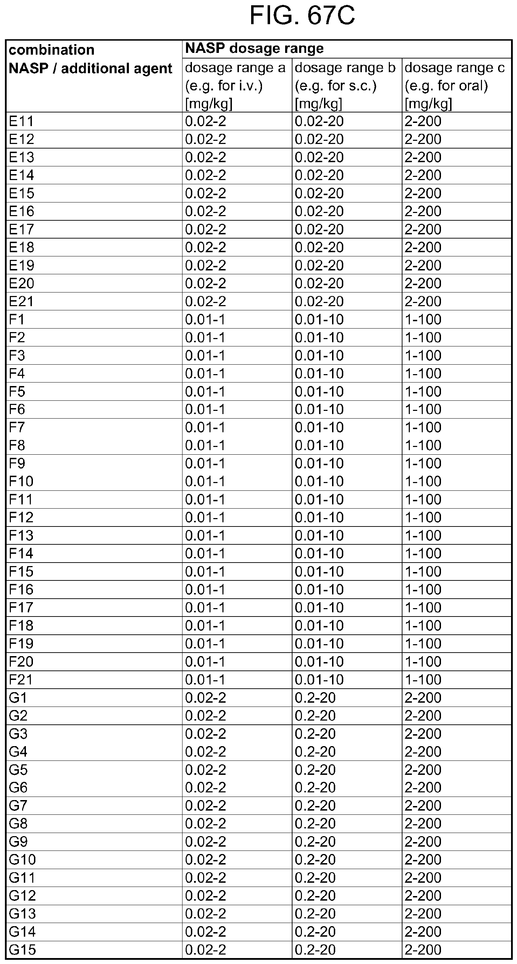

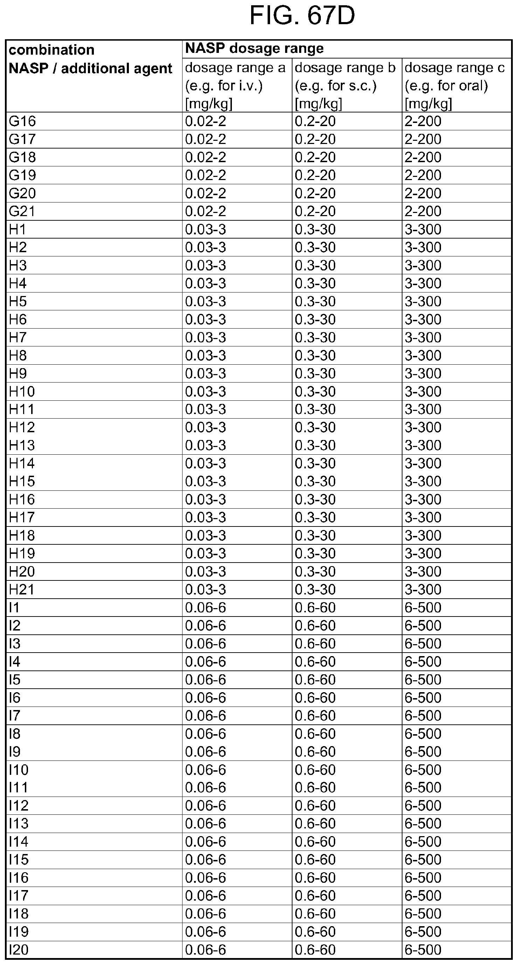

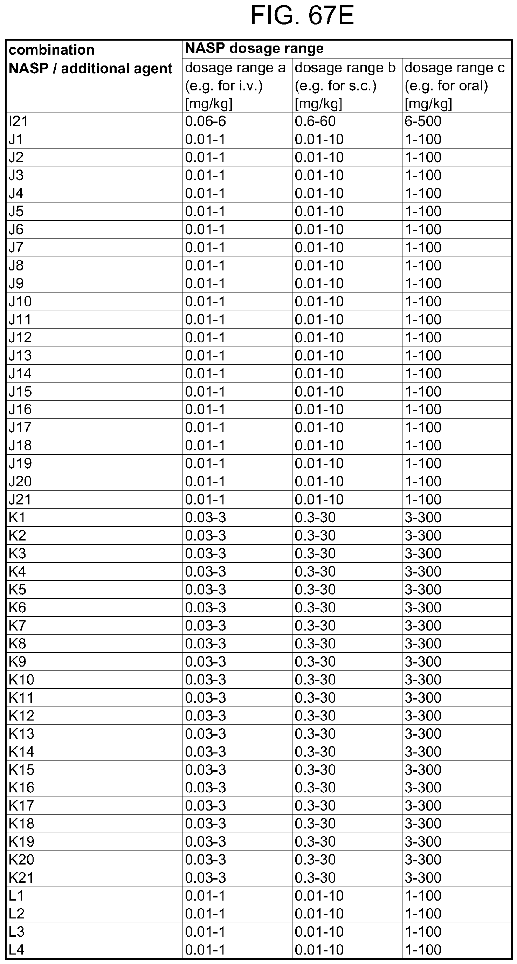

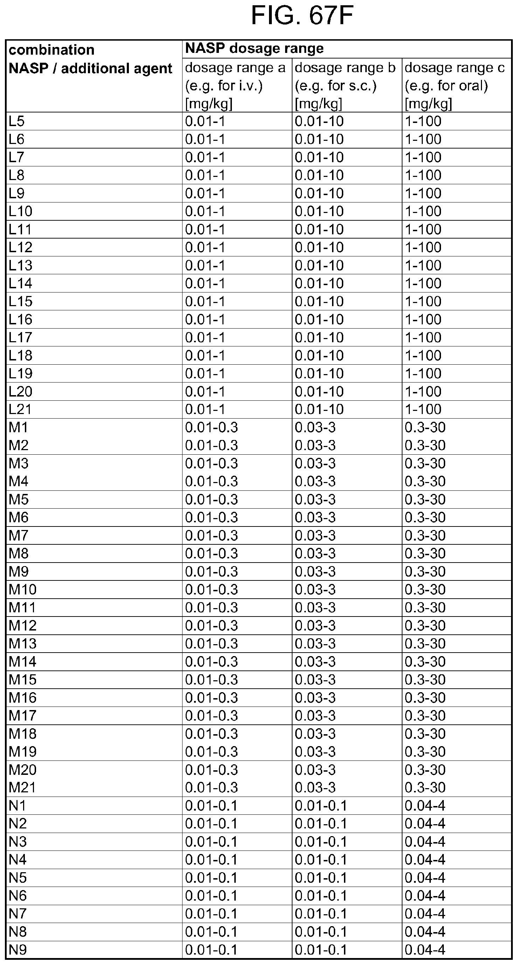

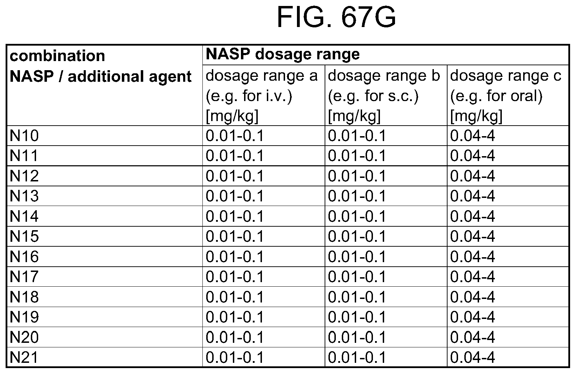

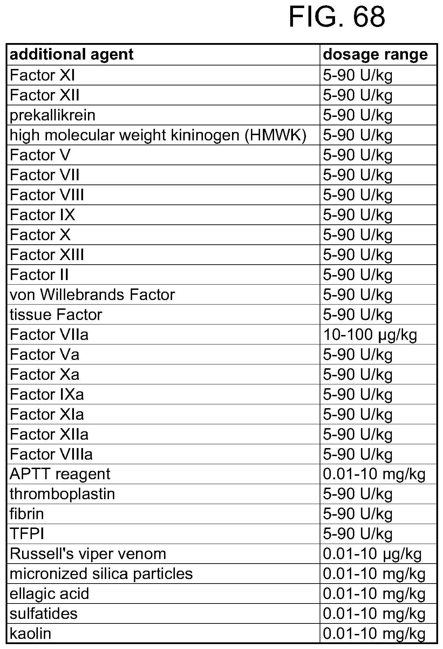

Exemplary dosages of the respective NASP in each of the combinations (type of NASP (based on the base polysaccharide) with additional agent) identified in **FIG. 66A-B** are shown in **FIG. 67****.** The lower case letter appended to the identifier of the combination from **FIG. 66A-B** refers to the dosage of the respective NASP in that combination. For example, "C5a" refers to a combination of a NASP having a cellopentaose base polysaccharide with Factor V, wherein the dose of the NASP is from aobut 0.01 to about 1 mg/kg. Exemplary dosages for the additional agent are provided in **FIG. 68****.**

In the various embodiments of the invention, the dosage (*e.g*., oral dosage) of compositions containing NASPs of the invention may vary, in exemplary embodiments, ranging from about 0.01 mg/kg to about 500 mg/kg per day, such as from about 0.01 mg/kg to about 400 mg/kg per day, such as about 0.01 mg/kg to about 200 mg/kg per day, such as about 0.1 mg/kg to about 100 mg/kg per day, such as about 0.01 mg/kg to about 10 mg/kg per day, such as about 0.01 mg/kg to about 2 mg/kg per day, including about 0.02 mg/kg to about 2 mg/kg per day. In other embodiments, the dosage (e.g., oral dosage) may range from about 0.01mg/kg to about 100 mg/kg four times per day (QID), such as about 0.01 to about 50 mg/kg QID, such as about 0.01 mg/kg to about 10 mg/kg QID, such as about 0.01 mg/kg to about 2 mg/kg QID, such as about 0.01 to about 0.2 mg/kg QID. In other embodiments, the dosage (*e.g*., oral dosage) may range from 0.01 mg/kg to 50 mg/kg three times per day (TID), such as 0.01 mg/kg to 10 mg/kg TID, such as 0.01 mg/kg to 2 mg/kg TID, and including as 0.01 mg/kg to 0.2 mg/kg TID. In yet other embodiments, the dosage (*e.g*., oral dosage) may range from 0.01 mg/kg to 100 mg/kg two times per day (BID), such as 0.01 mg/kg to 10 mg/kg BID, such as 0.01 mg/kg to 2 mg/kg BID, including 0.01 mg/kg to 0.2 mg/kg BID. The amount of compound administered will depend on the potency and concentration of the specific NASP, the magnitude or procoagulant effect desired, and the inherent absorptivity and/or bioavailability of the NASP. Each of these factors is readily determined by one of skill in the art using the methods set forth herein or methods recognized in the art.

In various embodiments of the methods herein, the NASP of the invention is orally administered in combination with one or more permeation enhancer. Appropriate permeation enhancers and their use with procoagulants, such as those provided by the present invention are disclosed in U.S. Provisional Patent Application No. 61/509,514, *supra.* In some embodiments the permeation enhancer is a gastrointestinal epithelial barrier permeation enhancer. In various embodiments, the invention provides methods for enhancing blood coagulation by orally administering a composition including a procoagulant amount of a NASP in combination with a gastrointestinal epithelial permeation enhancer to a subject. In various embodiments, the invention provides methods for enhancing blood coagulation by orally administering a composition including a procoagulant amount of a NASP in combination with a gastrointestinal epithelial permeation enhancer and a blood coagulation factor to a subject.

In another aspect, the invention provides an in vitro method of inhibiting TFPI activity with a sufficient amount of a NASP of the invention to inhibit TFPI activity. In certain embodiments, TFPI activity is inhibited in a subject by a method comprising administering a therapeutically effective amount of a composition comprising a NASP to the subject. In certain embodiments, the invention provides a method of inhibiting TFPI activity in a biological sample, the method comprising combining the biological sample *(e.g.,* blood or plasma) with a sufficient amount of a NASP to inhibit TFPI activity.

Exemplary NASPs of the invention for use in the methods of the invention are sulfated or sulfonated polysaccharides that have procoagulant activity. The properties of potential NASPs are determined using TFPI- dPT or aPTT clotting assays. Non-anticoagulant sulfated or sulfonated polysaccharides exhibit no more than one-third, and preferably less than one-tenth, the anticoagulant activity (measured by increase in clotting time) of unfractionated heparin.

Below are examples of specific embodiments for carrying out the present invention. The examples are offered for illustrative purposes only, and are not intended to limit the scope of the present invention in any way.

### EXAMPLES

### EXAMPLE 1

For the sulfation, different sulfation reagents were used: SO₃-NMe₃, SO₃-NEt₃ and SO₃-Py. Each of these reagents was tried for maltotriose, α-cyclodextrin and β-cyclodextrin under identical conditions to determine the most effective sulfation reagent. The general reaction characteristics were very similar for every reagent with only minor differences. SO₃-NEt₃ in DMF was chosen for use in the further experiments. In order to obtain a different degree of sulfation for each oligosaccharide the experiments were run at room temperature and at 70°C.

Literature procedures usually use Sephadex columns for the separation of the sugar components from the sulfation reagent and another Sephadex column or dialysis for cation exchange. Sephadex chromatography for the given oligosaccharides was unsuccessful, so a different protocol was established. The new protocol includes the precipitation of the saccharide components and the washing-out of the sulfation reagent with chloroform. In order to exchange triethylammonium- against sodium ions the mixture was redissolved in water and treated with the Na⁺- form of a cation exchange resin (Amberlyst 15).

### Analysis

The initial project plan envisioned the characterization of the resulting polysaccharide mixtures using NMR and elemental analysis. Since both of these methods have limitations for exemplary saccharides of the invention, a different method was established. Using a chromatographic method called HILIC (Hydrophilic Interaction Liquid Chromatography) meaningful results were obtained, including the identification of different products of varying sulfation grade and quantitation of the relative amounts thereof.

### 1) Results

At room temperature, cellotriose (1) was produced as 70% non-sulfated cellotriose, 30% mono (4 Isomers); At 70° C, cellotriose (2) was produced as 30% non-sulfated cellotriose, 70% mono (several isomers).

At room temperature, cellotetraose (3) was produced as 80% non-sulfated cellotetraose, 20% mono (2 Isomers); at 70° C cellotetraose (4) was produced as 40% non-sulfated cellotetraose, 30% mono (several isomers), 30% decomposition product (M = 693), traces of the nonsulfated decomposition product.

At room temperature, cellopentaose (5) was produced as 90% non-sulfated cellotetraose, 10% mono (2 Isomers); at 70° C cellopentaose (6) was produced as 80% non-sulfated cellopentaose, 10% mono (several Isomers), 10% decomposition product (M = 855).

At room temperature, maltotriose (7) was produced as 50% mono, 25% bis (2 isomers), 25% tris (4 Isomers); at 70° C, maltotriose (8) was produced as 60% mono (several Isomers), 40% decomposition product (M = 611, fragmentation shows sulfation),

At room temperature, maltotetraose (9) was produced as 70% non-sulfated maltotetraose, 30% mono (several Isomers); at 70° C, maltotetraose (10) was produced as 50% non-sulfated maltotetraose, 50% mono (2 Isomers).

At room temperature, maltopentaose (11) was produced as 60% non-sulfated maltopentaose, 40% mono (2 Isomers); at 70° C, maltopentaose (12) was produced as 50% non-sulfated maltopentaose, 30% mono (3 Isomers), 20% decomposition product (M=855).

At room temperature, raffinose (13) was produced as 70% non-sulfated raffinose, 30% mono (several Isomers); at 70° C, raffinose (14) was produced as 20% non-sulfated raffinose, 30% mono (4 Isomers), traces bis, 50% decomposition product (M=171).

At room temperature, melezitose (15) was produced as 40% non-sulfated melezitose, 50% mono (4 Isomers), 10% bis (2 Isomers); at 70° C, melezitose (16) was produced as 25% non-sulfated melezitose, 50% mono (4 Isomers), 25% bis (2 Isomers), traces tris.

At room temperature, α-cyclodextrin (17) 45% non-sulfated α-cyclodextrin, 50% mono, 5% Bis (2 Isomers), traces tris; at 70° C α-Cyclodextrin (18) was produced as 50% non-sulfated α-cyclodextrin, 50% mono.

At room temperature, β-cyclodextrin (19) was produced as 70% non-sulfated β-cyclodextrin, 30% mono, traces bis (2 Isomers); at 70° C β-cyclodextrin (20) was produced as 60% non-sulfated β-CD, 40% mono.

Stachyose (20) was produced as approx. 73% sulfated, with about 10 sulfates and 18.4% sulfur.

Xylohexaose (21) was produced as approx. 59% sulfated polysaccharide, with about 8 sulfates and 13.9% sulfur.

γ-cyclodextrin (22) [13-19% sulfur].

### EXAMPLE 2

### Thrombin Generation Assay

The procoagulant activity of the sulfated polysaccharides was assessed by the Thrombin Generation Assay (TGA). The influence of each sulfated polysaccharide on thrombin generation was measured in duplicate via CAT in a Fluoroskan Ascent® reader (Thermo Labsystems, Helsinki, Finland; filters 390 nm excitation and 460 nm emission) following the slow cleavage of the fluorogenic substrate Z-Gly-Gly-Arg-AMC (Hemker HC. Pathophysiol Haemost Thromb (2003) 33(4):15). To each well of a 96 well microplate (Immulon 2HB, clear U-bottom; Thermo Electron) 80 µL of pre-warmed (37° C) goat anti FVIII antibody treated human normal plasma pool was added. For triggering thrombin generation by tissue factor, 10 µL of PPP reagent containing a certain amount of recombinant human tissue factor (rTF) and phospholipid vesicles composed of phosphatidylserine, phosphatidylcholine and phosphatidylethanolamine (final concentration of 4 µM) (Thrombinoscope BV, Maastricht, The Netherlands) was added. For studying the procoagulant activity of sulfated polymers a final TF concentration of 1 pM was used to provide FVIII and TFPIsensitivity of the test system. Just prior to putting the plate into the pre-warmed (37°C) reader, 10 µL of test or reference sample or calibrator compound was added. Thrombin generation was started by dispensing 20 µL of FluCa reagent (Thrombinoscope BV, Maastricht, The Netherlands) containing fluorogenic substrate and Hepes buffered CaCl2 (100 mM) into each well and fluorescence intensity was recorded at 37°C.

The parameters of the resulting thrombin generation curves were calculated using the ThrombinoscopeTM software (Thrombinoscope BV, Maastricht, The Netherlands) and thrombin calibrator to correct for inner filter and substrate consumption effects (Hemker HC, Pathophysiol Haemost Thromb (2003) 33(4):15). With the thrombin calibrator as a reference, the molar concentration of thrombin in the test wells was calculated by the software. The thrombin amounts at the peak and peak times of each thrombin generation curve (peak thrombin, nM) were plotted against sulfated polysaccharide concentrations resulting in the procoagulant profile of these compounds. The thrombin generation assay results are illustrated in the figures appended hereto. From these plots half maximal effective concentrations (EC50) values are determined using a sigmoidal curve fit. The EC50 represents the concentration at which the half maximal thrombin peak is reached.

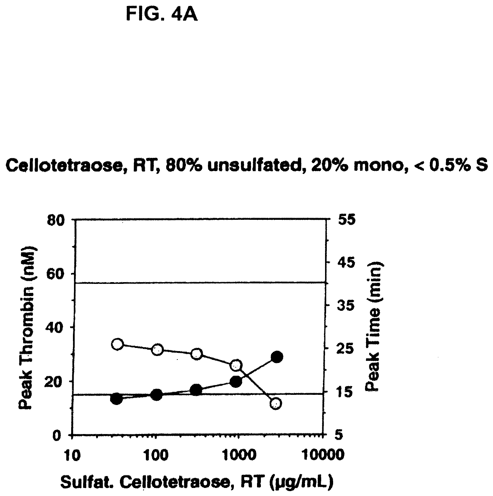

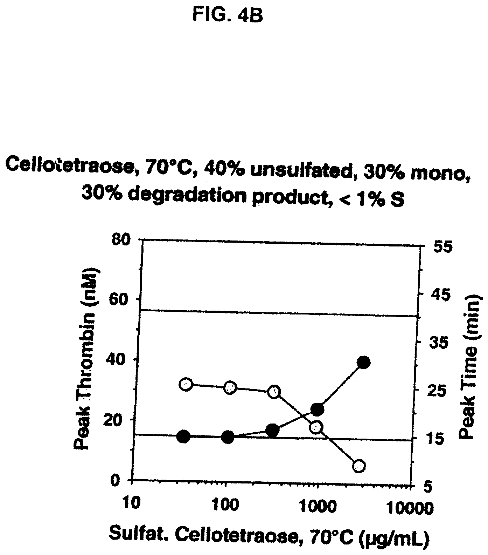

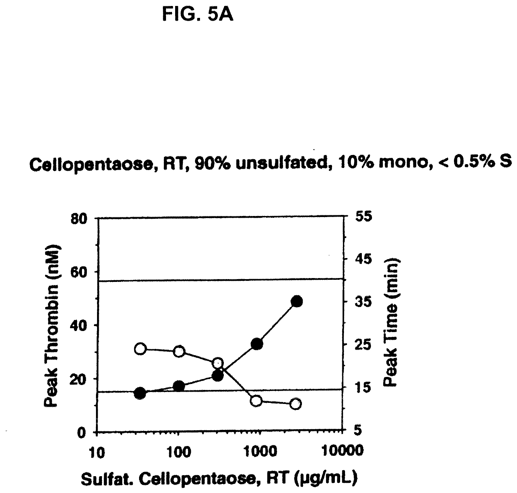

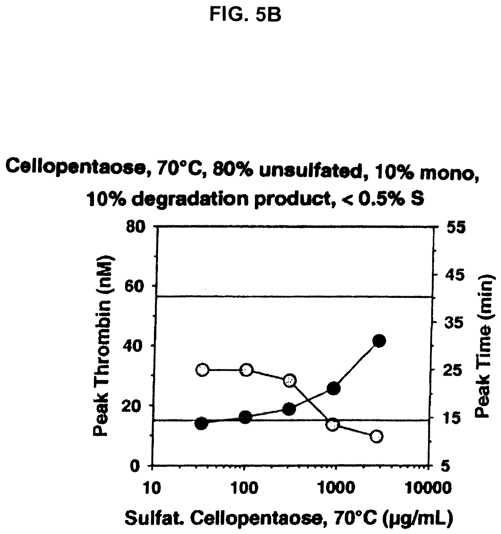

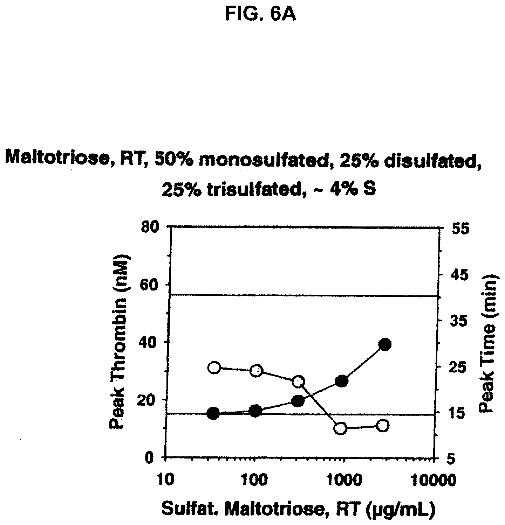

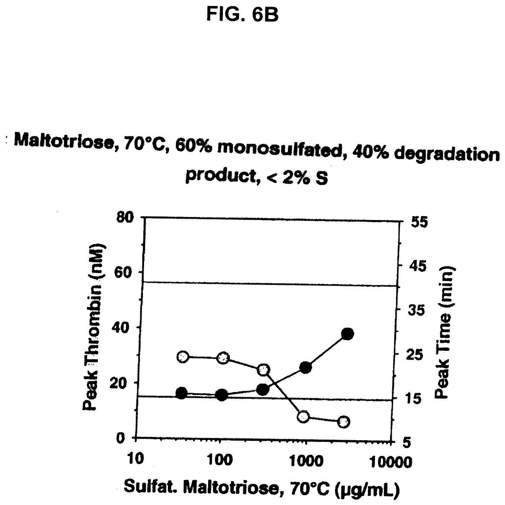

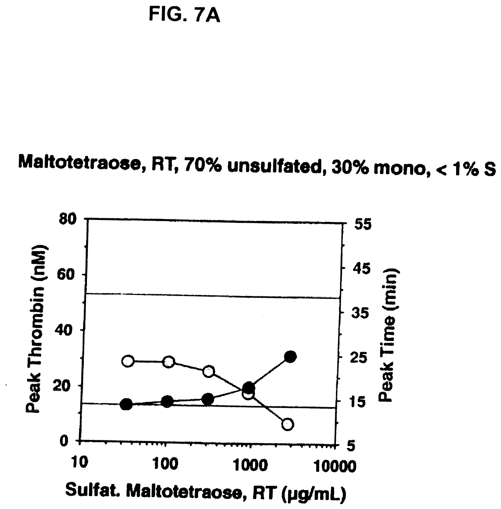

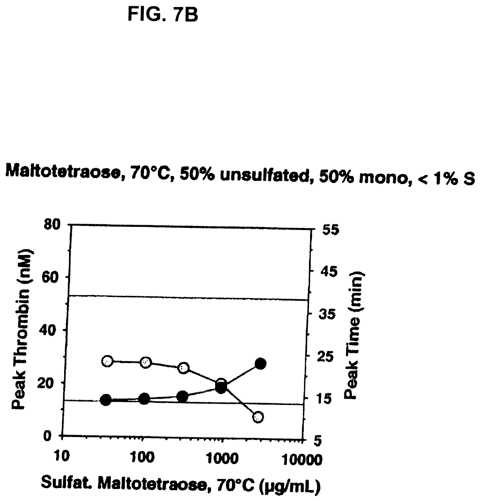

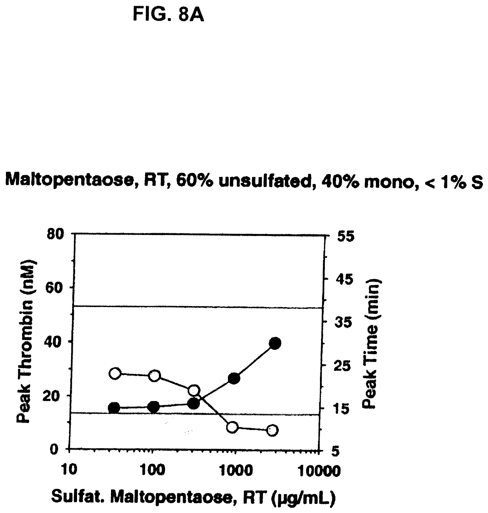

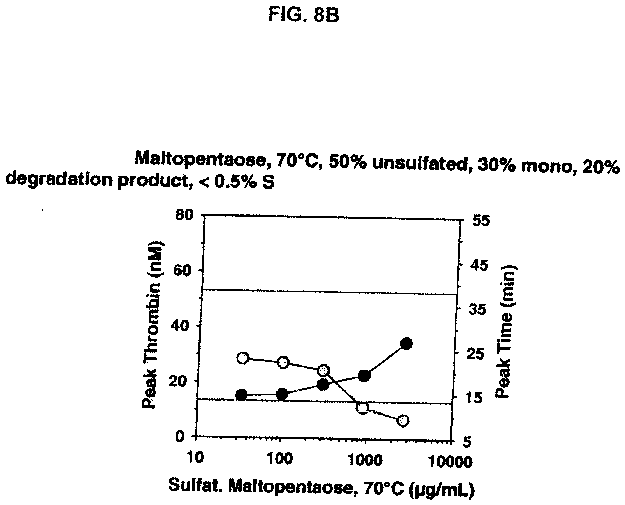

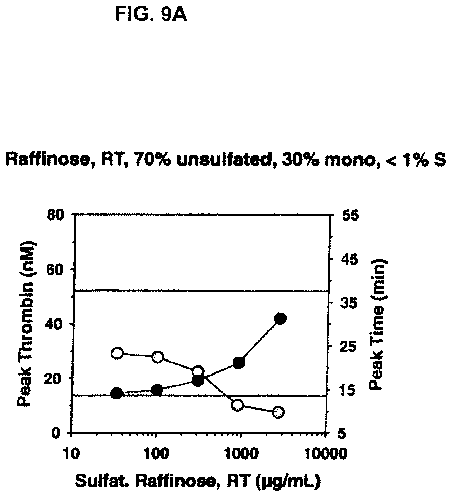

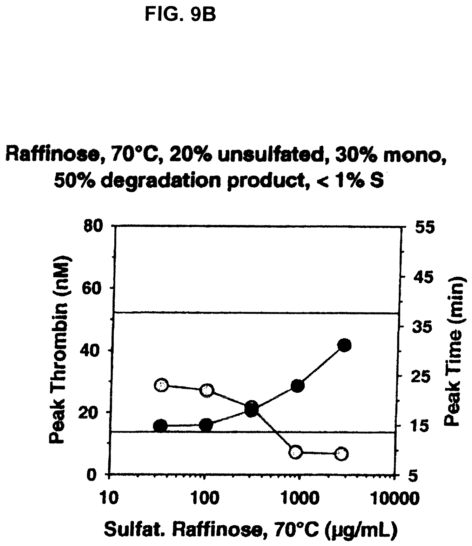

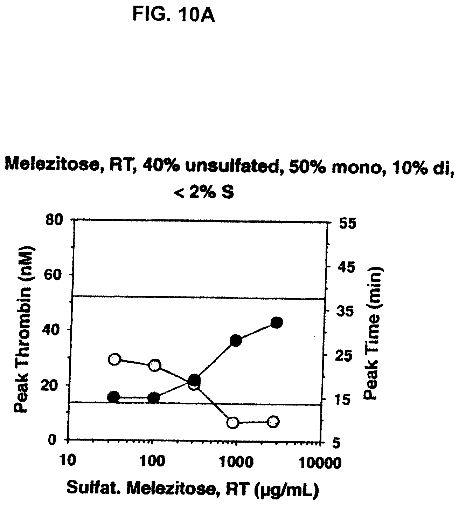

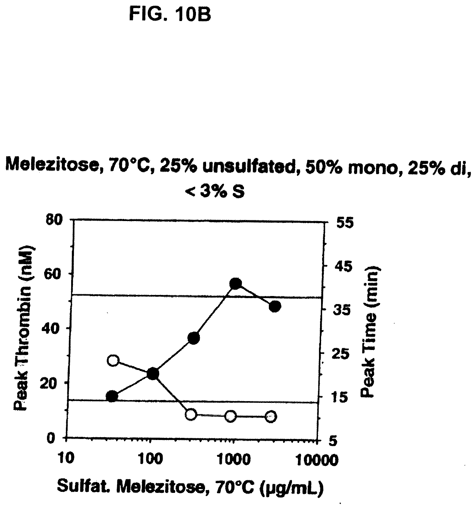

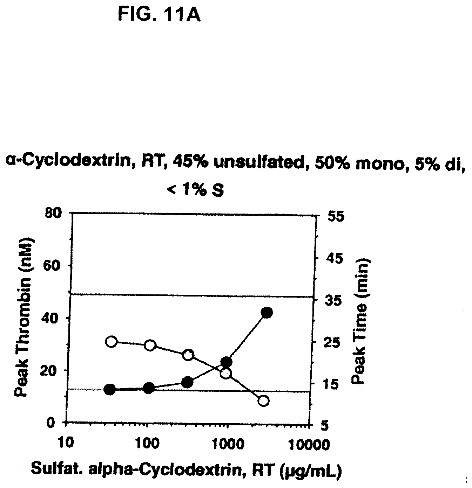

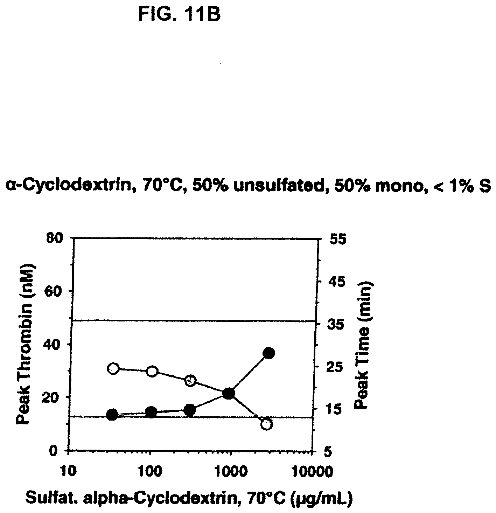

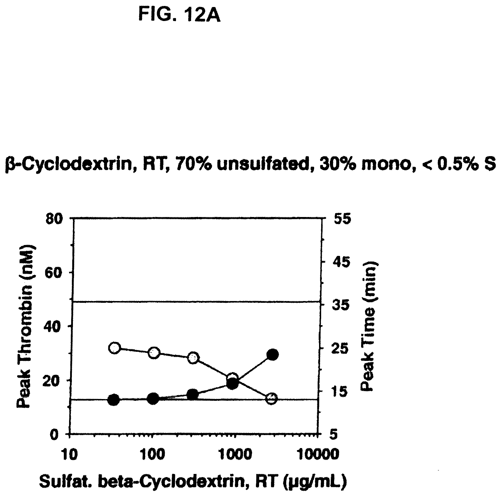

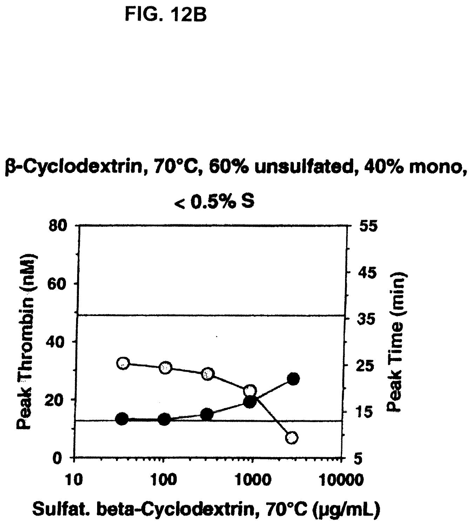

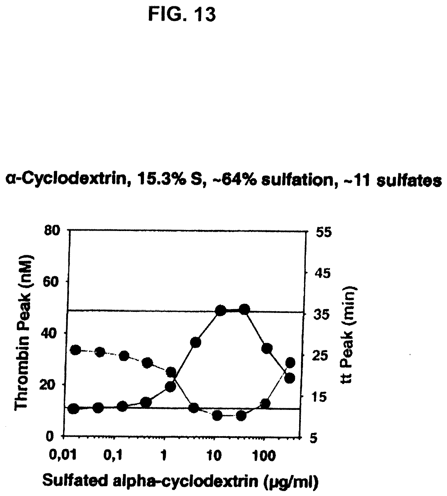

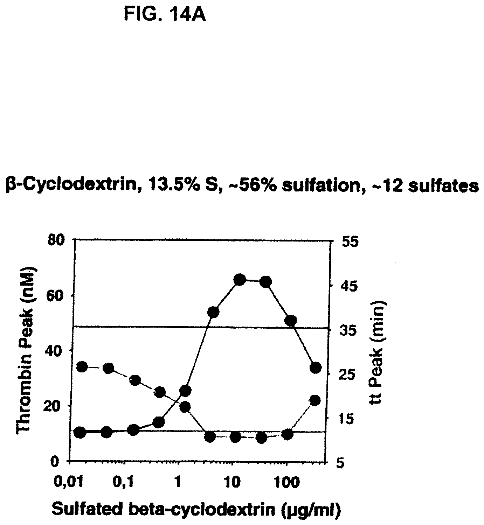

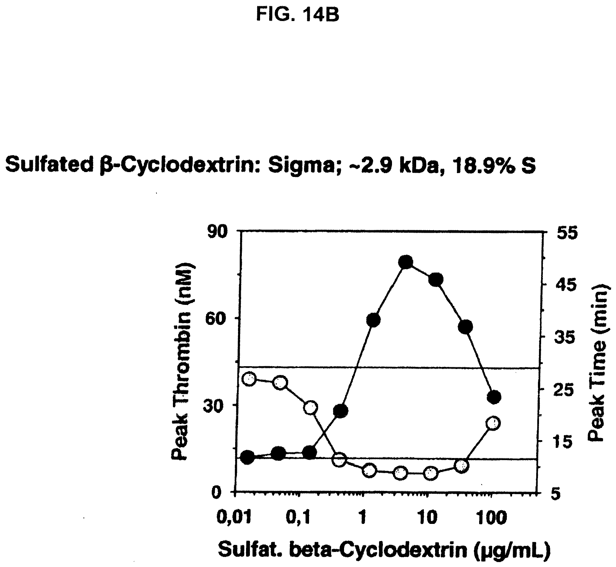

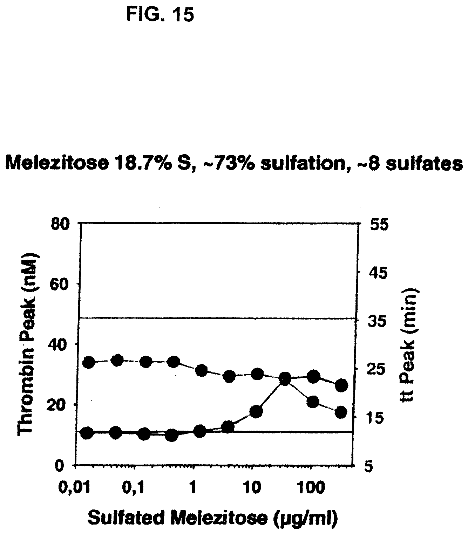

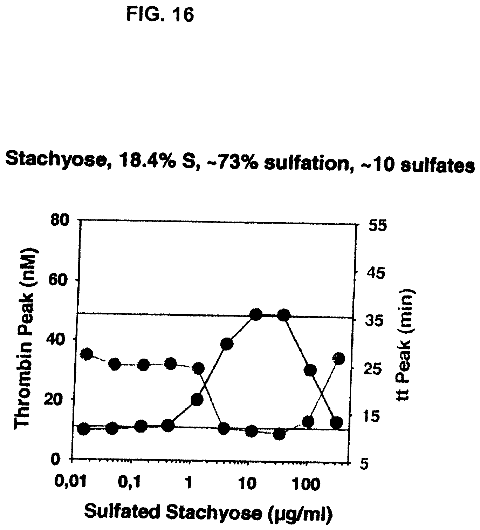

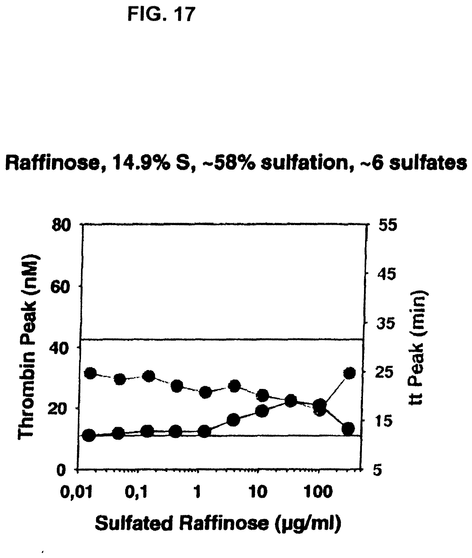

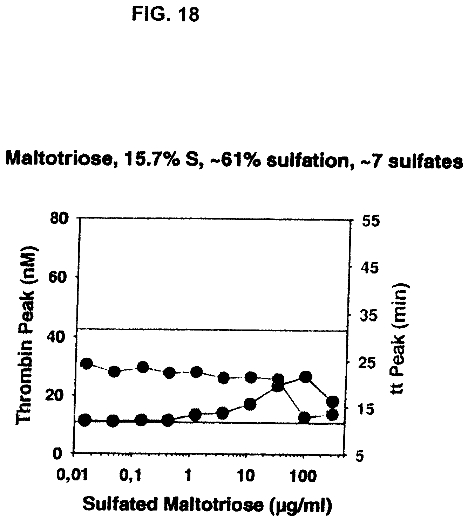

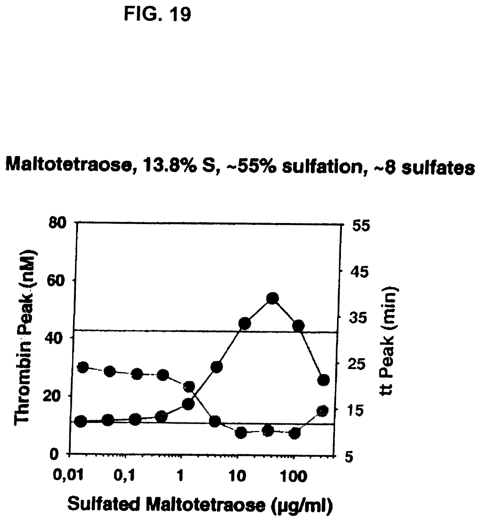

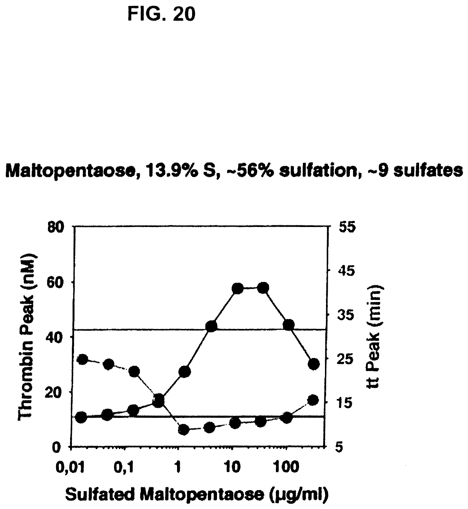

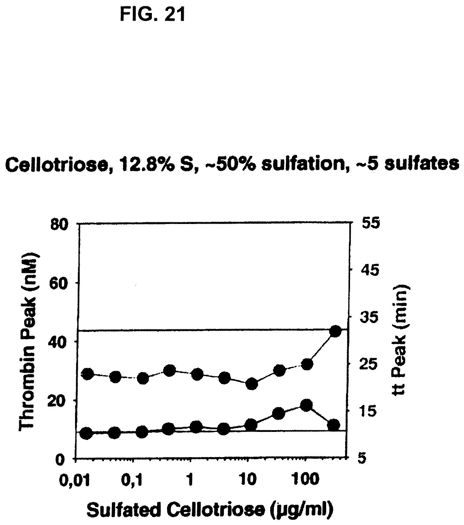

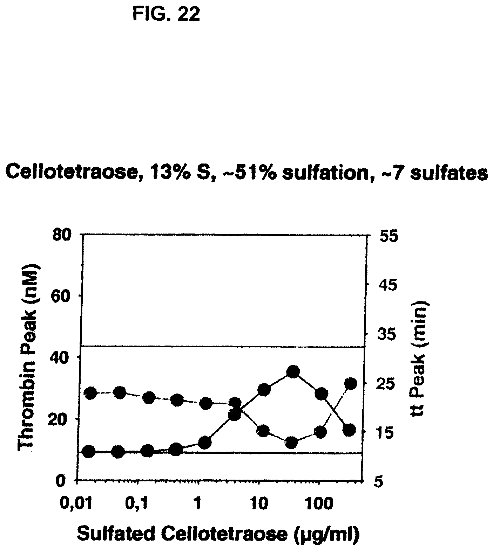

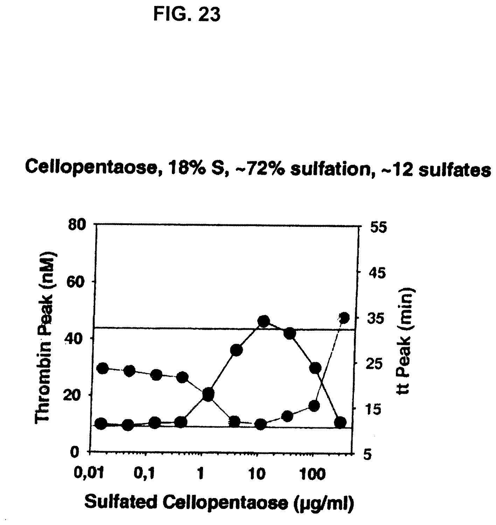

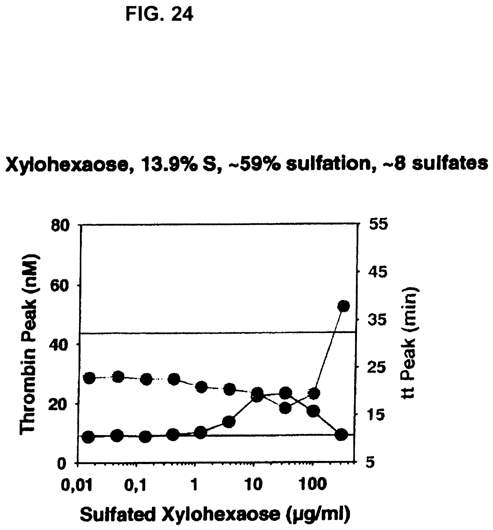

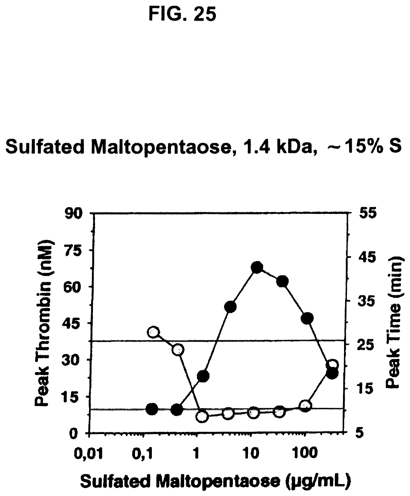

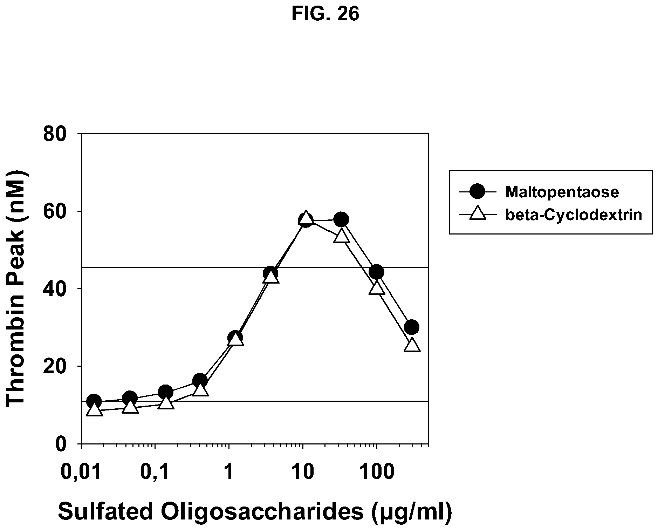

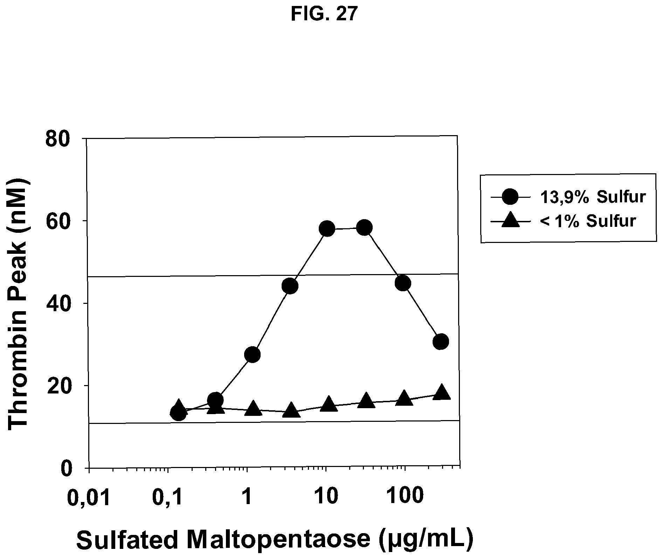

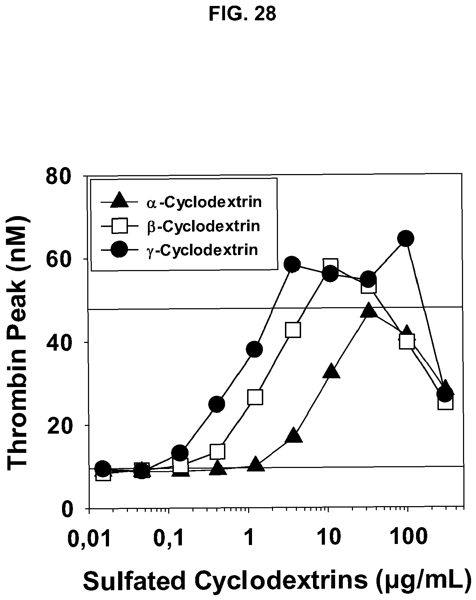

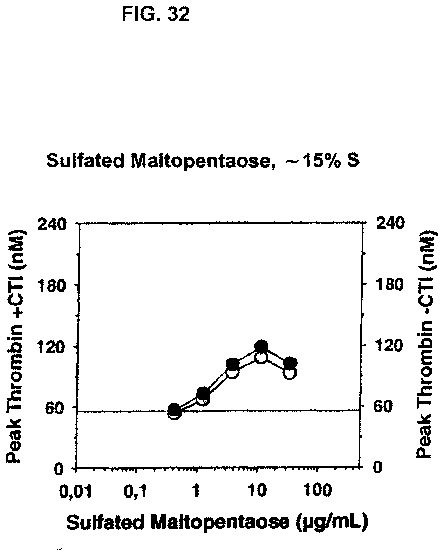

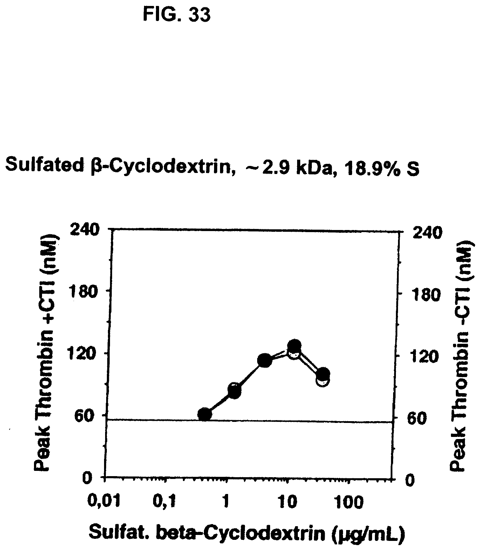

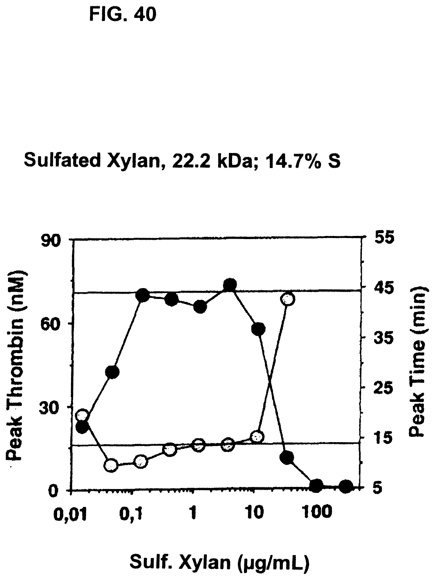

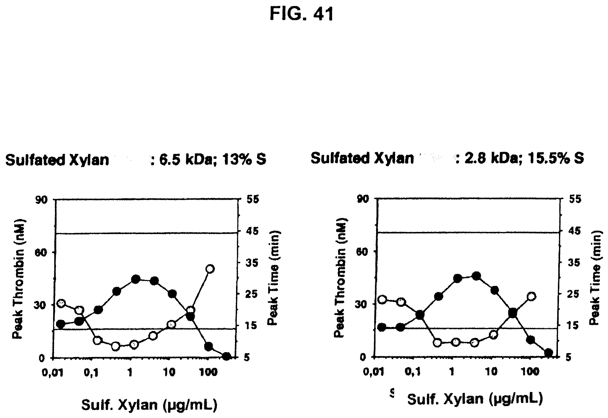

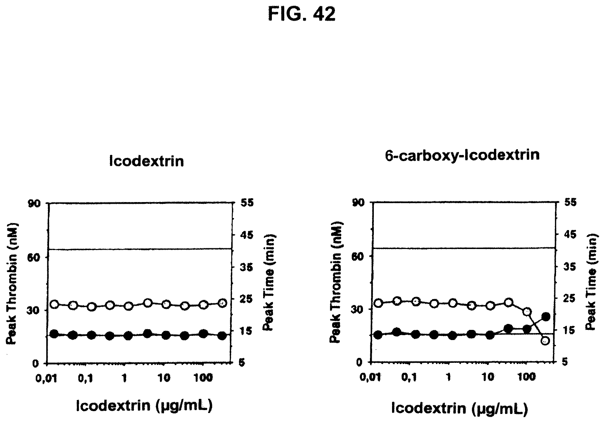

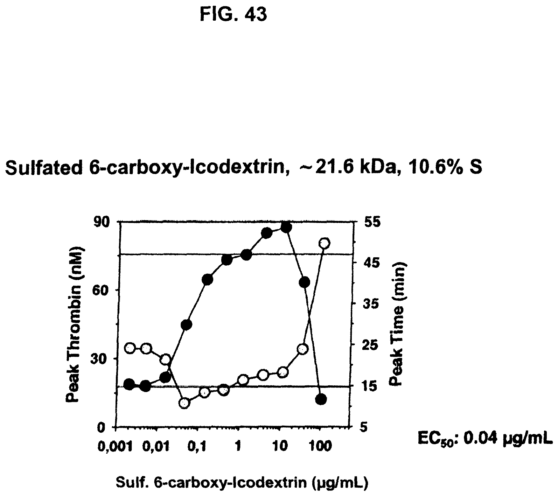

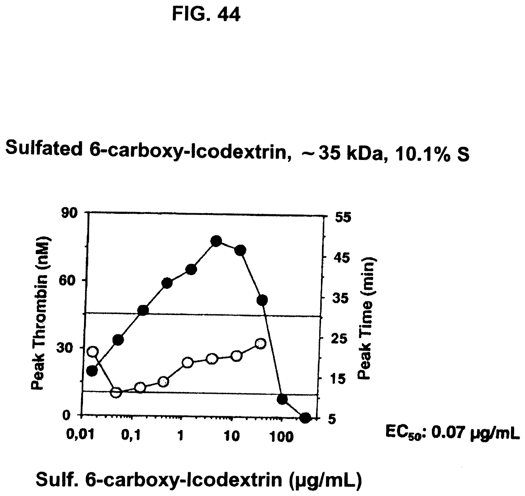

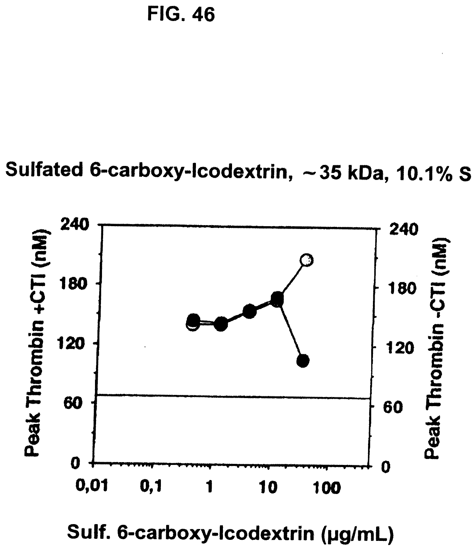

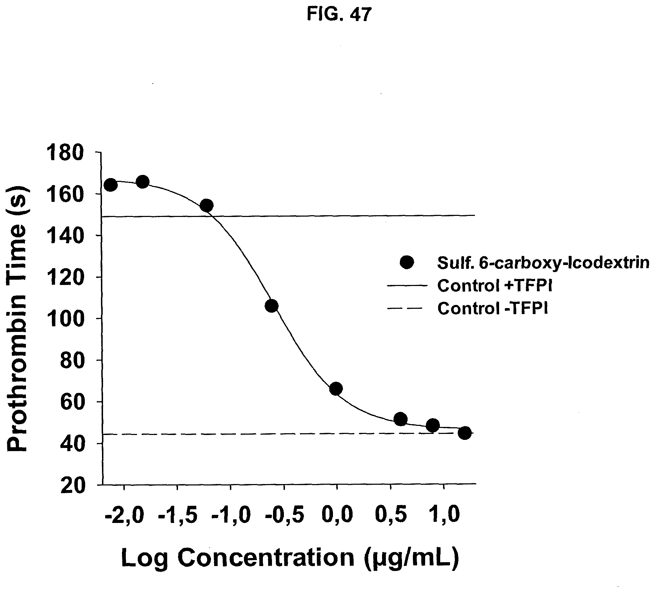

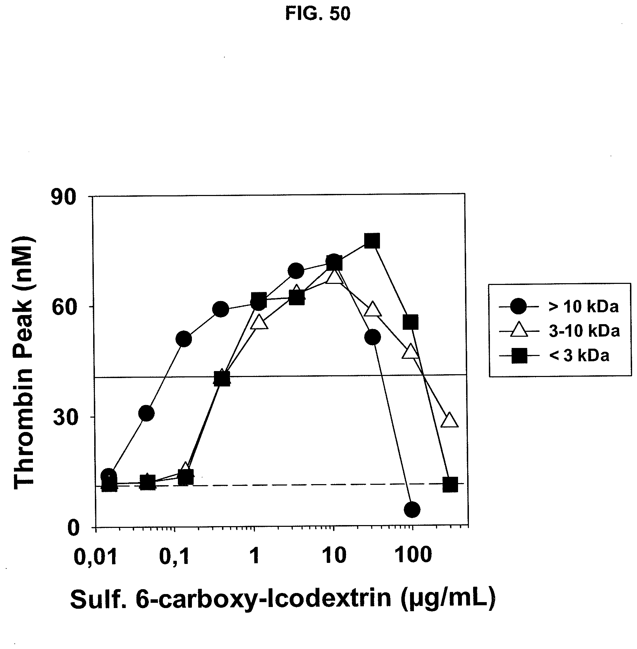

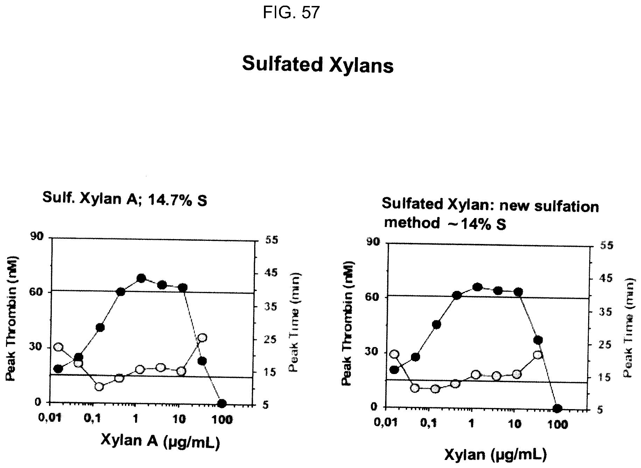

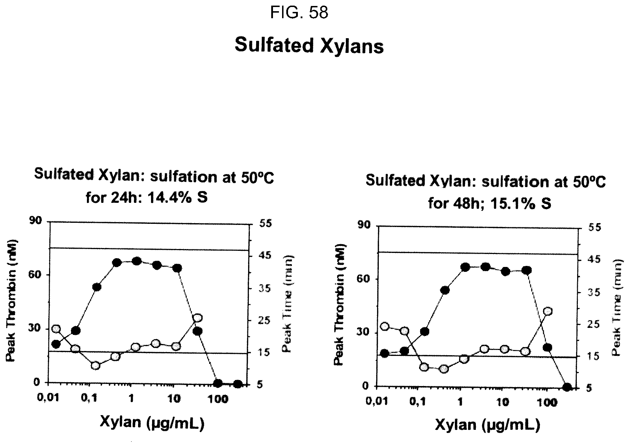

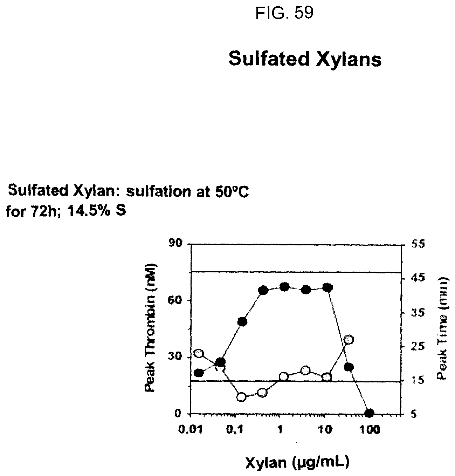

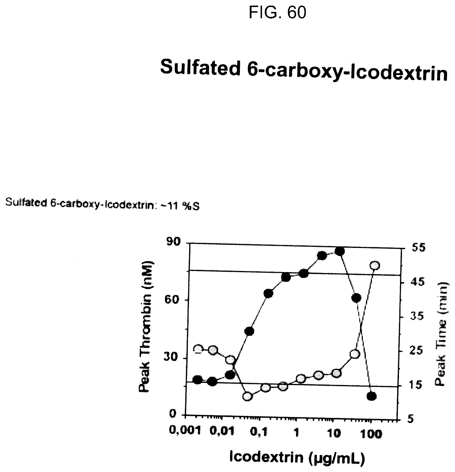

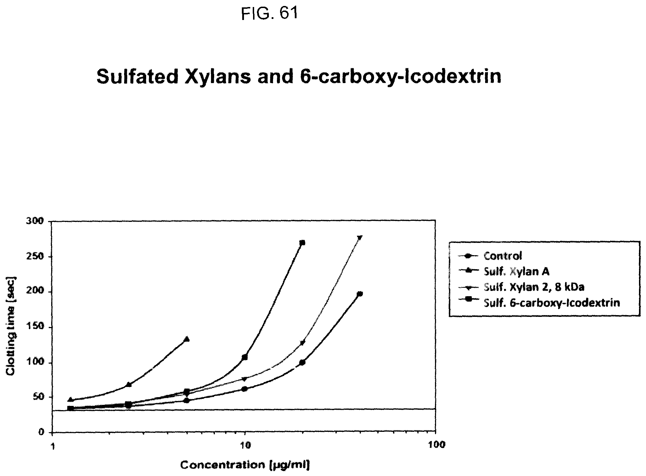

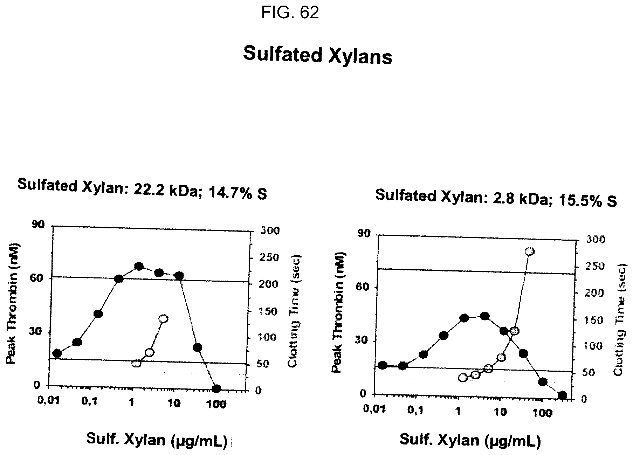

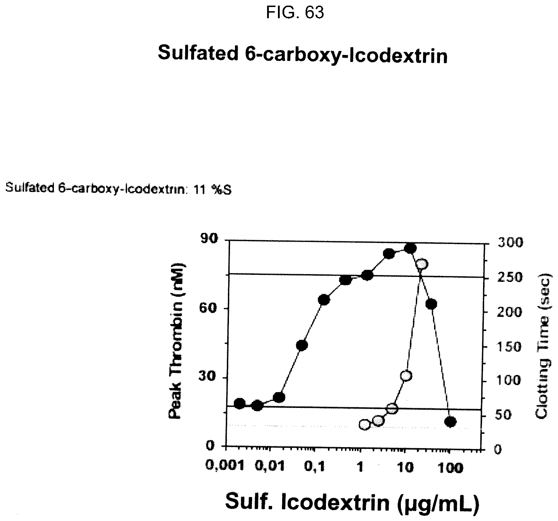

Results for cellotriose are shown in **FIG. 3A****,** **FIG. 3B** and **FIG. 21****.** Results for cellotetraose are shown in **FIG. 4A****,** **FIG. 4B** and **FIG. 22****.** Results for cellopentaose are shown in **FIG. 5A****,** **FIG. 5B** and **FIG 23.** Results for maltotriose are shown in **FIG. 6A****,** **FIG. 6B****,** **FIG. 18****.** Results for maltotetraose are shown in **FIG. 7A****,** **FIG. 7B** and **FIG. 19****.** Results for maltopentaose are shown in **FIG. 8A****,** **FIG. 8B****,** **FIG. 20****,** **FIG. 25** - **FIG. 27****,** **FIG. 30****,** **FIG. 32** and **FIG. 34****.** Results for raffinose are shown in **FIG. 9A****,** **FIG. 9B** and **FIG. 17****.** Results for melezitose are shown in **FIG. 10A****,** **FIG. 10B****,** **FIG. 15** and **FIG. 29****.** Results for α-cyclodextrin are shown in **FIG. 11A****,** **FIG. 11B****,** **FIG. 13****,** **FIG. 28** and **FIG. 29****.** Results for β-cyclodextrin are shown in **FIG. 12A****,** **FIG. 12B****,** **FIG. 14A****,** **FIG. 14B****,** **FIG. 28****,** **FIG. 29****,** **FIG. 31****,** **FIG. 33** and **FIG. 35****.** Results for xylohexaose are shown in **FIG. 24****.** Results for stachyose are shown in **FIG. 16****.** Results for Xylan are shown in **FIG. 40****,** **FIG. 41****,** **FIG. 57****,** **FIG. 58****,** **FIG. 59****,** **FIG. 61****,** and **FIG. 62****.** Results for icodextrin and 6-carboxyiciodextrin are shown in **FIG. 42** **-** **FIG. 47****,** **FIG. 50****,** **FIG. 60****,** **FIG. 61** and **FIG. 63****.**

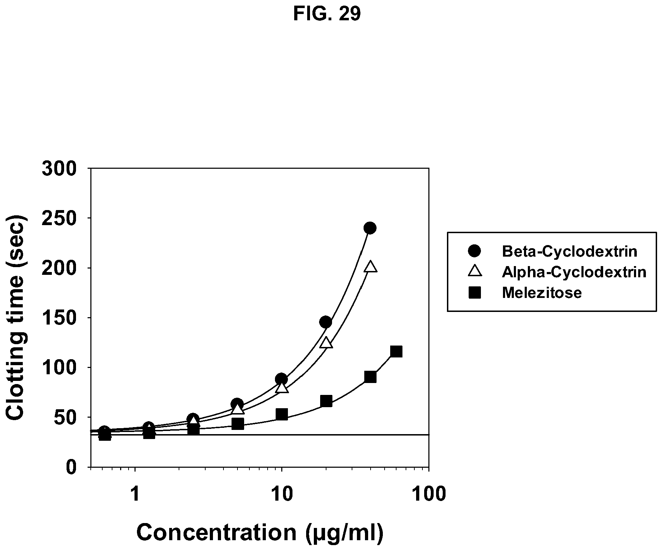

Sulfated polysaccharides are procoagulant in a broad concentration range spanning at least two orders of magnitude starting at about 0.01 µg/mL (e.g., sulfated xylan, sulfated 6-carboxy icodextrin), whereas a unsulfated polymer is essentially inactive under the conditions used herein, providing evidence for the importance of negatively charged sulfate groups. At concentrations of optimal procoagulant activity (1 to 30µg/mL) sulfated polysaccharides exceeded the thrombin generation of a human normal plasma pool. At concentrations higher than 100 µg/mL sulfated polysaccharides prolonged the activated partial thromboplastin time (**FIG. 29**) which is indicative of their anticoagulant activity.

### EXAMPLE 3

### TFPI- dPT and aPTT

### Dilute prothrombin time assay with TFPI

A dilute prothrombin time assay with added TFPI (TFPI-dPT) was used to evaluate the TFPI-inhibiting effect of the different NASPs. Pooled normal human plasma (George King Biomedical, Overland Park, KS) was pre-incubated with 0.5 µg/mL full-length TFPI (aa 1-276, constitutively produced by SKHep1) and the respective NASP (0-5 µg/mL) for 15 min at RT. TF reagent TriniClot PT Excel S (Trinity Biotech, Wicklow, Ireland), diluted in Hepes-buffered saline 1:200 with 0.5 % BSA was added to the plasma samples on an ACL Pro Elite hemostasis analyzer (Instrumentation Laboratory, Bedford, MA). Clotting was initiated with 25 mM CaCl₂. The volume ratio of plasma:TF:CaCl₂ was 1:1:1.

For data analysis, TFPI- dPT is plotted against the log concentration. Half maximal effective concentrations (EC50) values are determined using a sigmoidal curve fit.

### Activated partial thromboplastin time assay (aPTT)

The aPTT assay was performed to study anticoagulant activities of NASP. In brief, 50 µL of thawed normal human plasma pool (George King Biomedical, Overland Park, KS) was mixed with 5 µL of NASP sample (0-60 µg/mL final plasma concentration). NASPs were diluted in imidazole buffer (3.4 g/L imidazole; 5.85 g/L NaCl, pH 7.4) containing 1% albumin (Baxter, Austria). After addition of 50 µL aPTT reagent (STA APTT, Roche) the samples were incubated for 4 min at 37 °C. Clotting was initiated by 50 µL of 25 mM CaCl2 solution (Baxter, Austria) and recorded for up to 5 min. All pipetting steps and clotting time measurements were carried out with an ACL Pro Elite (Instrumentation Laboratory, Bedford, MA) instrument. Samples were run in duplicate.

For data analysis, clotting time is plotted against the NASP concentration. Concentrations where the clotting time is 50% increased over the normal plasma control are determined using a linear curve fit.

### Results

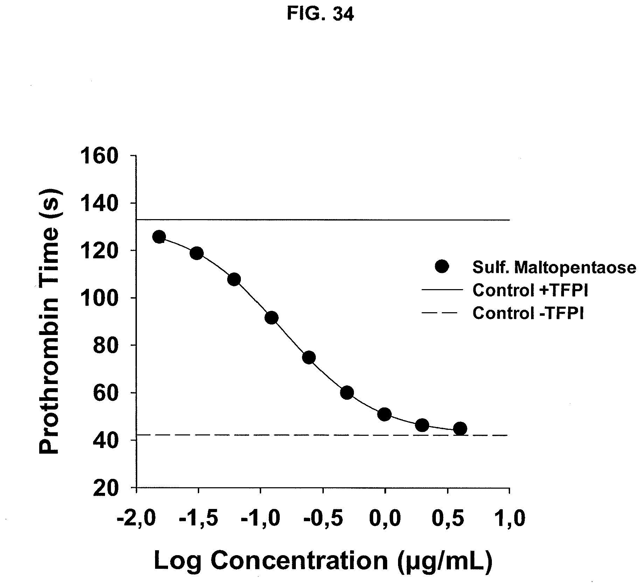

**FIG. 34** is a plot showing TFPI-dPT vs. log concentration of sulfated maltopentaose (15% S) in normal human plasma, showing that sulfated maltopentaose reverses the effect of rec. FL-TFPI in plasma. The EC50 of this compound is 0.15 µg/mL.

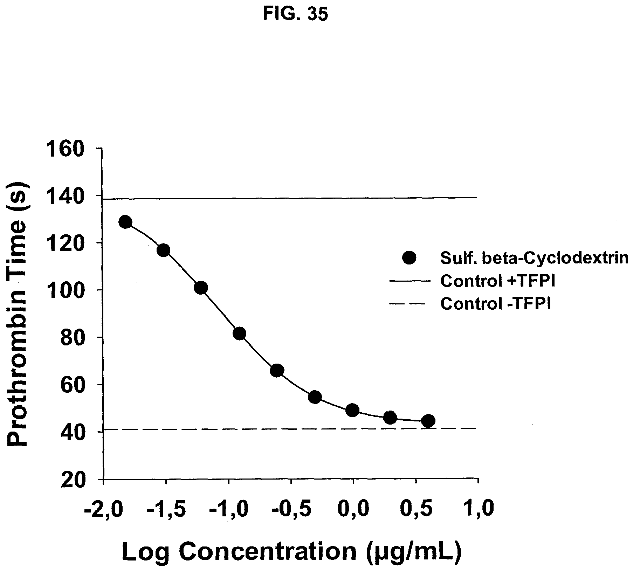

**FIG. 35** is a plot showing TFPI-dPT vs. log concentration of sulfated β-cyclodextrin (2.9 kDa, 18.9% S) in normal human plasma, showing that sulfated β-cyclodextrin reverses the effect of rec. FL-TFPI in plasma. The EC50 of this compound is 0.08 µg/mL.

EC50 values of representative compounds of the invention are set forth in Table 1 and Table 2. Table 2 further provides data for 50% clotting time deduced from aPTT assays for these compounds. (**FIG. 29**) The EC50 values were determined by CAT assay.

**Table 1**

| Sulfated Substance | EC₅₀ µg/mL |
|---|---|
| Maltotriose | 20.6 |
| Maltotetraose | 5.0 |
| Maltopentaose | 2.1 |
| Cellotriose | 30.9 |
| Cellotetraose | 4.8 |
| Cellopentaose | 1.9 |
| α-Cyclodextrin (Sigma) | 7.4 |
| β-Cyclodextrin (Sigma) | 1.8 |
| γ-Cyclodextrin (Baxter) | 0.8 |

**Table 2**

| Substance | 50% Increase Clotting Time µg/mL | EC₅₀ µg/mL | Ratio aPTT/CAT |
|---|---|---|---|
| Fucoidan (Control) | 7.0 | 0.3 | 23.3 |
| Maltopentaose (Baxter) | 3.3 | 2.4 | 1.4 |
| β-Cyclodextrin (Sigma) | 3.6 | 0.7 | 5.1 |

Exemplary NASPs of the invention were acquired from the sources shown in Table 3.

**Table 3**

| **Sulfated Compound** | **Provider** |
|---|---|
| Cellotriose | AnalytiCon, Germany |
| Cellotetraose | |
| Cellopentaose | |
| Maltotriose | |
| Maltotetraose | |
| Maltopentaose | |
| Raffinose | |
| Melezitose | |
| alpha-Cyclodextrin | |
| beta-Cyclodextrin | |
| Xylohexaose | |
| gamma-Cyclodextrin | Baxter RL |
| Maltopentaose | |
| 6-carboxy-Icodextrins | |
| Icodextrins | |
| Xylans | |
| alpha-Cyclodextrin | Sigma-Aldrich |
| beta-Cyclodextrin | |

Sulfated α- and β-cyclodextrin, stachyose, maltotetraose, maltopentaose and cellopentaose show substantial procoagulant activity. Sulfated maltopentaose, sulfated β-cyclodextrin and other sulfated polysaccharides become anticoagulant within their procoagulant window. Their EC₅₀ is similar to sulfated maltopentaose (2.4 µg/mL) and sulfated β-cyclodextrin (0.7 µg/mL) from Sigma. Procoagulant activity increases with compound molecular weight for cyclodextrins, maltotriose/-tetraose/-pentaose and cellotriose/-tetraose/-pentaose.

### EXAMPLE 4

### Rotation Thromboelastometry (ROTEM)-Method

NASPs (0.4 - 11 µg/mL) were studied in fresh citrated human whole blood by rotation thromboelastometry (ROTEM), an assay that allows continuous visco-elastic assessment of whole blood clot formation and firmness. Blood was incubated with antihuman FVIII plasma raised in goat (Baxter Bioscience, Austria) at 50 BU/mL to simulate hemophilic conditions. Clotting was triggered with 0.044 pM tissue factor (TF) (PRP reagent, Thrombinoscope BV) in the presence of 40 µg/mL CTI (Haematologic Technologies Inc., Essex Junction, VT, USA). Then 300 µL of the pre-warmed (37 °C) FVIII-inhibited blood was recalcified with 20 µL of a 0.2 M CaCl2 solution. Clot formation was monitored using a ROTEM instrument (Pentapharm Munich, Germany) at 37 °C. The final assay concentration of rTF was 44 fM. The ROTEM recording was started immediately and measured for at least 120 min. Each blood sample was analyzed in two independent measurements.

The thromboelastographic parameters of clotting time (CT), clot formation time (CFT) and maximum clot firmness (MCF) were recorded in accordance with the manufacturer's instructions. The first derivative of the thromboelastogram data is plotted to obtain a graph of velocity (mm/s) against time (s). From this graph, the maximum velocity (maxV) and the time at which the maximum velocity is reached (maxV-t) are also determined.

### Results

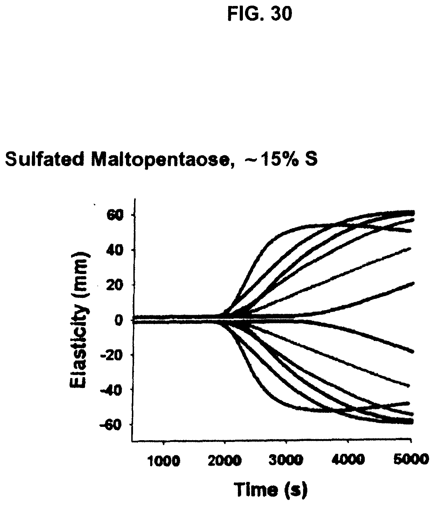

**FIG. 30** is a rotational thromboelastogram (ROTEM) of sulfated maltopentose (15% S) in human whole blood (0.044 pM TF), showing that sulfated maltopentose restores coagulation to normal in FVIII-inhibited blood.

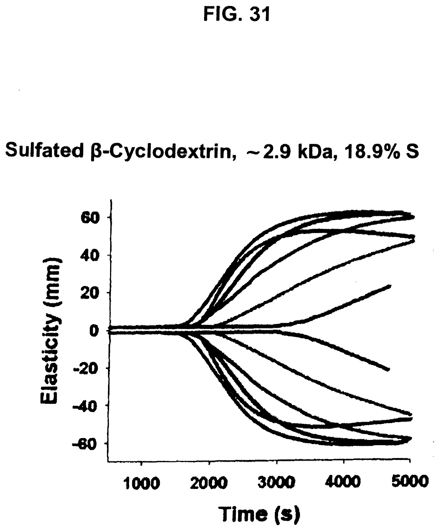

**FIG. 31** is a ROTEM of sulfated β-cyclodextrin (18.9% S) in human whole blood (0.044 pM TF), showing that sulfated β-cyclodextrin restores coagulation to normal in FVIII-inhibited blood.

Sulfated maltopentaose and sulfated β-cyclodextrin show good procoagulant activity and also restore coagulation parameters of FVIII-inhibited whole blood. No activation of the contact pathway by either sulfated maltopentaose and sulfated β-cyclodextrin and most other polysaccharides. Sulfated polysaccharides reverse the anticoagulant effect of exogenous full-length TFPI in normal human plasma.

### EXAMPLE 5

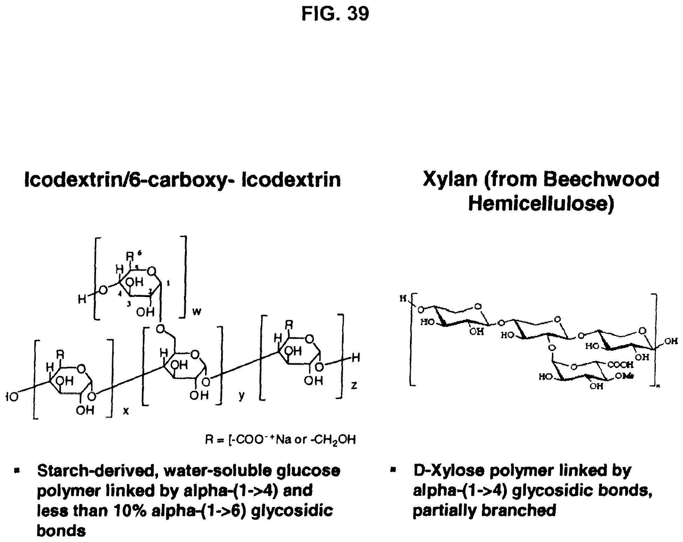

### Preparation of Sulfated and Depolymerized Xylan and Icodextrin (FIG. 39)

### 5.1a Synthesis of Sulfated Xylan

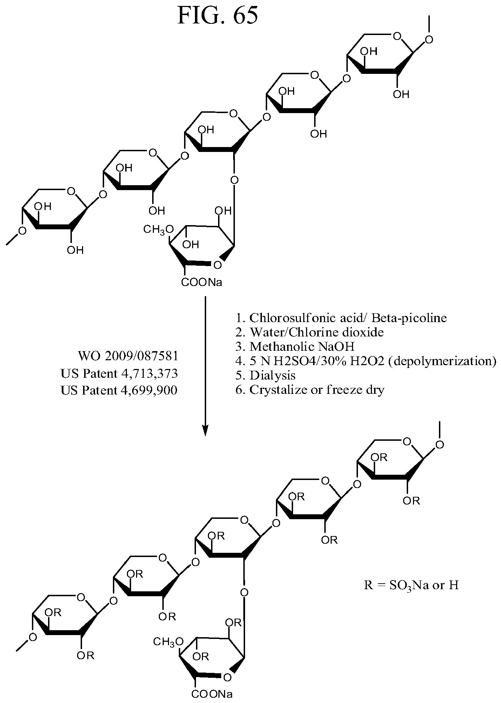

Xylan was sulfated as described in **FIG. 65****.** Following sulfation, the polymer was treated with sulfuric acid and hydrogen peroxide for varying lengths of time in an attempt to yield polymers with a target molecular weight range of 1,000 to 4,000 Da. After the depolymerization step, the solutions were dialyzed across a 3,000 MWCO membrane. The retentates and filtrates were collected and NMR was performed. NMR analysis revealed that only the 3 K retentates contained material resembling carbohydrates. Next, the 3 K retentates were dialyzed across a 10 K MWCO membrane. This time, only the filtrates were kept and characterized (NMR and SEC-MALLS). The NMR of the filtrates agrees with sulfated xylan. The molecular weights of the samples varied from 11 to 3 kDa. The molecular weights were in line with expectations, in that the molecular weights decreased as the depolymerization time increased.

Sulfated xylan was prepared according to WO 2009/087581. Chlorosulfonic acid (50 mL) was added to a round bottom flask to which beta-picoline (250 mL) was added dropwise while stirring rapidly with heating at 40 °C. Once the picoline was completely added, 25 g of xylan were added to the mixture while stirring vigorously and heating to 85° C. The reaction was held at 85° C for 3.5 hours, after which, the reaction was cooled down to RT and then 125 mL of water was added. The mixture was then poured into methanolic sodium hydroxide (500 mL MeOH + 34 g NaOH + 58 mL water), during which a precipitate formed. The mixture was stirred for approximately 20 min and then the solid was collected by filtration. The filter cake was washed several times with methanol and then finally dried on the filter funnel. The semi-dry solid was transferred to a tared drying pan and dried over night under high vacuum at 45° C to yield 111.2 g. The solid was dissolved in 200 mL of water (pH measured 4.72) and the pH was adjusted to 12.3 with 5 M NaOH, and then 1 L of methanol was added while stirring vigorously. The pH of the mixture was adjusted to 6.85 with glacial acetic acid and then stirred for an additional 10 min. The solid that formed was collected by vacuum filtration and transferred to a tared drying dish and dried over night under high vacuum at 50° C to yield 103 g of sulfated xylan. This dry powder was purified further by tangential ultrafiltration (PALL, Centramate, Omega membrane, 1000 MWCO). The dialyzed solution was freeze dried to yield a powder weighing 39.9 g.

### Depolymerization

5 g of sulfated xylan was dissolved in 15 mL of water and heated to 90° C. In parallel, 150 µL of concentrated sulfuric acid and 300 µL of 30% hydrogen peroxide were mixed together in a small vial, heated to 80° C, and then added to the heated sulfated xylan to start the depolymerization process. Aliquots (3 mL) were pulled from the reaction mixture at 15, 30, 60, and 75 min. The pulled aliquots were added to 1080 µL of 1 M NaOH and cooled on an ice-bath. The pH of the aliquot was adjusted to 7 with dropwise additions of 1 M NaOH or 1 M HCl. The aliquots were then transferred to separate ultrafiltration units (3000 molecular weight cutoff, PALL Corp., Macrosep, OMEGA membrane) and centrifuged at 5,000 rpm for 30 min at 5° C. Following centrifugation, the filtrates were collected, and deionized water was added to the retentates to the maximum volume line. The samples were then centrifuged again as done above. This cycle was repeated 9 more times. The combined filtrates and corresponding retentates were then freeze dried. The resulting samples are presented below in Table 1. NMR was performed on each of the samples presented in Table 1. The results are described below. Next, the 3 K retentate samples were dissolved in water and loaded into 10 K ultrafiltration devices (Amicon Ultra, Cat UFC801008) and ultrafiltered as described above with the 3 K devices. The collected filtrates were then freeze dried. The resulting samples are described in Table 2. The retentates were not freeze-dried.

### 5.1b Results

**Table 1. Depolymerized Sulfated Xylan, Subjected to 10 K Ultrafiltration.**

| Sample ID | Description | Weight (g) |
|---|---|---|
| A | 15 min depolymerization (filtrate) | 0.031 |
| E | 15 min depolymerization (retentate) | N/A |
| B | 30 min depolymerization (filtrate) | 0.121 |
| F | 30 min depolymerization (retentate) | N/A |
| C | 60 min depolymerization (filtrate) | 0.299 |
| G | 60 min depolymerization (retentate) | N/A |
| D | 75 min depolymerization (filtrate) | 0.276 |
| H | 75min depolymerization (retentate) | N/A |

### Analytical

***¹H NMR***

### Sample Preparation:

About 100 mg of each sample was dissolved in approximately 1 mL of deuterium oxide. NMR was obtained at room temperature using a 400 mHz instrument.

Proton NMR was performed on the 3 K filtrates and retentates. The 1H NMR of the 3K retentates was consistent with sulfated xylan. Changes in the spectrum are seen as the depolymerization time increases. The 1H NMR of the 3K filtrates (not shown) were not consistent with sulfated xylan, indicating the depolymerization conditions were not sufficient to produce sulfated xylans able to pass a 3 K MWCO membrane. NMR spectra were consistent with spectra presented in the literature (Chaidedgumjorn et al., Carbohydrate Res., 337: 925-933 (2002)).

### SEC-MALLS

Based on the NMR results, SEC-MALLS was only performed on the 10K filtrates (D). The results are presented below in Table 2. In addition, pentosan polysulfate (PPS, obtained from Bioscience) was analyzed. The molecular weights of the samples treated with sulfuric acid and hydrogen peroxide (D) decreased over time (as expected).

**Table 2. Molecular Weights of Sulfated Xylans.**

| Sample ID | Mw (Da) | Polydispersity (Mw/Mn) |
|---|---|---|
| A (15 min depolymerization) | 10,990 | 2.726 |
| B (30 min depolymerization) | 8,295 | 1.982 |
| C (60 min depolymerization) | 6,495 | 1.303 |
| D (75 min depolymerization) | 2,811 | 1.079 |
| 1 (Starting sulfated Xylan) | 22,200 | 1.648 |
| Pentosan Polysulfate (Bioscience) | 6,816 | 1.457 |

### Elemental Analysis

Elemental analysis was performed (QTI-Intertek, Whitehouse, NJ) on samples C and D. Results are presented in Table 3. Based on the MW obtained by SEC-MALLS, oligomers of 20 monomeric sugar units (icosamer) and 9 monomeric sugar units (enneamer) come closest in agreement. Based on this, the elemental results (found) can be compared to expected elemental results for oligomers such as these. These results are presented in Table 3.

**Table 3. Elemental Analysis (found) of Sulfated Xylan Oligomers.**

| Sample ID | C | H | Na | O | S |
|---|---|---|---|---|---|
| C | 19.24 | 2.99 | 10.24 | 54.66 | 12.87 |
| D | 19.76 | 3.09 | 10.19 | 52.09 | 14.87 |

**Table 4. Elemental Analysis (expected) for Sulfated Xylan Oligomers.**

| Oligomer | C | H | Na | O | S |
|---|---|---|---|---|---|
| Icosamer (20-mer) | 17.86 | 1.83 | 13.67 | 47.57 | 19.07 |
| Enneamer (9-mer) | 17.76 | 1.85 | 13.60 | 47.83 | 18.96 |

From the results presented in Table 4, the empirical formulae were calculated. The empirical formulae were calculated using the molecular weights (Mw) obtained by SEC-MALLS. The empirical formulae for samples C and D are presented in Table 5. Again, the theoretical (expected) empirical formulae for icosamer (20-mer) and enneamer (9-mer) are presented in Table 6.

**Table 5. Empirical Formulae (found) for Sulfated Xylan Oligomers.**

| Sample ID | C | H | Na | O | S |
|---|---|---|---|---|---|
| C | 104 | 192 | 29 | 222 | 26 |
| D | 46 | 86 | 12 | 92 | 13 |

**Table 6. Empirical Formulae (expected) for Sulfated Xylan Oligomers.**

| Oligomer | C | H | Na | O | S |
|---|---|---|---|---|---|
| Icosamer (20-mer) | 100 | 122 | 40 | 200 | 40 |
| Enneamer (9-mer) | 45 | 56 | 18 | 91 | 18 |

Sulfate content was also measured by conductivity, and the results are presented in Table 7.

**Table 7. Sulfate Content of Sulfated Polysaccharides.**

| Sample ID | %S (Conductivity) | %S (ICP-OES) |
|---|---|---|
| 1 (sulfated xylan)^{a} | 14.7 | Not submitted |
| 2 (re-sulfated xylan)^{b} | 15.6 | 16.5 |
| Pentosan Polysulfate (Bioscience) | 13.0, 12.9 | 10.7 |
| C (sulfated xylan oligosaccharide)^{c} | 13.0 | 12.9 |
| D (sulfated xylan oligosaccharide)^{c} | 16.1 | 14.9 |

| | | |
|---|---|---|
| ^{a} Sulfated by chlorosulfonic acid/beta-picoline method. ^{b} Repeated chlorosulfonic acid/beta-picoline sulfation method on sulfated xylan 1. ^{c} Sulfated by chlorosulfonic acid/beta-picoline method, then depolymerized w/ H₂0₂/H₂SO₄. ^{d} Sulfated by the sulfur trioxide pyridine complex method | | |

### Thrombin Generation Assay

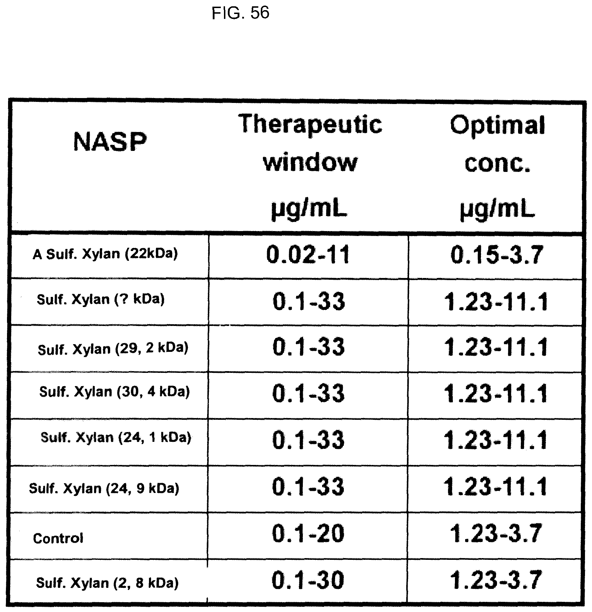

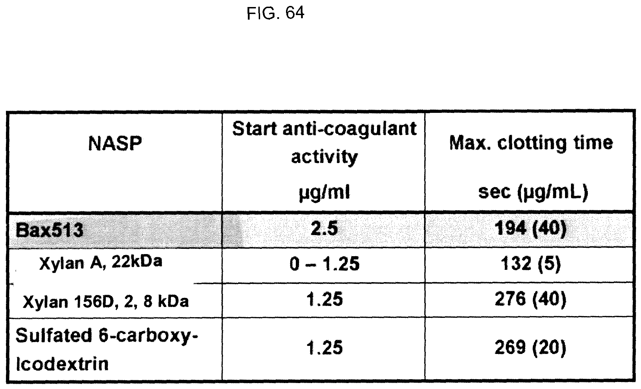

CATs and data derived from the thrombin generation assay for the representative sulfated xylans of the invention are set forth in **FIG. 40****,** **FIG. 41** and **FIG. 56** **-** **FIG. 62** and **FIG. 64****.**

### 5.2a Preparation of Sulfated 6-Carboxy-Icodextrin

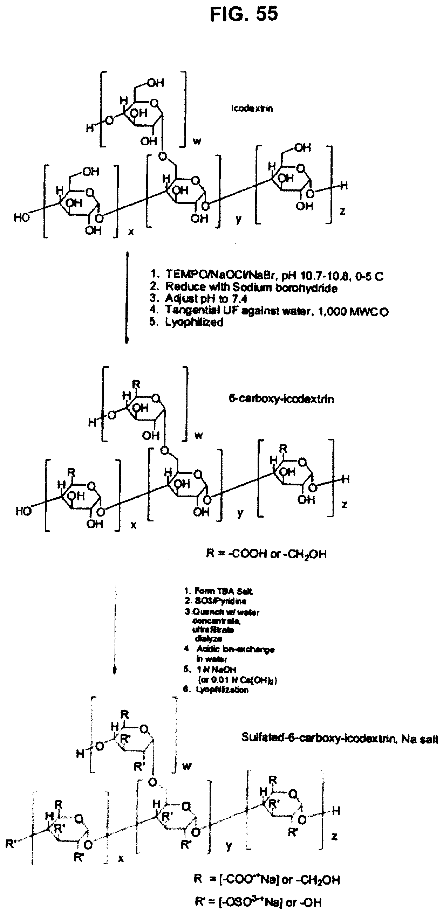

6-Carboxy-icodextrin was sulfated using the method of Maruyama (Maruyama, T., et al., Carbohydrate Research, 306 (1998) 35-43) as a guide (**FIG. 55**). 6-Carboxy-icodextrin (2.57 g) was converted to the free acid by treatment with strong cation-exchange resin (5 g, Bio-Rad AG 50W-X8 resin). The 6-carboxy-icodextrin, free acid, aqueous solution (100 mL) was treated with tributylamine (TBA) until a pH of 8 was obtained. The solution was then rotary evaporated to a solid. The residue was dissolved in about 100 mL of water and then freeze dried to yield 3.6 g of 6-carboxy-icodextrin, TBA salt. The 6-carboxy-icodextrin, TBA salt was sulfated as follows. 6-carboxy-icodextrin, TBA salt (3.6 g) was dissolved in 112 mL of DMF. The solution was placed in a 40° C oil bath, after which, 21.5 g of sulfur trioxide pyridine complex was added. The solution was stirred at 40° C for 1 h. During this time, a gummy "ball" formed in the reaction mixture. The ball was removed from the reaction mixture and dissolved in 100 mL of water. The solution was pH adjusted to 9.95 with 1 M NaOH and extracted with 3 x 50 mL portions of dichloromethane. The aqueous layer was separated and rotary evaporated to a volume of approximately 30 mL. The concentrate was treated with strong cation-exchange resin. The resulting solution measured pH 1.7 and was adjusted to pH 7 with 1 M NaOH. The solution was transferred to a dialysis cassette (3,500 MWCO membrane, Pierce Slide-A-Lyzer) and continuously dialyzed against DI water for approximately 15 h at RT. The dialyzed material was then freeze dried to yield 1.9 g of sulfated 6-carboxy-icodextrin.

### 5.2b Results

**Table 1. Sulfate Content of Sulfated Polysaccharides.**

| Sample ID | %S (Conductivity) | %S (ICP-OES) |
|---|---|---|
| (Sulfated 6-carboxy-icodextrin, batch 1)^{e} | 10.6 | 11.6 |

| | | |
|---|---|---|
| ^{e} 6-carboxy-icodextrin was converted to the tributylamine salt and then sulfated by sulfur trioxide pyridine complex method. | | |

The sulfated 6-carboxy-icodextrins were assayed for their ability to alter clotting. The results from the Thrombin Generation Assay are shown in **FIG. 42** **-** **FIG. 47****.**

**Table 2. Results from aPTT and CAT Assay for unfractionated 6-carboxyicodextrins**

| Substance | 50% Increase Clotting Time µg/mL | EC₅₀ µg/mL | Ratio aPTT/CAT |
|---|---|---|---|
| Fucoidan (Control) | 7.0 | 0.3 | 23.3 |
| 6-carboxy-Icodextrin (unfractionated, batch 1) | 3.2 | 0.04 | 80.0 |
| 6-carboxy-Icodextrin (unfractionated, batch 2) | 4.0 | 0.07 | 57.0 |

**Table 3. Results from aPTT and CAT Assay for fractionated sulfated icodextrins.**

| Substance | 50% Increase Clotting Time µg/mL | EC₅₀ µg/mL | Ratio aPTT/CAT |
|---|---|---|---|
| Fucoidan (Control) | 7.0 | 0.3 | 23.3 |
| Icodextrin >10K | 2.9 | 0.3 | 9.7 |
| Icodextrin 3-10K | 3.1 | 0.5 | 6.2 |
| Icodextrin <3K | >60.0 | 21.8 | ? |

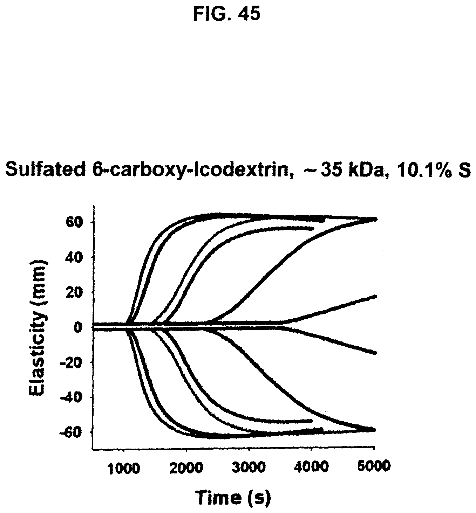

**Table 4 provides ROTEM data for sulfated 6-carboxy-icodextrin (Example 4, FIG. 45) Table 4.**

| | CT (s) | CFT (s) | MCF (mm) | Activity (%) |
|---|---|---|---|---|
| FVIII-inh. Blood | 3922 | 1986 | - | 0 |
| +0.41 µg/ml Icodex | 1542 | 388 | 62 | 108 |
| +1.23 µg/ml Icodex | 1078 | 172 | 63.5 | 129 |
| +3.7 µg/ml Icodex | 1152 | 223 | 63 | 125 |
| +11.1 µg/ml Icodex | 2526 | 934 | - | 63 |
| Normal blood | 1711 | 340 | 55 | 100 |

Sulfated xylan and sulfated 6-carboxy-icodextrin show good procoagulant activity in CAT assays at very low concentrations, having an EC₅₀ of 0.1 µg/mL and 0.07 µg/mL, respectively. 6-carboxy-icodextrin also restores coagulation in FVIII-inhibited whole blood as determined by ROTEM measurements. Sulfated 6-carboxy-icodextrin has a very good aPTT/EC₅₀ ratio. Overall, carboxylation of C6-OH results in a better procoagulant activity maybe due to higher stability of the molecule. Fractions with lower molecular weight display reduced activities. However, fraction 93A (<3K 6-carboxy-icodextrin) still reaches about two-fold normal plasma level with an EC₅₀ of 0.4 µg/mL. Slight activation of the contact pathway by sulfated 6-carboxy-icodextrin is observed at concentrations > 33 µg/mL. Sulfated 6-carboxy-icodextrin reverses the anticoagulant effect of exogenous full-length TFPI in normal human plasma.

### EXAMPLE 6

### Caco-2 cell/ In-vivo Studies

### Objective:

One strategy to improve the oral bioavailability of NASPs is the application of tight-junction-modulating permeation enhancers such as chitosan, bromelain, deoxycholine (DOC), or sodium caprate. The goal of this study was to determine the *in-vitro* resorption of selected NASPs in the Caco-2-cell model in the absence and presence of permeation enhancers.

### Methods:

Human colon adenocarcinoma (Caco-2) cells cultured on semi-permeable filters spontaneously differentiate to form a confluent monolayer. This cell layer resembles both, structurally and functionally, the small intestinal epithelium. Caco-2 cells were cultured in a PET transwell-24 plate in RPMI-cell growth medium supplemented with 10% fetal calf serum and 1% L-glutamine. After 21 days in an incubator at 37°C and 95% air, 5% CO₂ atmosphere, a confluent monolayer was obtained. Four selected NASPs dissolved in 200 µL growth medium with or without permeation enhancers were added onto the cells in the apical compartment at a concentration of 1 mg/mL and incubated at 37 °C. The NASPs and permeation enhancers used for this study are listed in Table 1. Medium samples (100 µL) were collected at 2, 4, 6 and 8 h from the basolateral side (850 µL volume) and before and at 8 h from the apical side. At each sample collection, the removed aliquot was replaced with fresh growth medium. To ensure that the cell layer stayed intact during the experiment, the transepithelial electric resistance (TEER) was monitored and recorded. In each experiment, triplicate wells were tested and the experiment performed one to three times.

The amount of NASP that was transferred from the apical into the basolateral compartment over a time period of 8 h was determined by a semi-quantitative activity-based thrombin generation assay (CAT) for all time points and a substance-specific liquid chromatography mass spectroscopy for the 8 h time point only.

### Results:

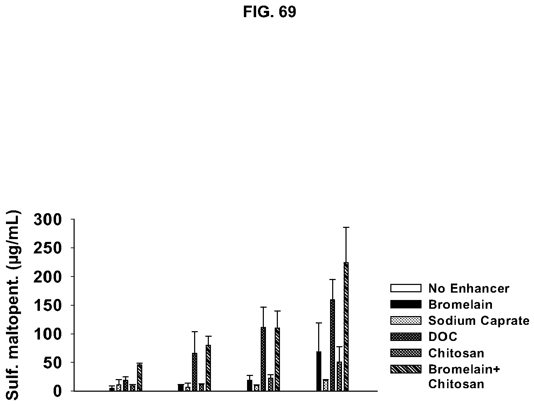

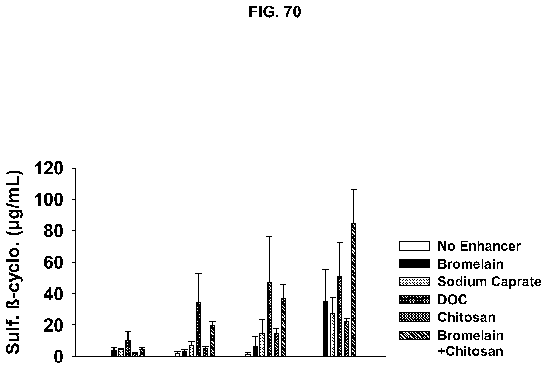

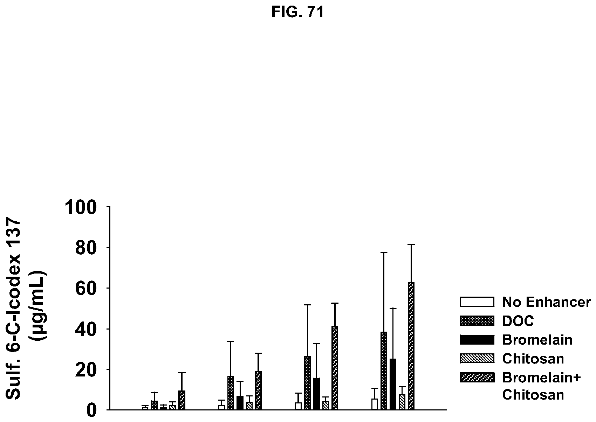

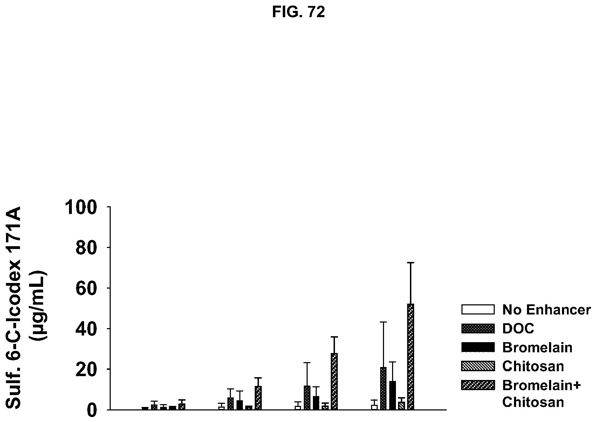

The resorption of 4 synthetic NASPs in combination with 4 or 5 enhancers was studied in the Caco-2 cell model. The amount of NASP on the basolateral side of the cells increased with time (**FIG. 69****,** **70****,** **71****,** **72**). The theoretically possible maximal concentration (µg/mL) was slightly different for each time point due to the dilution factor caused by the sampling. At 8 h, the possible maximum was 140 µg/mL NASP. The resorption at the 8 h time point was also expressed in % resorption While in the absence of permeation enhancers, no or only weak transport of all NASPs through the cells was observed, the resorption increased significantly in the presence of enhancers. Highest resorption was achieved with DOC and a combination of chitosan+bromelain. The observed effect was greater with the low MW NASPs maltopentaose and β-cyclodextrin than for the 6-carboxy-icodextrins.

### EXAMPLE 7

### Objective:

To study the efficacy of unfractionated sulfated 6-carboxy-icodextrin in an *ex-vivo* whole blood TEG FVIII-inhibited guinea pig model in improving clotting parameters.

### Methods:

Male Dunkin Hartley guinea pigs were intravenously injected with a goat antihuman FVIII inhibitor plasma at a dose of 42 BU/kg (1.9 mL/kg) 45 min before sampling. After 40 min, NASP was intravenously administered to the animals at 0.05, 0.15, 0.45 or 1.35 mg/kg (N=5 per group). 300 U/kg FEIBA (Baxter, BioScience, Austria) served as a positive control and saline as a vehicle control. Shortly after the injection, the *Vena cava* was punctured and blood was collected in the presence of citrate (ratio 1:9) for whole blood TEG analysis. Measurements were performed using a thromboelastography (TEG) hemostasis analyzer 5000 (Haemonetics Corp, USA) at 37°C. Every blood sample was prepared by pre-warming 20 µL of a 0.2 M CaCl₂ solution in a TEG cuvette at 37°C adding 340 µL of blood, mixing, and then immediately starting the TEG recording. The measurement proceeded for at least 120 min. The TEG parameters of clotting time (R-time), rapidity of clot strengthening (angle) and maximum clot firmness (MA) were recorded. The primary endpoint R-time was plotted and the median value of the different dosing groups compared with each other.

### Results:

Based on *in vitro* results from CAT assays with sulfated 6-carboxy-icodextrin, a dosage pattern for studying the procoagulant effect in FVIII-inhibited guinea pigs (n=5) after intravenous administration of 0.05; 0.15, 0.45 or 1.35 mg/kg NASP was designed. The animals showed a slightly reduced median R-time (clotting time) of 110 min when dosed with 0.15 and 0.45 mg/kg NASP compared to no signs of clotting after 2 hours without treatment (**FIG 73**).

### EXAMPLE 8

### Objective:

To study the pharmacokinetic properties of two sulfated 6-carboxy-icodextrins of different molecular weight in CD rats after oral administration. The study also addresses the question if two permeations enhancers that were selected based on Caco-2-cell-experiments improve the *in vivo* oral bioavailability.

### Methods:

After a night of fasting, male CD rats were orally gavaged with two liquid preparations of sulfated 6-carboxy-icodextrin (S-6-CI) at a dose of 50 mg/kg and 5 mL/kg. The preparations were either unfractionated (average MW of 24 kD) or fractionated by a filter method (average MW of 14 kD). The substances were prepared in a physiological saline solution without enhancer or with 0.8 wt% DOC or with 3 wt% chitosan+0.5 mg/mL bromelain. Each group dosed with NASP consisted of six rats. For each formulation, three additional rats served as vehicle controls. Blood samples were collected in the presence of citrate (ratio 1:9) before dosing and 15, 30 min, 1, 5, and 7 h after the dosing. Platelet-poor plasma was prepared by two centrifugation steps at 3000 rpm for 10 min.

The NASPs in the plasma samples were detected by a fluorescence assay that uses the dye HeparinRed that specifically binds to sulfated polysaccharides. Experiments were performed in a black half-area 96-well microtiter plate (Costar). For the S-6-CI standard curves (0.185 to 2.5 µg/mL), 10 µL of rat plasma was spiked with 5 µL of S-6-CI and mixed with 40 µL of human pooled plasma to minimize the matrix effect of individual animals. Then 5 µl HeparinRed dye was added and incubated in the dark at RT for 5 min. Plasma samples of the dosed animals were treated accordingly, however, the 5 µL S-6-CI were replaced by buffer. Fluorescent signals were detected with a Tecan Sailre2™ microplate fluorescence reader (Ex/Em 580/620 nm) and the S-6-CI quantified based on the S-6-CI standard curve. The detection limit for S-6-CI in rat plasma for this assay was 0.185 µg/mL.

### Results:

Two of the six rats orally dosed with 50 mg/kg unfractionated S-6-CI in saline showed plasma levels between 0.2 and 0.6 µg/mL, whereas three out of six rats had plasma levels of up to 3 µg/mL NASP when they were dosed with fractionated S-6-CI. The highest level was reached at 60 min after dosing. For both NASPs, the resorption was not further improved in the presence of DOC where similar plasma levels were reached for both NASPs. Using a combination of chitosan+bromelain as enhancers resulted in a similar outcome for S-6-CI. with chitosan+bromelain, plasma levels at all time points were <0.3 µg/mL which is lower than for the saline group. This reduced uptake may be due to the high viscosity of the chitosan+bromelain formulation. In summary, the oral bioavailability for S-6-CI varied between the individual rats, however, it was possible to detect NASP in the plasma of rats to which it had been orally administered.

### EXAMPLE 9

### Objective:

To study the pharmacokinetic properties of three sulfated 6-carboxy-icodextrins of different MW in CD rats after intravenous administration.

### Methods:

Male CD rats were intravenously administered with three different preparations of sulfated 6-carboxy-icodextrin (S-6-CI) at a dose of 5 mg/kg. The preparations were either unfractionated (average MW of 63 kD) or fractionated by a filter method (lots A and B with an average MW of 12 and 14 kD, respectively). The substances were prepared in a physiological saline solution and injected at 5 mL/kg. Each group consisted of three animals. Blood sample were collected in the presence of citrate (ratio 1:9) before dosing and 5, 30 min, 1, 3, 6, and 10 h after the dosing. Platelet-poor plasma was prepared by two centrifugation steps at 3000 rpm. The plasma samples were analyzed for by a liquid-chromatography -mass spectroscopy, a substance-specific method for S-6-CI.

### Results:

The in vivo recovery 5 min after i.v. dosing of 5 mg/kg S-6-CI was 28-44 µg/mL, which is less than half the expected plasma concentration (∼100 µg/mL). For lot A (12 kD) the recovery was the lowest. For the two fractionated S-6-CIs, the plasma concentration was below the detection limit 6 h after administration. Based on the plasma concentrations of the early time points, half-life is estimated to be between 30-45 min.

## Claims

1. A unit dosage formulation for use in a method for treating a subject in need of enhanced blood coagulation comprising administering a therapeutically effective amount of a composition comprising a non-anticoagulant sulfated or sulfonated polysaccharide (NASP) to the subject wherein the sulfated or sulfonated polysaccharide is a member selected from α-cyclodextrin, β-cyclodextrin, melezitose, stachyose, raffinose, maltotriose, maltotetraose, maltopentaose, cellotriose, cellotetraose, cellopentaose, icodextrin, 6-carboxyicodextrin, the unit dosage formulation comprising the NASP in an amount from about 0.5 mg to about 1000 mg.

2. The unit dosage formulation according to claim 1 for the use according to claim 1, wherein the NASP is in an amount sufficient to provide a dosage from about 0.01 mg/kg to about 100 mg/kg or about 0.01 mg/kg to 20 mg/kg.

3. The unit dosage formulation according to any one of claims 1-2 for the use according to any one of claims 1-2, wherein the NASP is in an amount sufficient to enhance blood coagulation in a subject to whom the unit dosage formulation is administered.

4. The unit dosage formulation according to any one of claims 1-3 for the use according to any one of claims 1-3, wherein the unit dosage formulation is an oral unit dosage formulation.

5. The unit dosage formulation according to any one of claims 1-4 for the use according to any one of claims 1-4, wherein the NASP is administered orally.

6. The unit dosage formulation according to any one of claims 1-5 for the use according to any one of claims 1-5, wherein the subject has a bleeding disorder selected from the group consisting of a chronic or acute bleeding disorder, a congenital coagulation disorder caused by a blood Factor deficiency, and an acquired coagulation disorder.

7. The unit dosage formulation according to any one of claims 1-6 for the use according to any one of claims 1-6, wherein the blood factor deficiency is a deficiency of one or more factors selected from the group consisting of Factor V, Factor VII, Factor VIII, Factor IX, Factor X, Factor XI, Factor XII, Factor XIII, prothrombin, fibrinogen, and von Willebrand Factor.

8. The unit dosage formulation according to any one of claims 1-7 for the use according to any one of claims 1-7, wherein the cause of the need for enhanced blood coagulation is prior administration of an anticoagulant, surgery or other invasive procedure.

9. The unit dosage formulation according to any one of claims 1-8 for the use according to any one of claims 1-8, wherein the method further comprises administering an agent selected from the group consisting of a procoagulant, an activator of the intrinsic coagulation pathway, an activator of the extrinsic coagulation pathway, and a second NASP.

10. The unit dosage formulation according to claim 9 for the use according to claim 9, wherein the agent is selected from the group consisting of tissue factor, Factor II, Factor V, Factor Va, Factor VII, Factor Vila, Factor VIII, Factor Villa, Factor X, Factor Xa, Factor IX, Factor IXa, Factor XI, Factor Xla, Factor XII, Factor Xlla, Factor XIII, prekallikrein, kallikrein, and HMWK, and von Willebrand Factor.

11. The unit dosage formulation according to claim 8 for the use according to claim 8, wherein the anticoagulant is selected from the group consisting of heparin, a coumarin derivative, such as warfarin or dicumarol, tissue factor pathway inhibitor (TFPI), antithrombin III, lupus anticoagulant, nematode anticoagulant peptide (NAPc2), active-site blocked Factor Vila (Factor Vllai), Factor IXa inhibitors, Factor Xa inhibitors, including fondaparinux, idraparinux, DX-9065a, and razaxaban (DPC906), inhibitors of Factors Va and Villa, including activated protein C (APC) and soluble thrombomodulin, thrombin inhibitors, including hirudin, bivalirudin, argatroban, and ximelagatran, and an antibody that binds a coagulation factor.

12. The unit dosage formulation according to claim 8 for the use according to claim 8, wherein the anticoagulant is an antibody that binds a coagulation factor selected from the group consisting of Factor V, Factor VII, Factor VIII, Factor IX, Factor X, Factor XIII, Factor II, Factor XI, Factor XII, von Willebrand Factor, prekallikrein, and HMWK.

13. A method of inhibiting Tissue Factor Pathway Inhibitor (TFPI) activity in a biological sample, the method comprising combining the biological sample with a sufficient amount of a non-anticoagulant sulfated or sulfonated polysaccharide (NASP) to inhibit the TFPI activity, wherein the NASP is a member selected from α-cyclodextrin, β-cyclodextrin, melezitose, stachyose, raffinose, maltotriose, maltotetraose, maltopentaose, cellotriose, cellotetraose, cellopentaose, icodextrin and 6-carboxyicodextrin.

14. A composition comprising: (a) a non-anticoagulant sulfated or sulfonated polysaccharide (NASP) which is a member selected from α-cyclodextrin, β-cyclodextrin, melezitose, stachyose, raffinose, maltotriose, maltotetraose, maltopentaose, cellotriose, cellotetraose, cellopentaose, icodextrin and 6-carboxyicodextrin; and (b) a pharmaceutically acceptable excipient; and one or more factors selected from the group consisting of Factor XI, Factor XII, prekallikrein, HMWK, Factor V, Factor VII, Factor VIII, Factor IX, Factor X, Factor XIII, Factor II, and von Willebrand Factor, tissue factor, Factor Vila, Factor Va, Factor Xa, Factor IXa, Factor Xla, Factor XIIa, and Factor Villa.

15. A method of measuring acceleration of blood clotting by a non-anticoagulant sulfated or sulfonated polysaccharide (NASP) in a biological sample, wherein the NASP is a member selected from α-cyclodextrin, β-cyclodextrin, melezitose, stachyose, raffinose, maltotriose, maltotetraose, maltopentaose, cellotriose, cellotetraose, cellopentaose, icodextrin and 6-carboxyicodextrin, the method comprising: a) combining the biological sample with a composition comprising the NASP; and b) measuring the clotting time of the biological sample, c) comparing the clotting time of the biological sample to the clotting time of a corresponding biological sample not exposed to the NASP, wherein a decrease in the clotting time of the biological sample exposed to the NASP is indicative of a NASP that accelerates the clotting time.

## Patentansprüche

1. Einheitsdosisformulierung zur Verwendung in einem Verfahren zur Behandlung eines Subjekts, das einer verstärkten Blutgerinnung bedarf, umfassend das Verabreichen einer therapeutisch wirksamen Menge einer Zusammensetzung, die ein nicht-antikoagulierendes sulfatiertes oder sulfoniertes Polysaccharid (NASP) umfasst, an das Subjekt, wobei das sulfatierte oder sulfonierte Polysaccharid ein Bestandteil ist, ausgewählt aus α-Cyclodextrin, β-Cyclodextrin, Melezitose, Stachyose, Raffinose, Maltotriose, Maltotetraose, Maltopentaose, Cellotriose, Cellotetraose, Cellopentaose, Icodextrin, 6-Carboxyicodextrin, wobei die Einheitsdosisformulierung das NASP in einer Menge von etwa 0,5 mg bis etwa 1000 mg umfasst.

2. Einheitsdosisformulierung gemäß Anspruch 1 zur Verwendung gemäß Anspruch 1, wobei das NASP in einer Menge vorliegt, die ausreichend ist, um eine Dosis von etwa 0,01 mg/kg bis etwa 100 mg/kg oder etwa 0,01 mg/kg bis 20 mg/kg bereitzustellen.

3. Einheitsdosisformulierung gemäß irgendeinem der Ansprüche 1-2 zur Verwendung gemäß irgendeinem der Ansprüche 1-2, wobei das NASP in einer Menge vorliegt, die ausreichend ist, um die Blutgerinnung in einem Subjekt, dem die Einheitsdosisformulierung verabreicht wird, zu verstärken.

4. Einheitsdosisformulierung gemäß irgendeinem der Ansprüche 1-3 zur Verwendung gemäß irgendeinem der Ansprüche 1-3, wobei die Einheitsdosisformulierung eine orale Einheitsdosisformulierung ist.

5. Einheitsdosisformulierung gemäß irgendeinem der Ansprüche 1-4 zur Verwendung gemäß irgendeinem der Ansprüche 1-4, wobei das NASP oral verabreicht wird.

6. Einheitsdosisformulierung gemäß irgendeinem der Ansprüche 1-5 zur Verwendung gemäß irgendeinem der Ansprüche 1-5, wobei das Subjekt eine Blutgerinnungsstörung hat, ausgewählt aus der Gruppe, bestehend aus einer chronischen oder akuten Blutgerinnungsstörung, einer angeborenen Gerinnungsstörung, verursacht durch einen Blutfaktormangel, und einer erworbenen Gerinnungsstörung.

7. Einheitsdosisformulierung gemäß irgendeinem der Ansprüche 1-6 zur Verwendung gemäß irgendeinem der Ansprüche 1-6, wobei der Blutfaktormangel ein Mangel eines oder mehrerer Faktoren ist, ausgewählt aus der Gruppe, bestehend aus Faktor V, Faktor VII, Faktor VIII, Faktor IX, Faktor X, Faktor XI, Faktor XII, Faktor XIII, Prothrombin, Fibrinogen und von-Willebrand-Faktor.

8. Einheitsdosisformulierung gemäß irgendeinem der Ansprüche 1-7 zur Verwendung gemäß irgendeinem der Ansprüche 1-7, wobei die Ursache der Notwendigkeit einer verstärkten Blutgerinnung die vorherige Verabreichung eines Antikoagulans, eine Operation oder ein anderes invasives Verfahren ist.

9. Einheitsdosisformulierung gemäß irgendeinem der Ansprüche 1-8 zur Verwendung gemäß irgendeinem der Ansprüche 1-8, wobei das Verfahren weiterhin das Verabreichen eines Agens, ausgewählt aus der Gruppe, bestehend aus einem Prokoagulans, einem Aktivator des intrinsischen Koagulationsweges, einem Aktivator des extrinsischen Koagulationsweges und einem zweiten NASP, umfasst.

10. Einheitsdosisformulierung gemäß Anspruch 9 zur Verwendung gemäß Anspruch 9, wobei das Agens aus der Gruppe ausgewählt ist, bestehend aus Gewebefaktor, Faktor II, Faktor V, Faktor Va, Faktor VII, Faktor VIIa, Faktor VIII, Faktor VIIIa, Faktor X, Faktor Xa, Faktor IX, Faktor IXa, Faktor XI, Faktor XIa, Faktor XII, Faktor XIIa, Faktor XIII, Präkallikrein, Kallikrein und HMWK und von-Willebrand-Faktor.

11. Einheitsdosisformulierung gemäß Anspruch 8 zur Verwendung gemäß Anspruch 8, wobei das Antikoagulans aus der Gruppe ausgewählt ist, bestehend aus Heparin, einem Cumarinderivat, wie beispielsweise Warfarin oder Dicumarol, Gewebefaktor-Weg-Inhibitor (tissue factor pathway inhibitor; TFPI), Antithrombin III, Lupus-Antikoagulans, Nematoden-Antikoagulans-Peptid (NAPc2), an der aktiven Stelle blockiertem Faktor Vlla (Faktor VIIai), Faktor IXa-Inhibitoren, Faktor Xa-Inhibitoren, einschließlich Fondaparinux, Idraparinux, DX-9065a und Razaxaban (DPC906), Inhibitoren der Faktoren Va und VIIIa, einschließlich aktiviertem Protein C (APC) und löslichem Thrombomodulin, Thrombin-Inhibitoren, einschließlich Hirudin, Bivalirudin, Argatroban und Ximelagatran, und einem Antikörper, der einen Gerinnungsfaktor bindet.

12. Einheitsdosisformulierung gemäß Anspruch 8 zur Verwendung gemäß Anspruch 8, wobei das Antikoagulans ein Antikörper ist, der einen Gerinnungsfaktor bindet, ausgewählt aus der Gruppe, bestehend aus Faktor V, Faktor VII, Faktor VIII, Faktor IX, Faktor X, Faktor XIII, Faktor II, Faktor XI, Faktor XII, von-Willebrand-Faktor, Präkallikrein und HMWK.

13. Verfahren zum Inhibieren von Gewebefaktor-Weg-Inhibitor (Tissue Factor Pathway Inhibitor; TFPI)-Aktivität in einer biologischen Probe, wobei das Verfahren das Kombinieren der biologischen Probe mit einer ausreichenden Menge eines nicht-antikoagulierenden sulfatierten oder sulfonierten Polysaccharids (NASP), um die TFPI-Aktivität zu inhibieren, umfasst, wobei das NASP ein Bestandteil ist, ausgewählt aus α-Cyclodextrin, β-Cyclodextrin, Melezitose, Stachyose, Raffinose, Maltotriose, Maltotetraose, Maltopentaose, Cellotriose, Cellotetraose, Cellopentaose, Icodextrin und 6-Carboxyicodextrin.

14. Zusammensetzung, umfassend: (a) ein nicht-antikoagulierendes sulfatiertes oder sulfoniertes Polysaccharid (NASP), welches ein Bestandteil ist, ausgewählt aus α-Cyclodextrin, β-Cyclodextrin, Melezitose, Stachyose, Raffinose, Maltotriose, Maltotetraose, Maltopentaose, Cellotriose, Cellotetraose, Cellopentaose, Icodextrin und 6-Carboxyicodextrin; und (b) einen pharmazeutisch akzeptablen Hilfsstoff; und einen oder mehrere Faktoren, ausgewählt aus der Gruppe, bestehend aus Faktor XI, Faktor XII, Präkallikrein, HMWK, Faktor V, Faktor VII, Faktor VIII, Faktor IX, Faktor X, Faktor XIII, Faktor II und von-Willebrand-Faktor, Gewebefaktor, Faktor VIIa, Faktor Va, Faktor Xa, Faktor IXa, Faktor XIa, Faktor XIIa und Faktor VIIIa.

15. Verfahren zum Messen der Beschleunigung der Blutgerinnung durch ein nicht-antikoagulierendes sulfatiertes oder sulfoniertes Polysaccharid (NASP) in einer biologischen Probe, wobei das NASP ein Bestandteil ist, ausgewählt aus α-Cyclodextrin, β-Cyclodextrin, Melezitose, Stachyose, Raffinose, Maltotriose, Maltotetraose, Maltopentaose, Cellotriose, Cellotetraose, Cellopentaose, Icodextrin und 6-Carboxyicodextrin, wobei das Verfahren umfasst: a) Kombinieren der biologischen Probe mit einer das NASP umfassenden Zusammensetzung; und b) Messen der Gerinnungszeit der biologischen Probe, c) Vergleichen der Gerinnungszeit der biologischen Probe mit der Gerinnungszeit einer entsprechenden biologischen Probe, die dem NASP nicht ausgesetzt ist, wobei eine Verringerung der Gerinnungszeit der dem NASP ausgesetzten biologischen Probe auf ein NASP hinweist, das die Gerinnungszeit beschleunigt.

## Revendications

1. Formulation de dosage unitaire à utiliser dans un procédé pour traiter un sujet ayant besoin d'une coagulation sanguine améliorée comprenant l'administration au sujet d'une quantité efficace d'un point de vue thérapeutique d'une composition comprenant un polysaccharide sulfaté ou sulfoné non anticoagulant (NASP), dans laquelle le polysaccharide sulfaté ou sulfoné est un élément sélectionné parmi l'α-cyclodextrine, la β-cyclodextrine, le mélézitose, le stachyose, le raffinose, le maltotriose, le maltotétraose, le maltopentaose, le cellotriose, le cellotétraose, le cellopentaose, l'icodextrine, la 6-carboxyicodextrine, la formulation de dosage unitaire comprenant le NASP en une quantité allant d'environ 0,5 mg à environ 1 000 mg.

2. Formulation de dosage unitaire selon la revendication 1 à utiliser selon la revendication 1, dans laquelle le NASP est en une quantité suffisante pour fournir un dosage allant d'environ 0,01 mg/kg à environ 100 mg/kg ou d'environ 0,01 mg/kg à 20 mg/kg.

3. Formulation de dosage unitaire selon l'une quelconque des revendications 1 à 2 à utiliser selon l'une quelconque des revendications 1 à 2, dans laquelle le NASP est en une quantité suffisante pour améliorer la coagulation sanguine chez un sujet auquel la formulation de dosage unitaire est administrée.

4. Formulation de dosage unitaire selon l'une quelconque des revendications 1 à 3 à utiliser selon l'une quelconque des revendications 1 à 3, dans laquelle la formulation de dosage unitaire est une formulation de dosage unitaire orale.

5. Formulation de dosage unitaire selon l'une quelconque des revendications 1 à 4 à utiliser selon l'une quelconque des revendications 1 à 4, dans laquelle le NASP est administré oralement.

6. Formulation de dosage unitaire selon l'une quelconque des revendications 1 à 5 à utiliser selon l'une quelconque des revendications 1 à 5, dans laquelle le sujet a une affection hémostatique sélectionnée à partir du groupe constitué par une affection hémostatique chronique ou aiguë, une affection de coagulation congénitale provoquée par un manque de Facteur sanguin, et une affection de coagulation acquise.

7. Formulation de dosage unitaire selon l'une quelconque des revendications 1 à 6 à utiliser selon l'une quelconque des revendications 1 à 6, dans laquelle le manque de facteur sanguin est un manque d'un ou plusieurs facteurs sélectionnés à partir du groupe constitué par le Facteur V, le Facteur VII, le Facteur VIII, le Facteur IX, le Facteur X, le Facteur XI, le Facteur XII, le Facteur XIII, la prothrombine, le fibrinogène et le Facteur de von Willebrand.

8. Formulation de dosage unitaire selon l'une quelconque des revendications 1 à 7 à utiliser selon l'une quelconque des revendications 1 à 7, dans laquelle la cause du besoin d'une coagulation sanguine améliorée est l'administration antérieure d'un anticoagulant, une chirurgie ou autre procédure invasive.

9. Formulation de dosage unitaire selon l'une quelconque des revendications 1 à 8 à utiliser selon l'une quelconque des revendications 1 à 8, dans laquelle le procédé comprend en outre l'administration d'un agent sélectionné à partir du groupe constitué par un procoagulant, un activateur de la voie de coagulation intrinsèque, un activateur de la voie de coagulation extrinsèque, et un second NASP.

10. Formulation de dosage unitaire selon la revendication 9 à utiliser selon la revendication 9, dans laquelle l'agent est sélectionné à partir du groupe constitué par un facteur tissulaire, le Facteur II, le Facteur V, le Facteur Va, le Facteur VII, le Facteur VIIa, le Facteur VIII, le Facteur VIIIa, le Facteur X, le Facteur Xa, le Facteur IX, le Facteur IXa, le Facteur XI, le Facteur XIa, le Facteur XII, le Facteur XIIa, le Facteur XIII, la prékallikréine, la kallicréine et HMWK, et le Facteur de von Willebrand.

11. Formulation de dosage unitaire selon la revendication 8 à utiliser selon la revendication 8, dans laquelle l'anticoagulant est sélectionné à partir du groupe constitué par l'héparine, un dérivé de la coumarine, comme la warfarine ou le dicumarol, un inhibiteur de la voie de facteur tissulaire (TFPI), l'antithrombine III, l'anticoagulant de lupus, le peptide anticoagulant de nématode (NAPc2), le Facteur VIIa bloqué sur site actif (Facteur VIIai), des inhibiteurs de Facteur IXa, des inhibiteurs de Facteur Xa, incluant le fondaparinux, l'idraparinux, le DX-9065a et le razaxaban (DPC906), des inhibiteurs de Facteurs Va et VIIIa, incluant la protéine C activée (APC) et la thrombomoduline soluble, des inhibiteurs de thrombine, incluant l'hirudine, la bivalirudine, l'argatroban et le ximélagatran et un anticorps qui se lie à un Facteur de coagulation.

12. Formulation de dosage unitaire selon la revendication 8 à utiliser selon la revendication 8, dans laquelle l'anticoagulant est un anticorps qui se lie à un facteur de coagulation sélectionné à partir du groupe constitué par le Facteur V, le Facteur VII, le Facteur VIII, le Facteur IX, le Facteur X, le Facteur XIII, le Facteur II, le Facteur XI, le Facteur XII, le Facteur de von Willebrand, la prékallikréine et HMWK.

13. Procédé d'inhibition de l'activité d'Inhibiteur de la voie de Facteur Tissulaire (TFPI) dans un échantillon biologique, le procédé comprenant la combinaison de l'échantillon biologique avec une quantité suffisante d'un polysaccharide sulfaté ou sulfoné non anticoagulant (NASP) pour inhiber l'activité de TFPI, dans lequel le NASP est un élément sélectionné parmi l'α-cyclodextrine, la β-cyclodextrine, le mélézitose, le stachyose, le raffinose, le maltotriose, le maltotétraose, le maltopentaose, le cellotriose, le cellotétraose, le cellopentaose, l'icodextrine et la 6-carboxyicodextrine.

14. Composition comprenant : (a) un polysaccharide sulfaté ou sulfoné non anticoagulant (NASP) qui est un élément sélectionné parmi l'α-cyclodextrine, la β-cyclodextrine, le mélézitose, le stachyose, le raffinose, le maltotriose, le maltotétraose, le maltopentaose, le cellotriose, le cellotétraose, le cellopentaose, l'icodextrine et la 6-carboxyicodextrine ; et (b) un excipient acceptable d'un point de vue pharmaceutique ; et un ou plusieurs facteurs sélectionnés à partir du groupe constitué par le Facteur XI, le Facteur XII, la prékallikréine, HMWK, le Facteur V, le Facteur VII, le Facteur VIII, le Facteur IX, le Facteur X, le Facteur XIII, le Facteur II et le Facteur de von Willebrand, un facteur tissulaire, le Facteur VIIa, le Facteur Va, le Facteur Xa, le Facteur IXa, le Facteur XIa, le Facteur XIIa et le Facteur VIIIa.

15. Procédé de mesure de l'accélération de la coagulation sanguine par un polysaccharide sulfaté ou sulfoné non anticoagulant (NASP) dans un échantillon biologique, dans lequel le NASP est un élément sélectionné parmi l'α-cyclodextrine, la β-cyclodextrine, le mélézitose, le stachyose, le raffinose, le maltotriose, le maltotétraose, le maltopentaose, le cellotriose, le cellotétraose, le cellopentaose, l'icodextrine et la 6-carboxyicodextrine, le procédé comprenant : a) la combinaison de l'échantillon biologique avec une composition comprenant le NASP ; et b) la mesure du temps de coagulation de l'échantillon biologique, c) la comparaison du temps de coagulation de l'échantillon biologique au temps de coagulation d'un échantillon biologique correspondant non exposé au NASP, dans lequel une diminution du temps de coagulation de l'échantillon biologique exposé au NASP est indicative d'un NASP qui accélère le temps de coagulation.
